# EUROPEAN PATENT SPECIFICATION

(11) **EP 1 232 182 B1**
(45) Date of publication and mention of the grant of the patent: **03.10.2007**
(21) Application number: 00978456.2
(22) Date of filing: 10.11.2000
(51) Int. Cl.: C07K 14/78, C12N 15/82

(54) **BOVINE COLLAGEN AND METHOD FOR PRODUCING RECOMBINANT GELATIN**
RINDER-KOLLAGEN UND VERFAHREN ZUR HERSTELLING REKOMBINANTES GELATIN
COLLAGENE BOVINE ET PROCEDE DE PRODUCTION DE GELATINE RECOMBINANTE

(30) Priority: 12.11.1999 US 439058
(43) Date of publication of application: 21.08.2002
(73) Proprietor: FIBROGEN, INC., South San Francisco, CA 94080 (US)
(72) Inventor: Bell, Marcum P., Nashville, TN 37212 (US); Neff, Thomas B., Atherton, CA 94027 (US); Polarek, James W., Sausalito, CA 94965 (US); Seeley, Todd W., Moraga, CA 94556 (US)
(74) Representative: Hallybone, Huw George
(86) International application number: PCT/US2000/030792
(87) International publication number: WO 2001/034647

(56) References cited:
- WO-A-97/04123
- FR-A- 2 757 874
- DATABASE SWISSPROT [Online] XP002164542 cited in the application

### SEQUENCE LISTING

<110> FIBROGEN, INC.
<120> ANIMAL COLLAGENS AND GELATINS
<130> FG0217 PCT
<140>
   <141>
<160> 72
<170> PatentIn Ver. 2.0
<210> 1
   <211> 4748
   <212> DNA
   <213> bovine
<400> 1
   cagacgggag tttctcctcg gggtcggagc aggaggcacg cggagtgtga ggccacgcat 60
   gagcggacgc taacccccac cccagccgca aagagtctac atgtctaggg tctagacatg 120
   ttcagctttg tggacctccg gctcctgctc ctcttagcgg ccaccgccct cctgacgcac 180
   ggccaagagg agggccagga agaaggccaa gaagaagaca tcccaccagt cacctgcgta 240
   cagaacggcc tcaggtacca tgaccgagac gtgtggaaac ccgtgccctg ccagatctgt 300
   gtctgcgaca acggcaacgt gctgtgcgat gacgtgatct gcgacgaact taaggactgt 360
   cctaacgcca aagtccccac ggacgaatgc tgccccgtct gccccgaagg ccaggaatca 420
   cccacggacc aagaaaccac cggagtcgag ggaccgaaag gagacactgg cccccgaggc 480
   ccaaggggac ccgccggccc ccccggccga gatggcatcc ctggacaacc tggacttccc 540
   ggaccccctg gaccccccgg acctcccgga ccccctggcc tcggaggaaa ctttgctccc 600
   cagttgtctt acggctatga tgagaaatca acaggaattt ccgtgcctgg tcccatgggt 660
   ccttctggtc ctcgtggtct ccctggcccc cctggcgcac ctggtcccca aggtttccaa 720
   ggcccccctg gtgagcctgg cgagccagga gcctcaggtc ccatgggtcc ccgtggtccc 780
   cctggccccc ctggcaagaa cggagatgat ggcgaagctg gaaagcctgg tcgtcctggt 840
   gagcgcgggc ctcccggacc tcagggtgct cggggattgc ctggaacagc tggcctccct 900
   ggaatgaagg gacacagagg tttcagtggt ttggatggtg ccaagggaga tgctggtcct 960
   gctggcccca agggcgagcc tggtagcccc ggtgaaaatg gagctcctgg tcagatgggc 1020
   ccccgtggtc tgcctggtga gagaggtcgc cctggagccc ctggccctgc tggtgctcga 1080
   ggaaatgatg gtgcgactgg tgctgctggg ccccctggtc ccactggccc cgctggtcct 1140
   cctggtttcc ctggtgctgt gggtgctaag ggtgaaggtg gtccccaagg accccgaggt 1200
   tctgaaggtc cccagggtgt acgtggtgag cctggccccc ctggccctgc tggtgctgct 1260
   ggccctgctg gcaaccctgg tgctgatgga cagcctggtg ctaaaggagc caatggcgct 1320
   cctggtattg ctggtgctcc tggcttccct ggtgcccgag gcccctctgg accccagggc 1380
   cccagcggcc cccctggccc caagggtaac agcggtgaac ctggtgctcc tggcagcaaa 1440
   ggagacactg gcgccaaggg agaacccggt cccactggta ttcaaggccc ccctggcccc 1500
   gctggggaag aaggaaagcg aggagcccga ggtgaacctg gacctgctgg cctgcctgga 1560
   ccccctggcg agcgtggtgg acctggaagc cgtggtttcc ctggcgccga cggtgttgct 1620
   ggtcccaagg gtcctgctgg tgaacgcggt gctcctggcc ctgctggccc caaaggttct 1680
   cctggtgaag ctggtcgccc cggtgaagct ggtctgcccg gtgccaaggg tctgactgga 1740
   agccctggca gcccgggtcc tgatggcaaa actggccccc ctggtcccgc cggtcaagat 1800
   ggccgccctg gacctccagg ccctcccggt gcccgtggtc aggctggcgt gatgggtttc 1860
   cctggaccta aaggtgctgc tggagagcct ggaaaagctg gagagcgagg tgttcctgga 1920
   ccccctggcg ctgttggtcc tgctggcaaa gacggagaag ctggagctca gggaccccca 1980
   ggacctgctg gcccgctggt gagagaggcg aacaaggccc tgctggctcc cctggattcc 2040
   agggtctccc cggccctgct ggtcctcctg gtgaagcagg caaacctggt gaacagggtg 2100
   ttcctggaga tcttggtgcc cccggcccct ctggagcaag aggcgagaga ggtttccccg 2160
   gcgagcgtgg tgtgcaaggg ccgcccggtc ctgcaggtcc ccgtggggcc aatggtgccc 2220
   ctggcaacga tggtgctaag ggtgatgctg gtgcccctgg agcccccggt agccagggtg 2280
   cccctggcct tcaaggaatg cctggtgaac gaggtgcagc tggtcttcca ggccctaagg 2340
   gtgacagagg ggatgctggt cccaaaggtg ctgatggtgc tcctggcaaa gatggcgtcc 2400
   gtggtctgac tggtcccatc ggtcctcctg gccccgctgg tgcccctggt gacaagggtg 2460
   aagctggtcc tagcggccca gccggtccca ctggagctcg tggtgccccc ggtgaccgtg 2520
   gtgagcctgg tccccccggc cctgctggct tcgctggccc ccctggtgct gatggccaac 2580
   ctggtgctaa aggcgaacct ggtgatgctg gtgctaaagg tgacgctggt ccccccggcc 2640
   ctgctgggcc cgctggaccc cccggcccca ttggtaacgt tggtgctccc ggacccaaag 2700
   gtgctcgtgg cagcgctggt ccccctggtg ctactggttt cccaggtgct gctggccgag 2760
   ttggtccccc cggcccctct ggaaatgctg gaccccctgg ccctcctggc cctgctggca 2820
   aagaaggcag caaaggcccc cgcggtgaga ctggccccgc tgggcgtccc ggtgaagtcg 2880
   gtccccctgg tccccctggc cccgctggtg agaaaggagc ccctggtgct gacggacctg 2940
   ctggagctcc tggcactcct ggacctcaag gtattgctgg acagcgtggt gtggtcggcc 3000
   tgcctggtca gagaggagaa agaggcttcc ctggtcttcc tggcccctct ggtgaacccg 3060
   gcaaacaagg tccttctgga gcaagtggtg aacgtggccc ccctggtccc atgggccccc 3120
   ctggattggc tggaccccct ggcgagtctg gacgtgaggg agctcctggt gctgaaggat 3180
   cccctggacg agatggttct cctggcgcca agggtgaccg tggtgagacc ggccctgctg 3240
   gacctcctgg tgctcctggc gctcccggtg cccccggccc tgtcggacct gccggcaaga 3300
   gcggtgatcg tggtgagacc ggtcctgctg gtcctgctgg tcccattggc cccgttggtg 3360
   cccgtggccc cgctggaccc caaggccccc gtggtgacaa gggtgagaca ggcgaacagg 3420
   gcgacagagg cattaagggt caccgtggct tctctggtct ccagggtccc cccggccctc 3480
   ccggctctcc tggtgagcaa ggtccttccg gagcctctgg tcctgctggt ccccgcggtc 3540
   cccctggctc tgctggttct cccggcaaag atggactcaa tggtctccca ggccccatcg 3600
   gtccccctgg gcctcgaggt cgcactggtg atgctggtcc tgctggtcct cccggccctc 3660
   ctggaccccc tggtccccca ggtcctccca gcggcggcta cgacttgagc ttcctgcccc 3720
   agccacctca agagaaggct cacgatggtg gccgctacta ccgggctgat gatgccaatg 3780
   tggtccgtga ccgtgacctc gaggtggaca ccaccctcaa gagcctgagc cagcagatcg 3840
   agaacatccg gagccctgaa ggcagccgca agaaccccgc ccgcacctgc cgtgacctca 3900
   agatgtgcca ctctgactgg aagagcggag aatactggat tgaccccaac caaggctgca 3960
   acctggatgc cattaaggtc ttctgcaaca tggaaaccgg tgagacctgt gtatacccca 4020
   ctcagcccag cgtggcccag aagaactggt atatcagcaa gaaccccaag gaaaagaggc 4080
   acgtctggta cggcgagagc atgaccggcg gattccagtt cgagtatggc ggccaggggt 4140
   ccgatcctgc cgatgtggcc atccagctga ctttcctgcg cctgatgtcc accgaggcct 4200
   cccagaacat cacctaccac tgcaagaaca gcgtggccta catggaccag cagactggca 4260
   acctcaagaa ggccctgctc ctccagggct ccaacgagat cgagatccgg gccgagggca 4320
   acagccgctt cacctacagc gtcacctacg atggctgcac gagtcacacc ggagcctggg 4380
   gcaagacagt gatcgaatac aaaaccacca agacctcccg cttgcccatc atcgatgtgg 4440
   cccccttgga cgttggcgcc ccagaccagg aattcggttt cgacgttggc cctgcctgct 4500
   tcctgtaaac tccttccacc ccaacctggc tccctcccac ccaacccact tgcccctgac 4560
   tctggaaaca gacaaacaac ccaaactgaa acccccgaaa agccaaaaaa tgggagacaa 4620
   tttcacatgg actttggaaa atattttttt cctttgcatt catctctcaa acttagtttt 4680
   tatctttgac caactgaaca tgaccaaaaa ccaaaagtgc attcaacctt accaaaaaaa 4740
   aaaaaaaa 4748
<210> 2
   <211> 1463
   <212> PRT
   <213> bovine
<400> 2
   Met Phe Ser Phe Val Asp Leu Arg Leu Leu Leu Leu Leu Ala Ala Thr
    1               5                   10                  15
   Ala Leu Leu Thr His Gly Gln Glu Glu Gly Gln Glu Glu Gly Gln Glu
               20                  25                  30
   Glu Asp Ile Pro Pro Val Thr Cys Val Gln Asn Gly Leu Arg Tyr His
           35                  40                  45
   Asp Arg Asp Val Trp Lys Pro Val Pro Cys Gln Ile Cys Val Cys Asp
        50                  55                  60
   Asn Gly Asn Val Leu Cys Asp Asp Val Ile Cys Asp Glu Leu Lys Asp
    65                  70                  75                  80
   Cys Pro Asn Ala Lys Val Pro Thr Asp Glu Cys Cys Pro Val Cys Pro
                    85                  90                  95
   Glu Gly Gln Glu Ser Pro Thr Asp Gln Glu Thr Thr Gly Val Glu Gly
               100                 105                 110
   Pro Lys Gly Asp Thr Gly Pro Arg Gly Pro Arg Gly Pro Ala Gly Pro
           115                 120                 125
   Pro Gly Arg Asp Gly Ile Pro Gly Gln Pro Gly Leu Pro Gly Pro Pro
       130                 135                 140
   Gly Pro Pro Gly Pro Pro Gly Pro Pro Gly Leu Gly Gly Asn Phe Ala
   145                 150                 155                 160
   Pro Gln Leu Ser Tyr Gly Tyr Asp Glu Lys Ser Thr Gly Ile Ser Val
                   165                 170                 175
   Pro Gly Pro Met Gly Pro Ser Gly Pro Arg Gly Leu Pro Gly Pro Pro
               180                 185                 190
   Gly Ala Pro Gly Pro Gln Gly Phe Gln Gly Pro Pro Gly Glu Pro Gly
           195                 200                 205
   Glu Pro Gly Ala Ser Gly Pro Met Gly Pro Arg Gly Pro Pro Gly Pro
       210                 215                 220
   Pro Gly Lys Asn Gly Asp Asp Gly Glu Ala Gly Lys Pro Gly Arg Pro
   225                 230                 235                 240
   Gly Glu Arg Gly Pro Pro Gly Pro Gln Gly Ala Arg Gly Leu Pro Gly
                   245                 250                 255
   Thr Ala Gly Leu Pro Gly Met Lys Gly His Arg Gly Phe Ser Gly Leu
               260                 265                 270
   Asp Gly Ala Lys Gly Asp Ala Gly Pro Ala Gly Pro Lys Gly Glu Pro
           275                 280                 285
   Gly Ser Pro Gly Glu Asn Gly Ala Pro Gly Gln Met Gly Pro Arg Gly
       290                 295                 300
   Leu Pro Gly Glu Arg Gly Arg Pro Gly Ala Pro Gly Pro Ala Gly Ala
   305                 310                 315                 320
   Arg Gly Asn Asp Gly Ala Thr Gly Ala Ala Gly Pro Pro Gly Pro Thr
                   325                 330                 335
   Gly Pro Ala Gly Pro Pro Gly Phe Pro Gly Ala Val Gly Ala Lys Gly
               340                 345                 350
   Glu Gly Gly Pro Gln Gly Pro Arg Gly Ser Glu Gly Pro Gln Gly Val
           355                 360                 365
   Arg Gly Glu Pro Gly Pro Pro Gly Pro Ala Gly Ala Ala Gly Pro Ala
       370                 375                 380
   Gly Asn Pro Gly Ala Asp Gly Gln Pro Gly Ala Lys Gly Ala Asn Gly
   385                 390                 395                 400
   Ala Pro Gly Ile Ala Gly Ala Pro Gly Phe Pro Gly Ala Arg Gly Pro
                   405                 410                 415
   Ser Gly Pro Gln Gly Pro Ser Gly Pro Pro Gly Pro Lys Gly Asn Ser
               420                 425                 430
   Gly Glu Pro Gly Ala Pro Gly Ser Lys Gly Asp Thr Gly Ala Lys Gly
           435                 440                 445
   Glu Pro Gly Pro Thr Gly Ile Gln Gly Pro Pro Gly Pro Ala Gly Glu
       450                 455                 460
   Glu Gly Lys Arg Gly Ala Arg Gly Glu Pro Gly Pro Ala Gly Leu Pro
   465                 470                 475                 480
   Gly Pro Pro Gly Glu Arg Gly Gly Pro Gly Ser Arg Gly Phe Pro Gly
                   485                 490                 495
   Ala Asp Gly Val Ala Gly Pro Lys Gly Pro Ala Gly Glu Arg Gly Ala
               500                 505                 510
   Pro Gly Pro Ala Gly Pro Lys Gly Ser Pro Gly Glu Ala Gly Arg Pro
           515                 520                 525
   Gly Glu Ala Gly Leu Pro Gly Ala Lys Gly Leu Thr Gly Ser Pro Gly
       530                 535                 540
   Ser Pro Gly Pro Asp Gly Lys Thr Gly Pro Pro Gly Pro Ala Gly Gln
   545                 550                 555                 560
   Asp Gly Arg Pro Gly Pro Pro Gly Pro Pro Gly Ala Arg Gly Gln Ala
                   565                 570                 575
   Gly Val Met Gly Phe Pro Gly Pro Lys Gly Ala Ala Gly Glu Pro Gly
               580                 585                 590
   Lys Ala Gly Glu Arg Gly Val Pro Gly Pro Pro Gly Ala Val Gly Pro
           595                 600                 605
   Ala Gly Lys Asp Gly Glu Ala Gly Ala Gln Gly Pro Pro Gly Pro Ala
       610                 615                 620
   Gly Pro Ala Gly Glu Arg Gly Glu Gln Gly Pro Ala Gly Ser Pro Gly
   625                 630                 635                 640
   Phe Gln Gly Leu Pro Gly Pro Ala Gly Pro Pro Gly Glu Ala Gly Lys
                   645                 650                 655
   Pro Gly Glu Gln Gly Val Pro Gly Asp Leu Gly Ala Pro Gly Pro Ser
               660                 665                 670
   Gly Ala Arg Gly Glu Arg Gly Phe Pro Gly Glu Arg Gly Val Gln Gly
           675                 680                 685
   Pro Pro Gly Pro Ala Gly Pro Arg Gly Ala Asn Gly Ala Pro Gly Asn
   690 695 700
   Asp Gly Ala Lys Gly Asp Ala Gly Ala Pro Gly Ala Pro Gly Ser Gln
   705 710 715 720
   Gly Ala Pro Gly Leu Gln Gly Met Pro Gly Glu Arg Gly Ala Ala Gly
   725 730 735
   Leu Pro Gly Pro Lys Gly Asp Arg Gly Asp Ala Gly Pro Lys Gly Ala
   740 745 750
   Asp Gly Ala Pro Gly Lys Asp Gly Val Arg Gly Leu Thr Gly Pro Ile
   755 760 765
   Gly Pro Pro Gly Pro Ala Gly Ala Pro Gly Asp Lys Gly Glu Ala Gly
   770 775 780
   Pro Ser Gly Pro Ala Gly Pro Thr Gly Ala Arg Gly Ala Pro Gly Asp
   785 790 795 800
   Arg Gly Glu Pro Gly Pro Pro Gly Pro Ala Gly Phe Ala Gly Pro Pro
   805 810 815
   Gly Ala Asp Gly Gln Pro Gly Ala Lys Gly Glu Pro Gly Asp Ala Gly
   820 825 830
   Ala Lys Gly Asp Ala Gly Pro Pro Gly Pro Ala Gly Pro Ala Gly Pro
   835 840 845
   Pro Gly Pro Ile Gly Asn Val Gly Ala Pro Gly Pro Lys Gly Ala Arg
   850 855 860
   Gly Ser Ala Gly Pro Pro Gly Ala Thr Gly Phe Pro Gly Ala Ala Gly
   865 870 875 880
   Arg Val Gly Pro Pro Gly Pro Ser Gly Asn Ala Gly Pro Pro Gly Pro
   885 890 895
   Pro Gly Pro Ala Gly Lys Glu Gly Ser Lys Gly Pro Arg Gly Glu Thr
   900 905 910
   Gly Pro Ala Gly Arg Pro Gly Glu Val Gly Pro Pro Gly Pro Pro Gly
   915 920 925
   Pro Ala Gly Glu Lys Gly Ala Pro Gly Ala Asp Gly Pro Ala Gly Ala
   930 935 940
   Pro Gly Thr Pro Gly Pro Gln Gly Ile Ala Gly Gln Arg Gly Val Val
   945 950 955 960
   Gly Leu Pro Gly Gln Arg Gly Glu Arg Gly Phe Pro Gly Leu Pro Gly
   965 970 975
   Pro Ser Gly Glu Pro Gly Lys Gln Gly Pro Ser Gly Ala Ser Gly Glu
   980 985 990
   Arg Gly Pro Pro Gly Pro Met Gly Pro Pro Gly Leu Ala Gly Pro Pro
   995 1000 1005
   Gly Glu Ser Gly Arg Glu Gly Ala Pro Gly Ala Glu Gly Ser Pro Gly
   1010 1015 1020
   Arg Asp Gly Ser Pro Gly Ala Lys Gly Asp Arg Gly Glu Thr Gly Pro
   1025 1030 1035 1040
   Ala Gly Pro Pro Gly Ala Pro Gly Ala Pro Gly Ala Pro Gly Pro Val
   1045 1050 1055
   Gly Pro Ala Gly Lys Ser Gly Asp Arg Gly Glu Thr Gly Pro Ala Gly
   1060 1065 1070
   Pro Ala Gly Pro Ile Gly Pro Val Gly Ala Arg Gly Pro Ala Gly Pro
   1075 1080 1085
   Gln Gly Pro Arg Gly Asp Lys Gly Glu Thr Gly Glu Gln Gly Asp Arg
   1090 1095 1100
   Gly Ile Lys Gly His Arg Gly Phe Ser Gly Leu Gln Gly Pro Pro Gly
   1105 1110 1115 1120
   Pro Pro Gly Ser Pro Gly Glu Gln Gly Pro Ser Gly Ala Ser Gly Pro
   1125 1130 1135
   Ala Gly Pro Arg Gly Pro Pro Gly Ser Ala Gly Ser Pro Gly Lys Asp
   1140 1145 1150
   Gly Leu Asn Gly Leu Pro Gly Pro Ile Gly Pro Pro Gly Pro Arg Gly
   1155 1160 1165
   Arg Thr Gly Asp Ala Gly Pro Ala Gly Pro Pro Gly Pro Pro Gly Pro
   1170 1175 1180
   Pro Gly Pro Pro Gly Pro Pro Ser Gly Gly Tyr Asp Leu Ser Phe Leu
   1185 1190 1195 1200
   Pro Gln Pro Pro Gln Glu Lys Ala His Asp Gly Gly Arg Tyr Tyr Arg
   1205 1210 1215
   Ala Asp Asp Ala Asn Val Val Arg Asp Arg Asp Leu Glu Val Asp Thr
   1220 1225 1230
   Thr Leu Lys Ser Leu Ser Gln Gln Ile Glu Asn Ile Arg Ser Pro Glu
   1235 1240 1245
   Gly Ser Arg Lys Asn Pro Ala Arg Thr Cys Arg Asp Leu Lys Met Cys
   1250 1255 1260
   His Ser Asp Trp Lys Ser Gly Glu Tyr Trp Ile Asp Pro Asn Gln Gly
   1265 1270 1275 1280
   Cys Asn Leu Asp Ala Ile Lys Val Phe Cys Asn Met Glu Thr Gly Glu
   1285 1290 1295
   Thr Cys Val Tyr Pro Thr Gln Pro Ser Val Ala Gln Lys Asn Trp Tyr
   1300 1305 1310
   Ile Ser Lys Asn Pro Lys Glu Lys Arg His Val Trp Tyr Gly Glu Ser
   1315 1320 1325
   Met Thr Gly Gly Phe Gln Phe Glu Tyr Gly Gly Gln Gly Ser Asp Pro
       1330                1335                1340
   Ala Asp Val Ala Ile Gln Leu Thr Phe Leu Arg Leu Met Ser Thr Glu
   1345                1350                1355                1360
   Ala Ser Gln Asn Ile Thr Tyr His Cys Lys Asn Ser Val Ala Tyr Met
                   1365                1370                1375
   Asp Gln Gln Thr Gly Asn Leu Lys Lys Ala Leu Leu Leu Gln Gly Ser
               1380                1385                1390
   Asn Glu Ile Glu Ile Arg Ala Glu Gly Asn Ser Arg Phe Thr Tyr Ser
           1395                1400                1405
   Val Thr Tyr Asp Gly Cys Thr Ser His Thr Gly Ala Trp Gly Lys Thr
       1410                1415                1420
   Val Ile Glu Tyr Lys Thr Thr Lys Thr Ser Arg Leu Pro Ile Ile Asp
   1425                1430                1435                1440
   Val Ala Pro Leu Asp Val Gly Ala Pro Asp Gln Glu Phe Gly Phe Asp
                   1445                1450                1455
   Val Gly Pro Ala Cys Phe Leu
               1460
<210> 3
   <211> 4428
   <212> DNA
   <213> bovine
<400> 3
   gaattcaggg acatgatgag ctttgtgcaa aaggggacct ggttactttt cgctctgctt 60
   catcccactg ttattttggc acaacaggaa gctgttgacg gaggatgctc ccatctcggt 120
   cagtcttatg cagatagaga tgtatggaaa ccagaaccgt gccaaatatg cgtctgtgac 180
   tcaggatccg ttctctgtga tgacataata tgtgacgacc aagaattaga ctgccccaac 240
   cctgaaatcc cgtttggaga atgttgtgca gtttgcccac agcctccaac agctcccact 300
   cgccctccta atggtcaagg acctcaaggc cccaagggag atccaggtcc tcctggtatt 360
   cctgggcgaa atggcgatcc tggtcctcca ggatcaccag gctccccagg ttctcccggc 420
   cctcctggaa tctgtgaatc atgtcctact ggtggccaga actattctcc ccagtacgaa 480
   gcatatgatg tcaagtctgg agtagcagga ggaggaatcg caggctatcc tgggccagct 540
   ggtcctcctg gcccacccgg accccctggc acatctggcc atcctggtgc ccctggcgct 600
   ccaggatacc aaggtccccc cggtgaacct gggcaagctg gtccggcagg tcctccagga 660
   cctcctggtg ctataggtcc atctggccct gctggaaaag atggggaatc aggaagaccc 720
   ggacgacctg gagagcgagg atttcctggc cctcctggta tgaaaggccc agctggtatg 780
   cctggattcc ctggtatgaa aggacacaga ggctttgatg gacgaaatgg agagaaaggc 840
   gaaactggtg ctcctggatt aaagggggaa aatggcgttc caggtgaaaa tggagctcct 900
   ggacccatgg gtccaagagg ggctcccggt gagagaggac ggccaggact tcctggagcc 960
   gcaggggctc gaggtaatga tggagctcga ggaagtgatg gacaaccggg cccccctggt 1020
   cctcctggaa ctgcaggatt ccctggttcc cctggtgcta agggtgaagt tggacctgca 1080
   ggatctcctg gttcaagtgg cgcccctgga caaagaggag aacctggacc tcagggacat 1140
   gctggtgctc caggtccccc tgggcctcct gggagtaatg gtagtcctgg tggcaaaggt 1200
   gaaatgggtc ctgctggcat tcctggggct cctgggctga taggagctcg tggtcctcca 1260
   gggccacctg gcaccaatgg tgttcccggg caacgaggtg ctgcaggtga acccggtaag 1320
   aatggagcca aaggagaccc aggaccacgt ggggaacgcg gagaagctgg ttctccaggt 1380
   atcgcaggac ctaagggtga agatggcaaa gatggttctc ctggagaacc tggtgcaaat 1440
   ggacttcctg gagctgcagg agaaaggggt gtgcctggat tccgaggacc tgctggagca 1500
   aatggccttc caggagaaaa gggtcctcct ggggaccgtg gtggcccagg ccctgcaggg 1560
   cccagaggtg ttgctggaga gcccggcaga gatggtctcc ctggaggtcc aggattgagg 1620
   ggtattcctg gtagcccggg aggaccaggc agtgatggga aaccagggcc tcctggaagc 1680
   caaggagaga cgggtcgacc cggtcctcca ggttcacctg gtccgcgagg ccagcctggt 1740
   gtcatgggct tccctggtcc caaaggaaac gatggtgctc ctggaaaaaa tggagaacga 1800
   ggtggccctg gaggtcctgg ccctcagggt cctgctggaa agaatggtga gaccggacct 1860
   cagggtcctc caggacctac tggcccttct ggtgacaaag gagacacagg accccctggt 1920
   ccacaaggac tacaaggctt gcctggaacg agtggtcccc caggagaaaa cggaaaacct 1980
   ggtgaacctg gtccaaaggg tgaggctggt gcacctggaa ttccaggagg caagggtgat 2040
   tctggtgctc ccggtgaacg cggacctcct ggagcaggag ggccccctgg acctagaggt 2100
   ggagctggcc cccctggtcc cgaaggagga aagggtgctg ctggtccccc tgggccacct 2160
   ggttctgctg gtacacctgg tctgcaagga atgcctggag aaagaggggg tcctggaggc 2220
   cctggtccaa agggtgataa gggtgagcct ggcagctcag gtgtcgatgg tgctccaggg 2280
   aaagatggtc cacggggtcc cactggtccc attggtcctc ctggcccagc tggtcagcct 2340
   ggagataagg gtgaaagtgg tgcccctgga gttccgggta tagctggtcc tcgcggtggc 2400
   cctggtgaga gaggcgaaca ggggccccca ggacctgctg gcttccctgg tgctcctggc 2460
   cagaatggtg agcctggtgc taaaggagaa agaggcgctc ctggtgagaa aggtgaagga 2520
   ggccctcccg gagccgcagg acccgccgga ggttctgggc ctgccggtcc cccaggcccc 2580
   caaggtgtca aaggcgaacg tggcagtcct ggtggtcctg gtgctgctgg cttccccggt 2640
   ggtcgtggtc ctcctggccc tcctggcagt aatggtaacc caggcccccc aggctccagt 2700
   ggtgctccag gcaaagatgg tcccccaggt ccacctggca gtaatggtgc tcctggcagc 2760
   cccgggatct ctggaccaaa gggtgattct ggtccaccag gtgagagggg agcacctggc 2820
   ccccaggggc ctccgggagc tccaggccca ctaggaattg caggacttac tggagcacga 2880
   ggtcttgcag gcccaccagg catgccaggt gctaggggca gccccggccc acagggcatc 2940
   aagggtgaaa atggtaaacc aggacctagt ggtcagaatg gagaacgtgg tcctcctggc 3000
   ccccagggtc ttcctggtct ggctggtaca gctggtgagc ctggaagaga tggaaaccct 3060
   ggatcagatg gtctgccagg ccgagatgga gcgccaggtg ccaagggtga ccgtggtgaa 3120
   aatggctctc ctggtgcccc tggagctcct ggtcacccag gccctcctgg tcctgtcggt 3180
   ccagctggaa agagcggtga cagaggagaa actggccctg ctggtccttc tggggccccc 3240
   ggtcctgccg gatcaagagg tcctcctggt ccccaaggcc cacgcggtga caaaggggaa 3300
   accggtgagc gtggtgctat gggcatcaaa ggacatcgcg gattccctgg caacccaggg 3360
   gcccccggat ctccgggtcc cgctggtcat caaggtgcag ttggcagtcc aggccctgca 3420
   ggccccagag gacctgttgg acctagcggg ccccctggaa aggacggagc aagtggacac 3480
   cctggtccca ttggaccacc ggggccccga ggtaacagag gtgaaagagg atctgagggc 3540
   tccccaggcc acccaggaca accaggccct cctggacctc ctggtgcccc tggtccatgt 3600
   tgtggtgctg gcggggttgc tgccattgct ggtgttggag ccgaaaaagc tggtggtttt 3660
   gccccatatt atggagatga accgatagat ttcaaaatca ataccgatga gattatgacc 3720
   tcactcaaat cagtcaatgg acaaatagaa agcctcatta gtcctgatgg ttcccgtaaa 3780
   aaccctgcac ggaactgcag ggacctgaaa ttctgccatc ctgaactcca gagtggagaa 3840
   tattgggttg atcctaacca aggttgcaaa ttggatgcta ttaaagtcta ctgtaacatg 3900
   gaaactgggg aaacgtgcat aagtgccagt cctttgacta tcccacagaa gaactggtgg 3960
   acagattctg gtgctgagaa gaaacatgtt tggtttggag aatccatgga gggtggtttt 4020
   cagtttagct atggcaatcc tgaacttccc gaagacgtcc tcgatgtcca gctggcattc 4080
   ctccgacttc tctccagccg ggcctctcag aacatcacat atcactgcaa gaatagcatt 4140
   gcatacatgg atcatgccag tgggaatgta aagaaagcct tgaagctgat ggggtcaaat 4200
   gaaggtgaat tcaaggctga aggaaatagc aaattcacat acacagttct ggaggatggt 4260
   tgcacaaaac acactgggga atggggcaaa acagtcttcc agtatcaaac acgcaaggcc 4320
   gtcagactac ctattgtaga tattgcaccc tatgatatcg gtggtcctga tcaagaattt 4380
   ggtgcggaca ttggccctgt ttgcttttta taaaccaaac ctgaattc 4428
<210> 4
   <211> 1466
   <212> PRT
   <213> bovine
<400> 4
   Met Met Ser Phe Val Gln Lys Gly Thr Trp Leu Leu Phe Ala Leu Leu
   1 5 10 15
   His Pro Thr Val Ile Leu Ala Gln Gln Glu Ala Val Asp Gly Gly Cys
   20 25 30
   Ser His Leu Gly Gln Ser Tyr Ala Asp Arg Asp Val Trp Lys Pro Glu
   35 40 45
   Pro Cys Gln Ile Cys Val Cys Asp Ser Gly Ser Val Leu Cys Asp Asp
   50 55 60
   Ile Ile Cys Asp Asp Gln Glu Leu Asp Cys Pro Asn Pro Glu Ile Pro
   65 70 75 80
   Phe Gly Glu Cys Cys Ala Val Cys Pro Gln Pro Pro Thr Ala Pro Thr
   85 90 95
   Arg Pro Pro Asn Gly Gln Gly Pro Gln Gly Pro Lys Gly Asp Pro Gly
   100 105 110
   Pro Pro Gly Ile Pro Gly Arg Asn Gly Asp Pro Gly Pro Pro Gly Ser
   115 120 125
   Pro Gly Ser Pro Gly Ser Pro Gly Pro Pro Gly Ile Cys Glu Ser Cys
   130 135 140
   Pro Thr Gly Gly Gln Asn Tyr Ser Pro Gln Tyr Glu Ala Tyr Asp Val
   145 150 155 160
   Lys Ser Gly Val Ala Gly Gly Gly Ile Ala Gly Tyr Pro Gly Pro Ala
   165 170 175
   Gly Pro Pro Gly Pro Pro Gly Pro Pro Gly Thr Ser Gly His Pro Gly
   180 185 190
   Ala Pro Gly Ala Pro Gly Tyr Gln Gly Pro Pro Gly Glu Pro Gly Gln
   195 200 205
   Ala Gly Pro Ala Gly Pro Pro Gly Pro Pro Gly Ala Ile Gly Pro Ser
   210 215 220
   Gly Pro Ala Gly Lys Asp Gly Glu Ser Gly Arg Pro Gly Arg Pro Gly
   225 230 235 240
   Glu Arg Gly Phe Pro Gly Pro Pro Gly Met Lys Gly Pro Ala Gly Met
   245 250 255
   Pro Gly Phe Pro Gly Met Lys Gly His Arg Gly Phe Asp Gly Arg Asn
   260 265 270
   Gly Glu Lys Gly Glu Thr Gly Ala Pro Gly Leu Lys Gly Glu Asn Gly
   275 280 285
   Val Pro Gly Glu Asn Gly Ala Pro Gly Pro Met Gly Pro Arg Gly Ala
   290 295 300
   Pro Gly Glu Arg Gly Arg Pro Gly Leu Pro Gly Ala Ala Gly Ala Arg
   305 310 315 320
   Gly Asn Asp Gly Ala Arg Gly Ser Asp Gly Gln Pro Gly Pro Pro Gly
   325 330 335
   Pro Pro Gly Thr Ala Gly Phe Pro Gly Ser Pro Gly Ala Lys Gly Glu
   340 345 350
   Val Gly Pro Ala Gly Ser Pro Gly Ser Ser Gly Ala Pro Gly Gln Arg
   355 360 365
   Gly Glu Pro Gly Pro Gln Gly His Ala Gly Ala Pro Gly Pro Pro Gly
   370 375 380
   Pro Pro Gly Ser Asn Gly Ser Pro Gly Gly Lys Gly Glu Met Gly Pro
   385 390 395 400
   Ala Gly Ile Pro Gly Ala Pro Gly Leu Ile Gly Ala Arg Gly Pro Pro
   405 410 415
   Gly Pro Pro Gly Thr Asn Gly Val Pro Gly Gln Arg Gly Ala Ala Gly
   420 425 430
   Glu Pro Gly Lys Asn Gly Ala Lys Gly Asp Pro Gly Pro Arg Gly Glu
   435 440 445
   Arg Gly Glu Ala Gly Ser Pro Gly Ile Ala Gly Pro Lys Gly Glu Asp
   450 455 460
   Gly Lys Asp Gly Ser Pro Gly Glu Pro Gly Ala Asn Gly Leu Pro Gly
   465 470 475 480
   Ala Ala Gly Glu Arg Gly Val Pro Gly Phe Arg Gly Pro Ala Gly Ala
   485 490 495
   Asn Gly Leu Pro Gly Glu Lys Gly Pro Pro Gly Asp Arg Gly Gly Pro
   500 505 510
   Gly Pro Ala Gly Pro Arg Gly Val Ala Gly Glu Pro Gly Arg Asp Gly
   515 520 525
   Leu Pro Gly Gly Pro Gly Leu Arg Gly Ile Pro Gly Ser Pro Gly Gly
   530 535 540
   Pro Gly Ser Asp Gly Lys Pro Gly Pro Pro Gly Ser Gln Gly Glu Thr
   545 550 555 560
   Gly Arg Pro Gly Pro Pro Gly Ser Pro Gly Pro Arg Gly Gln Pro Gly
   565 570 575
   Val Met Gly Phe Pro Gly Pro Lys Gly Asn Asp Gly Ala Pro Gly Lys
   580 585 590
   Asn Gly Glu Arg Gly Gly Pro Gly Gly Pro Gly Pro Gln Gly Pro Ala
   595 600 605
   Gly Lys Asn Gly Glu Thr Gly Pro Gln Gly Pro Pro Gly Pro Thr Gly
   610 615 620
   Pro Ser Gly Asp Lys Gly Asp Thr Gly Pro Pro Gly Pro Gln Gly Leu
   625 630 635 640
   Gln Gly Leu Pro Gly Thr Ser Gly Pro Pro Gly Glu Asn Gly Lys Pro
   645 650 655
   Gly Glu Pro Gly Pro Lys Gly Glu Ala Gly Ala Pro Gly Ile Pro Gly
   660 665 670
   Gly Lys Gly Asp Ser Gly Ala Pro Gly Glu Arg Gly Pro Pro Gly Ala
   675 680 685
   Gly Gly Pro Pro Gly Pro Arg Gly Gly Ala Gly Pro Pro Gly Pro Glu
   690 695 700
   Gly Gly Lys Gly Ala Ala Gly Pro Pro Gly Pro Pro Gly Ser Ala Gly
   705 710 715 720
   Thr Pro Gly Leu Gln Gly Met Pro Gly Glu Arg Gly Gly Pro Gly Gly
   725 730 735
   Pro Gly Pro Lys Gly Asp Lys Gly Glu Pro Gly Ser Ser Gly Val Asp
   740 745 750
   Gly Ala Pro Gly Lys Asp Gly Pro Arg Gly Pro Thr Gly Pro Ile Gly
   755 760 765
   Pro Pro Gly Pro Ala Gly Gln Pro Gly Asp Lys Gly Glu Ser Gly Ala
   770 775 780
   Pro Gly Val Pro Gly Ile Ala Gly Pro Arg Gly Gly Pro Gly Glu Arg
   785 790 795 800
   Gly Glu Gln Gly Pro Pro Gly Pro Ala Gly Phe Pro Gly Ala Pro Gly
   805 810 815
   Gln Asn Gly Glu Pro Gly Ala Lys Gly Glu Arg Gly Ala Pro Gly Glu
   820 825 830
   Lys Gly Glu Gly Gly Pro Pro Gly Ala Ala Gly Pro Ala Gly Gly Ser
   835 840 845
   Gly Pro Ala Gly Pro Pro Gly Pro Gln Gly Val Lys Gly Glu Arg Gly
   850 855 860
   Ser Pro Gly Gly Pro Gly Ala Ala Gly Phe Pro Gly Gly Arg Gly Pro
   865 870 875 880
   Pro Gly Pro Pro Gly Ser Asn Gly Asn Pro Gly Pro Pro Gly Ser Ser
   885 890 895
   Gly Ala Pro Gly Lys Asp Gly Pro Pro Gly Pro Pro Gly Ser Asn Gly
   900 905 910
   Ala Pro Gly Ser Pro Gly Ile Ser Gly Pro Lys Gly Asp Ser Gly Pro
   915 920 925
   Pro Gly Glu Arg Gly Ala Pro Gly Pro Gln Gly Pro Pro Gly Ala Pro
   930 935 940
   Gly Pro Leu Gly Ile Ala Gly Leu Thr Gly Ala Arg Gly Leu Ala Gly
   945 950 955 960
   Pro Pro Gly Met Pro Gly Ala Arg Gly Ser Pro Gly Pro Gln Gly Ile
   965 970 975
   Lys Gly Glu Asn Gly Lys Pro Gly Pro Ser Gly Gln Asn Gly Glu Arg
   980 985 990
   Gly Pro Pro Gly Pro Gln Gly Leu Pro Gly Leu Ala Gly Thr Ala Gly
   995 1000 1005
   Glu Pro Gly Arg Asp Gly Asn Pro Gly Ser Asp Gly Leu Pro Gly Arg
   1010 1015 1020
   Asp Gly Ala Pro Gly Ala Lys Gly Asp Arg Gly Glu Asn Gly Ser Pro
   1025 1030 1035 1040
   Gly Ala Pro Gly Ala Pro Gly His Pro Gly Pro Pro Gly Pro Val Gly
   1045 1050 1055
   Pro Ala Gly Lys Ser Gly Asp Arg Gly Glu Thr Gly Pro Ala Gly Pro
   1060 1065 1070
   Ser Gly Ala Pro Gly Pro Ala Gly Ser Arg Gly Pro Pro Gly Pro Gln
   1075 1080 1085
   Gly Pro Arg Gly Asp Lys Gly Glu Thr Gly Glu Arg Gly Ala Met Gly
   1090 1095 1100
   Ile Lys Gly His Arg Gly Phe Pro Gly Asn Pro Gly Ala Pro Gly Ser
   1105 1110 1115 1120
   Pro Gly Pro Ala Gly His Gln Gly Ala Val Gly Ser Pro Gly Pro Ala
   1125 1130 1135
   Gly Pro Arg Gly Pro Val Gly Pro Ser Gly Pro Pro Gly Lys Asp Gly
   1140 1145 1150
   Ala Ser Gly His Pro Gly Pro Ile Gly Pro Pro Gly Pro Arg Gly Asn
   1155 1160 1165
   Arg Gly Glu Arg Gly Ser Glu Gly Ser Pro Gly His Pro Gly Gln Pro
   1170 1175 1180
   Gly Pro Pro Gly Pro Pro Gly Ala Pro Gly Pro Cys Cys Gly Ala Gly
   1185 1190 1195 1200
   Gly Val Ala Ala Ile Ala Gly Val Gly Ala Glu Lys Ala Gly Gly Phe
   1205 1210 1215
   Ala Pro Tyr Tyr Gly Asp Glu Pro Ile Asp Phe Lys Ile Asn Thr Asp
   1220 1225 1230
   Glu Ile Met Thr Ser Leu Lys Ser Val Asn Gly Gln Ile Glu Ser Leu
   1235 1240 1245
   Ile Ser Pro Asp Gly Ser Arg Lys Asn Pro Ala Arg Asn Cys Arg Asp
   1250 1255 1260
   Leu Lys Phe Cys His Pro Glu Leu Gln Ser Gly Glu Tyr Trp Val Asp
   1265 1270 1275 1280
   Pro Asn Gln Gly Cys Lys Leu Asp Ala Ile Lys Val Tyr Cys Asn Met
   1285 1290 1295
   Glu Thr Gly Glu Thr Cys Ile Ser Ala Ser Pro Leu Thr Ile Pro Gln
   1300 1305 1310
   Lys Asn Trp Trp Thr Asp Ser Gly Ala Glu Lys Lys His Val Trp Phe
   1315 1320 1325
   Gly Glu Ser Met Glu Gly Gly Phe Gln Phe Ser Tyr Gly Asn Pro Glu
   1330 1335 1340
   Leu Pro Glu Asp Val Leu Asp Val Gln Leu Ala Phe Leu Arg Leu Leu
   1345 1350 1355 1360
   Ser Ser Arg Ala Ser Gln Asn Ile Thr Tyr His Cys Lys Asn Ser Ile
   1365 1370 1375
   Ala Tyr Met Asp His Ala Ser Gly Asn Val Lys Lys Ala Leu Lys Leu
   1380 1385 1390
   Met Gly Ser Asn Glu Gly Glu Phe Lys Ala Glu Gly Asn Ser Lys Phe
   1395 1400 1405
   Thr Tyr Thr Val Leu Glu Asp Gly Cys Thr Lys His Thr Gly Glu Trp
   1410 1415 1420
   Gly Lys Thr Val Phe Gln Tyr Gln Thr Arg Lys Ala Val Arg Leu Pro
   1425 1430 1435 1440
   Ile Val Asp Ile Ala Pro Tyr Asp Ile Gly Gly Pro Asp Gln Glu Phe
   1445 1450 1455
   Gly Ala Asp Ile Gly Pro Val Cys Phe Leu
   1460 1465
<210> 5
   <211> 4428
   <212> DNA
   <213> bovine
<400> 5
   gaattcaggg acatgatgag ctttgtgcaa aaggggacct ggttactttt cgctctgctt 60
   catcccactg ttattttggc acaacaggaa gctgttgacg gaggatgctc ccatctcggt 120
   cagtcttatg cagatagaga tgtatggaaa ccagaaccgt gccaaatatg cgtctgtgac 180
   tcaggatccg ttctctgtga tgacataata tgtgacgacc aagaattaga ctgccccaac 240
   cctgaaatcc cgtttggaga atgttgtgca gtttgcccac agcctccaac agctcccact 300
   cgccctccta atggtcaagg acctcaaggc cccaagggag atccaggtcc tcctggtatt 360
   cctgggcgaa atggcgatcc tggtcctcca ggatcaccag gctccccagg ttctcccggc 420
   cctcctggaa tctgtgaatc atgtcctact ggtggccaga actattctcc ccagtacgaa 480
   gcatatgatg tcaagtctgg agtagcagga ggaggaatcg caggctatcc tgggccagct 540
   ggtcctcctg gcccacccgg accccctggc acatctggcc atcctggtgc ccctggcgct 600
   ccaggatacc aaggtccccc cggtgaacct gggcaagctg gtccggcagg tcctccagga 660
   cctcctggtg ctataggtcc atctggccct gctggaaaag atggggaatc aggaagaccc 720
   ggacgacctg gagagcgagg atttcctggc cctcctggta tgaaaggccc agctggtatg 780
   cctggattcc ctggtatgaa aggacacaga ggctttgatg gacgaaatgg agagaaaggc 840
   gaaactggtg ctcctggatt aaagggggaa aatggcgttc caggtgaaaa tggagctcct 900
   ggacccatgg gtccaagagg ggctcccggt gagagaggac ggccaggact tcctggagcc 960
   gcaggggctc gaggtaatga tggagctcga ggaagtgatg gacaaccggg cccccctggt 1020
   cctcctggaa ctgcaggatt ccctggttcc cctggtgcta agggtgaagt tggacctgca 1080
   ggatctcctg gttcaagtgg cgcccctgga caaagaggag aacctggacc tcagggacat 1140
   gctggtgctc caggtccccc tgggcctcct gggagtaatg gtagtcctgg tggcaaaggt 1200
   gaaatgggtc ctgctggcat tcctggggct cctgggctga taggagctcg tggtcctcca 1260
   gggccacctg gcaccaatgg tgttcccggg caacgaggtg ctgcaggtga acccggtaag 1320
   aatggagcca aaggagaccc aggaccacgt ggggaacgcg gagaagctgg ttctccaggt 1380
   atcgcaggac ctaagggtga agatggcaaa gatggttctc ctggagaacc tggtgcaaat 1440
   ggacttcctg gagctgcagg agaaaggggt gtgcctggat tccgaggacc tgctggagca 1500
   aatggccttc caggagaaaa gggtcctcct ggggaccgtg gtggcccagg ccctgcaggg 1560
   cccagaggtg ttgctggaga gcccggcaga gatggtctcc ctggaggtcc aggattgagg 1620
   ggtattcctg gtagcccggg aggaccaggc agtgatggga aaccagggcc tcctggaagc 1680
   caaggagaga cgggtcgacc cggtcctcca ggttcacctg gtccgcgagg ccagcctggt 1740
   gtcatgggct tccctggtcc caaaggaaac gatggtgctc ctggaaaaaa tggagaacga 1800
   ggtggccctg gaggtcctgg ccctcagggt cctgctggaa agaatggtga gaccggacct 1860
   cagggtcctc caggacctac tggcccttct ggtgacaaag gagacacagg accccctggt 1920
   ccacaaggac tacaaggctt gcctggaacg agtggtcccc caggagaaaa cggaaaacct 1980
   ggtgaacctg gtccaaaggg tgaggctggt gcacctggaa ttccaggagg caagggtgat 2040
   tctggtgctc ccggtgaacg cggacctcct ggagcaggag ggccccctgg acctagaggt 2100
   ggagctggcc cccctggtcc cgaaggagga aagggtgctg ctggtccccc tgggccacct 2160
   ggttctgctg gtacacctgg tctgcaagga atgcctggag aaagaggggg tcctggaggc 2220
   cctggtccaa agggtgataa gggtgagcct ggcagctcag gtgtcgatgg tgctccaggg 2280
   aaagatggtc cacggggtcc cactggtccc attggtcctc ctggcccagc tggtcagcct 2340
   ggagataagg gtgaaagtgg tgcccctgga gttccgggta tagctggtcc tcgcggtggc 2400
   cctggtgaga gaggcgaaca ggggccccca ggacctgctg gcttccctgg tgctcctggc 2460
   cagaatggtg agcctggtgc taaaggagaa agaggcgctc ctggtgagaa aggtgaagga 2520
   ggccctcccg gagccgcagg acccgccgga ggttctgggc ctgccggtcc cccaggcccc 2580
   caaggtgtca aaggcgaacg tggcagtcct ggtggtcctg gtgctgctgg cttccccggt 2640
   ggtcgtggtc ctcctggccc tcctggcagt aatggtaacc caggcccccc aggctccagt 2700
   ggtgctccag gcaaagatgg tcccccaggt ccacctggca gtaatggtgc tcctggcagc 2760
   cccgggatct ctggaccaaa gggtgattct ggtccaccag gtgagagggg agcacctggc 2820
   ccccaggggc ctccgggagc tccaggccca ctaggaattg caggacttac tggagcacga 2880
   ggtcttgcag gcccaccagg catgccaggt gctaggggca gccccggccc acagggcatc 2940
   aagggtgaaa atggtaaacc aggacctagt ggtcagaatg gagaacgtgg tcctcctggc 3000
   ccccagggtc ttcctggtct ggctggtaca gctggtgagc ctggaagaga tggaaaccct 3060
   ggatcagatg gtctgccagg ccgagatgga gcgccaggtg ccaagggtga ccgtggtgaa 3120
   aatggctctc ctggtgcccc tggagctcct ggtcacccag gccctcctgg tcctgtcggt 3180
   ccagctggaa agagcggtga cagaggagaa actggccctg ctggtccttc tggggccccc 3240
   ggtcctgccg gatcaagagg tcctcctggt ccccaaggcc cacgcggtga caaaggggaa 3300
   accggtgagc gtggtgctat gggcatcaaa ggacatcgcg gattccctgg caacccaggg 3360
   gcccccggat ctccgggtcc cgctggtcat caaggtgcag ttggcagtcc aggccctgca 3420
   ggccccagag gacctgttgg acctagcggg ccccctggaa aggacggagc aagtggacac 3480
   cctggtccca ttggaccacc ggggccccga ggtaacagag gtgaaagagg atctgagggc 3540
   tccccaggcc acccaggaca accaggccct cctggacctc ctggtgcccc tggtccatgt 3600
   tgtggtgctg gcggggttgc tgccattgct ggtgttggag ccgaaaaagc tggtggtttt 3660
   gccccatatt atggagatga accgatagat ttcaaaatca acaccaatga gattatgacc 3720
   tcactcaaat cagtcaatgg acaaatagaa agcctcatta gtcctgatgg ttcccgtaaa 3780
   aaccctgcac ggaactgcag ggacctgaaa ttctgccatc ctgaactcca gagtggagaa 3840
   tattgggttg atcctaacca aggttgcaaa ttggatgcta ttaaagtcta ctgtaacatg 3900
   gaaactgggg aaacgtgcat aagtgccagt cctttgacta tcccacagaa gaactggtgg 3960
   acagattctg gtgctgagaa gaaacatgtt tggtttggag aatccatgga gggtggtttt 4020
   cagtttagct atggcaatcc tgaacttccc gaagacgtcc tcgatgtcca gctggcattc 4080
   ctccgacttc tctccagccg ggcctctcag aacatcacat atcactgcaa gaatagcatt 4140
   gcatacatgg atcatgtcag tgggaatgta aagaaagcct tgaagctgat ggggtcaaat 4200
   gaaggtgaat tcaaggctga aggaaatagc aaattcacat acacagttct ggaggatggt 4260
   tgcacaaaac acactgggga atggggcaaa acagtcttcc agtatcaaac acgcaaggcc 4320
   gtcagactac ctattgtaga tattgcaccc tatgatatcg gtggtcctga tcaagaattt 4380
   ggtgcggaca ttggccctgt ttgcttttta taaaccaaac ctgaattc             4428
<210> 6
   <211> 1466
   <212> PRT
   <213> porcine
<400> 6
   Met Met Ser Phe Val Gln Lys Gly Thr Trp Leu Leu Phe Ala Leu Leu
   1 5 10 15
   His Pro Thr Val Ile Leu Ala Gln Gln Glu Ala Val Asp Gly Gly Cys
   20 25 30
   Ser His Leu Gly Gln Ser Tyr Ala Asp Arg Asp Val Trp Lys Pro Glu
   35 40 45
   Pro Cys Gln Ile Cys Val Cys Asp Ser Gly Ser Val Leu Cys Asp Asp
   50 55 60
   Ile Ile Cys Asp Asp Gln Glu Leu Asp Cys Pro Asn Pro Glu Ile Pro
   65 70 75 80
   Phe Gly Glu Cys Cys Ala Val Cys Pro Gln Pro Pro Thr Ala Pro Thr
   85 90 95
   Arg Pro Pro Asn Gly Gln Gly Pro Gln Gly Pro Lys Gly Asp Pro Gly
   100 105 110
   Pro Pro Gly Ile Pro Gly Arg Asn Gly Asp Pro Gly Pro Pro Gly Ser
   115 120 125
   Pro Gly Ser Pro Gly Ser Pro Gly Pro Pro Gly Ile Cys Glu Ser Cys
   130 135 140
   Pro Thr Gly Gly Gln Asn Tyr Ser Pro Gln Tyr Glu Ala Tyr Asp Val
   145 150 155 160
   Lys Ser Gly Val Ala Gly Gly Gly Ile Ala Gly Tyr Pro Gly Pro Ala
   165 170 175
   Gly Pro Pro Gly Pro Pro Gly Pro Pro Gly Thr Ser Gly His Pro Gly
   180 185 190
   Ala Pro Gly Ala Pro Gly Tyr Gln Gly Pro Pro Gly Glu Pro Gly Gln
   195 200 205
   Ala Gly Pro Ala Gly Pro Pro Gly Pro Pro Gly Ala Ile Gly Pro Ser
   210 215 220
   Gly Pro Ala Gly Lys Asp Gly Glu Ser Gly Arg Pro Gly Arg Pro Gly
   225 230 235 240
   Glu Arg Gly Phe Pro Gly Pro Pro Gly Met Lys Gly Pro Ala Gly Met
   245 250 255
   Pro Gly Phe Pro Gly Met Lys Gly His Arg Gly Phe Asp Gly Arg Asn
   260 265 270
   Gly Glu Lys Gly Glu Thr Gly Ala Pro Gly Leu Lys Gly Glu Asn Gly
   275 280 285
   Val Pro Gly Glu Asn Gly Ala Pro Gly Pro Met Gly Pro Arg Gly Ala
   290 295 300
   Pro Gly Glu Arg Gly Arg Pro Gly Leu Pro Gly Ala Ala Gly Ala Arg
   305                 310                 315                 320
   Gly Asn Asp Gly Ala Arg Gly Ser Asp Gly Gln Pro Gly Pro Pro Gly
                   325                 330                 335
   Pro Pro Gly Thr Ala Gly Phe Pro Gly Ser Pro Gly Ala Lys Gly Glu
               340                 345                 350
   Val Gly Pro Ala Gly Ser Pro Gly Ser Ser Gly Ala Pro Gly Gln Arg
           355                 360                 365
   Gly Glu Pro Gly Pro Gln Gly His Ala Gly Ala Pro Gly Pro Pro Gly
       370                 375                 380
   Pro Pro Gly Ser Asn Gly Ser Pro Gly Gly Lys Gly Glu Met Gly Pro
   385                 390                 395                 400
   Ala Gly Ile Pro Gly Ala Pro Gly Leu Ile Gly Ala Arg Gly Pro Pro
                   405                 410                 415
   Gly Pro Pro Gly Thr Asn Gly Val Pro Gly Gln Arg Gly Ala Ala Gly
               420                 425                 430
   Glu Pro Gly Lys Asn Gly Ala Lys Gly Asp Pro Gly Pro Arg Gly Glu
           435                 440                 445
   Arg Gly Glu Ala Gly Ser Pro Gly Ile Ala Gly Pro Lys Gly Glu Asp
       450                 455                 460
   Gly Lys Asp Gly Ser Pro Gly Glu Pro Gly Ala Asn Gly Leu Pro Gly
   465                 470                 475                 480
   Ala Ala Gly Glu Arg Gly Val Pro Gly Phe Arg Gly Pro Ala Gly Ala
                   485                 490                 495
   Asn Gly Leu Pro Gly Glu Lys Gly Pro Pro Gly Asp Arg Gly Gly Pro
               500                 505                 510
   Gly Pro Ala Gly Pro Arg Gly Val Ala Gly Glu Pro Gly Arg Asp Gly
           515                 520                 525
   Leu Pro Gly Gly Pro Gly Leu Arg Gly Ile Pro Gly Ser Pro Gly Gly
       530                 535                 540
   Pro Gly Ser Asp Gly Lys Pro Gly Pro Pro Gly Ser Gln Gly Glu Thr
   545                 550                 555                 560
   Gly Arg Pro Gly Pro Pro Gly Ser Pro Gly Pro Arg Gly Gln Pro Gly
                   565                 570                 575
   Val Met Gly Phe Pro Gly Pro Lys Gly Asn Asp Gly Ala Pro Gly Lys
               580                 585                 590
   Asn Gly Glu Arg Gly Gly Pro Gly Gly Pro Gly Pro Gln Gly Pro Ala
           595                 600                 605
   Gly Lys Asn Gly Glu Thr Gly Pro Gln Gly Pro Pro Gly Pro Thr Gly
       610                 615                 620
   Pro Ser Gly Asp Lys Gly Asp Thr Gly Pro Pro Gly Pro Gln Gly Leu
   625 630 635 640
   Gln Gly Leu Pro Gly Thr Ser Gly Pro Pro Gly Glu Asn Gly Lys Pro
   645 650 655
   Gly Glu Pro Gly Pro Lys Gly Glu Ala Gly Ala Pro Gly Ile Pro Gly
   660 665 670
   Gly Lys Gly Asp Ser Gly Ala Pro Gly Glu Arg Gly Pro Pro Gly Ala
   675 680 685
   Gly Gly Pro Pro Gly Pro Arg Gly Gly Ala Gly Pro Pro Gly Pro Glu
   690 695 700
   Gly Gly Lys Gly Ala Ala Gly Pro Pro Gly Pro Pro Gly Ser Ala Gly
   705 710 715 720
   Thr Pro Gly Leu Gln Gly Met Pro Gly Glu Arg Gly Gly Pro Gly Gly
   725 730 735
   Pro Gly Pro Lys Gly Asp Lys Gly Glu Pro Gly Ser Ser Gly Val Asp
   740 745 750
   Gly Ala Pro Gly Lys Asp Gly Pro Arg Gly Pro Thr Gly Pro Ile Gly
   755 760 765
   Pro Pro Gly Pro Ala Gly Gln Pro Gly Asp Lys Gly Glu Ser Gly Ala
   770 775 780
   Pro Gly Val Pro Gly Ile Ala Gly Pro Arg Gly Gly Pro Gly Glu Arg
   785 790 795 800
   Gly Glu Gln Gly Pro Pro Gly Pro Ala Gly Phe Pro Gly Ala Pro Gly
   805 810 815
   Gln Asn Gly Glu Pro Gly Ala Lys Gly Glu Arg Gly Ala Pro Gly Glu
   820 825 830
   Lys Gly Glu Gly Gly Pro Pro Gly Ala Ala Gly Pro Ala Gly Gly Ser
   835 840 845
   Gly Pro Ala Gly Pro Pro Gly Pro Gln Gly Val Lys Gly Glu Arg Gly
   850 855 860
   Ser Pro Gly Gly Pro Gly Ala Ala Gly Phe Pro Gly Gly Arg Gly Pro
   865 870 875 880
   Pro Gly Pro Pro Gly Ser Asn Gly Asn Pro Gly Pro Pro Gly Ser Ser
   885 890 895
   Gly Ala Pro Gly Lys Asp Gly Pro Pro Gly Pro Pro Gly Ser Asn Gly
   900 905 910
   Ala Pro Gly Ser Pro Gly Ile Ser Gly Pro Lys Gly Asp Ser Gly Pro
   915 920 925
   Pro Gly Glu Arg Gly Ala Pro Gly Pro Gln Gly Pro Pro Gly Ala Pro
   930 935 940
   Gly Pro Leu Gly Ile Ala Gly Leu Thr Gly Ala Arg Gly Leu Ala Gly
   945 950 955 960
   Pro Pro Gly Met Pro Gly Ala Arg Gly Ser Pro Gly Pro Gln Gly Ile
   965 970 975
   Lys Gly Glu Asn Gly Lys Pro Gly Pro Ser Gly Gln Asn Gly Glu Arg
   980 985 990
   Gly Pro Pro Gly Pro Gln Gly Leu Pro Gly Leu Ala Gly Thr Ala Gly
   995 1000 1005
   Glu Pro Gly Arg Asp Gly Asn Pro Gly Ser Asp Gly Leu Pro Gly Arg
   1010 1015 1020
   Asp Gly Ala Pro Gly Ala Lys Gly Asp Arg Gly Glu Asn Gly Ser Pro
   1025 1030 1035 1040
   Gly Ala Pro Gly Ala Pro Gly His Pro Gly Pro Pro Gly Pro Val Gly
   1045 1050 1055
   Pro Ala Gly Lys Ser Gly Asp Arg Gly Glu Thr Gly Pro Ala Gly Pro
   1060 1065 1070
   Ser Gly Ala Pro Gly Pro Ala Gly Ser Arg Gly Pro Pro Gly Pro Gln
   1075 1080 1085
   Gly Pro Arg Gly Asp Lys Gly Glu Thr Gly Glu Arg Gly Ala Met Gly
   1090 1095 1100
   Ile Lys Gly His Arg Gly Phe Pro Gly Asn Pro Gly Ala Pro Gly Ser
   1105 1110 1115 1120
   Pro Gly Pro Ala Gly His Gln Gly Ala Val Gly Ser Pro Gly Pro Ala
   1125 1130 1135
   Gly Pro Arg Gly Pro Val Gly Pro Ser Gly Pro Pro Gly Lys Asp Gly
   1140 1145 1150
   Ala Ser Gly His Pro Gly Pro Ile Gly Pro Pro Gly Pro Arg Gly Asn
   1155 1160 1165
   Arg Gly Glu Arg Gly Ser Glu Gly Ser Pro Gly His Pro Gly Gln Pro
   1170 1175 1180
   Gly Pro Pro Gly Pro Pro Gly Ala Pro Gly Pro Cys Cys Gly Ala Gly
   1185 1190 1195 1200
   Gly Val Ala Ala Ile Ala Gly Val Gly Ala Glu Lys Ala Gly Gly Phe
   1205 1210 1215
   Ala Pro Tyr Tyr Gly Asp Glu Pro Ile Asp Phe Lys Ile Asn Thr Asn
   1220 1225 1230
   Glu Ile Met Thr Ser Leu Lys Ser Val Asn Gly Gln Ile Glu Ser Leu
   1235 1240 1245
   Ile Ser Pro Asp Gly Ser Arg Lys Asn Pro Ala Arg Asn Cys Arg Asp
   1250 1255 1260
   Leu Lys Phe Cys His Pro Glu Leu Gln Ser Gly Glu Tyr Trp Val Asp
   1265 1270 1275 1280
   Pro Asn Gln Gly Cys Lys Leu Asp Ala Ile Lys Val Tyr Cys Asn Met
   1285 1290 1295
   Glu Thr Gly Glu Thr Cys Ile Ser Ala Ser Pro Leu Thr Ile Pro Gln
   1300 1305 1310
   Lys Asn Trp Trp Thr Asp Ser Gly Ala Glu Lys Lys His Val Trp Phe
   1315 1320 1325
   Gly Glu Ser Met Glu Gly Gly Phe Gln Phe Ser Tyr Gly Asn Pro Glu
   1330 1335 1340
   Leu Pro Glu Asp Val Leu Asp Val Gln Leu Ala Phe Leu Arg Leu Leu
   1345 1350 1355 1360
   Ser Ser Arg Ala Ser Gln Asn Ile Thr Tyr His Cys Lys Asn Ser Ile
   1365 1370 1375
   Ala Tyr Met Asp His Val Ser Gly Asn Val Lys Lys Ala Leu Lys Leu
   1380 1385 1390
   Met Gly Ser Asn Glu Gly Glu Phe Lys Ala Glu Gly Asn Ser Lys Phe
   1395 1400 1405
   Thr Tyr Thr Val Leu Glu Asp Gly Cys Thr Lys His Thr Gly Glu Trp
   1410 1415 1420
   Gly Lys Thr Val Phe Gln Tyr Gln Thr Arg Lys Ala Val Arg Leu Pro
   1425 1430 1435 1440
   Ile Val Asp Ile Ala Pro Tyr Asp Ile Gly Gly Pro Asp Gln Glu Phe
   1445 1450 1455
   Gly Ala Asp Ile Gly Pro Val Cys Phe Leu
   1460 1465
<210> 7
   <211> 4425
   <212> DNA
   <213> porcine
<400> 7
   gaattcaggg acatgttcag ctttgtggac ctccggctcc tgctcctctt agcggccacc 60
   gccctcctga cgcacggcca agaggagggc caagaagaag gccaacaagg ccaagaagaa 120
   gacatcccac cagtcacctg cgtacagaac ggcctcaggt accatgaccg agacgtgtgg 180
   aaacccgtgc cctgccagat ctgtgtctgc gacaacggca atgtgttgtg cgatgacgtg 240
   atctgcgacg aaatcaagaa ctgtcccagc gccagagtcc ctgcgggcga gtgctgcccc 300
   gtctgccccg aaggcgaggt gtcacccacc gaccaggaaa ccacgggagt cgagggaccc 360
   aagggagaca ctggcccccg aggccccagg ggaccctctg gcccccctgg ccgagacggc 420
   atccctggac aacctggact tcctggaccc cccggacctc ctggaccccc cggaccccct 480
   ggcctcggag gaaactttgc tccccagttg tcttatggct atgatgagaa gtcagcagga 540
   atttccgtgc ccggccccat gggtccttct ggtcctcgtg gtctctctgg cccccctggc 600
   gcacctggtc cccaaggttt ccaaggcccc cctggtgagc ctggcgagcc tggcgcctcc 660
   ggtcccatgg gtccccgtgg tcctcctggc ccccctggca agaacggaga tgatggtgaa 720
   gctggaaagc ctggtcgccc tggtgagcgt gggcctcctg gacctcaggg tgctcgggga 780
   ttgcccggaa cagctggcct ccctggaatg aagggacaca gaggtttcag tggtttggat 840
   ggtgccaagg gagatgctgg tcctgctggt cccaagggtg agcctggtag ccctggtgaa 900
   aatggagctc ctggtcagat gggcccccgt ggtctgcctg gtgagcgagg tcgccctgga 960
   ccccctggcc ctgctggtgc tcgtggaaat gatggtgcta ctggtgctgc tggaccccct 1020
   ggtcccactg gccccgctgg tcctcctggc ttccctggtg ctgttggtgc taagggtgaa 1080
   gctggtcccc aaggagcccg aggctctgaa ggtccccagg gtgtgcgtgg tgagcctggc 1140
   ccccctggcc ctgctggtgc tgctggccct gctggaaacc ctggtgctga tggacagcct 1200
   ggtggcaaag gtgccaacgg cgctcctggt attgctggtg ctcctggctt ccctggtgcc 1260
   cgaggcccct ctggacccca gggtcccagc ggcccccctg gtcccaaggg taacagcggt 1320
   gaacctggtg ctcccggcag caaaggagac actggcgcca agggagagcc cggtcccact 1380
   ggtgttcaag gaccccctgg ccctgctgga gaagaaggaa agcgaggagc ccgaggtgaa 1440
   cctggacctg ctggcctgcc tggaccccct ggcgagcgtg gtggacctgg tagccgtggt 1500
   ttccctggcg ccgatggtgt tgctggtccc aagggtcccg ctggtgaacg tggttctcct 1560
   ggccctgctg gtcccaaagg ttctcctggt gaagctggtc gccccggtga agctggtctg 1620
   cctggtgcca agggtctgac tggaagccct ggcagccctg gtcctgatgg caaaactggc 1680
   ccccctggtc ccgccggtca agatggtcgc cctggacccc caggccctcc tggtgcccgt 1740
   ggtcaggctg gtgtgatggg tttccctgga cctaaaggtg ctgctggaga gcctggcaaa 1800
   gctggagagc gaggtgttcc cggaccccct ggcgcagttg gtcctgctgg caaagatgga 1860
   gaagctggag ctcagggacc ccccggacct gctggccccg ctggtgagag aggagaacaa 1920
   ggccccgctg gctcccctgg attccagggt ctccctggcc ctgctggtcc tcctggtgaa 1980
   gcaggcaaac ccggtgaaca gggtgttcct ggagatctcg gtgcccccgg cccctctgga 2040
   gcaagaggcg agagaggttt ccccggcgag cgtggtgtgc aaggtccccc cggtcctgca 2100
   ggtccccgtg gagccaacgg tgcccctggc aatgatggtg ctaagggtga tgctggtgcc 2160
   cctggagccc ctggtagcca gggcgcccct ggccttcagg gaatgcctgg cgaacgaggt 2220
   gcagctggtc tcccaggtcc taagggtgac agaggagatg ctggtcccaa aggtgctgat 2280
   ggtgctcctg gcaaagatgg cgtccgtggt ctgactggcc ccattggtcc tcccggcccc 2340
   gctggtgccc ctggtgacaa gggtgaaact ggtcctagcg gtcctgctgg tcccactgga 2400
   gctcgtggtg cccccggtga ccgtggtgag cctggtcccc ccggccctgc tggcttcgct 2460
   ggcccccctg gtgctgatgg ccaacctggt gctaaaggcg aacctggtga tgctggtgct 2520
   aaaggcgatg ctggtccccc cggccctgct ggacccactg gcccccctgg ccccattggt 2580
   agcgttggtg ctcccggacc caaaggtgct cgtggcagcg ctggtcctcc tggtgctact 2640
   ggtttccctg gtgctgctgg ccgagtcggt ccccccggcc cctctggaaa tgctggaccc 2700
   cctggccctc ctggtcctgc tggcaaagaa ggcagcaaag gtccccgtgg tgagactggc 2760
   cccgctgggc gtcccggtga agccggtccc cctggccccc ctggccccgc tggtgagaaa 2820
   ggatcccctg gtgctgacgg acctgctggt gctcccggta ctcctggacc tcagggtatt 2880
   gctggacagc gtggtgtggt cggcctgccc ggtcaacgag gagaaagagg cttccctggt 2940
   cttcccggcc catctggtga acccggcaaa caaggtcctt ctggaccaag cggcgaacgt 3000
   ggcccccctg gtcccatggg cccccctgga ttggctggac cccctggcga gtctggacgt 3060
   gagggagccc ctggcgctga aggatcccct ggacgagatg gtgctcctgg ccccaagggt 3120
   gaccgtggtg agagcggccc tgctggaccc cctggtgctc ctggtgctcc tggtgccccc 3180
   ggccccgttg gccctgctgg caagagcggc gatcgtggtg agactggtcc tgctggtcct 3240
   gctggtcccg ttggccccgt tggtgcccgt ggccctgctg gaccccaagg cccccgtggt 3300
   gacaagggtg agacaggcga acagggcgac agaggcatta agggtcaccg tggcttctct 3360
   ggtctccagg gtccccctgg ccctcccggc tctcctggtg agcaaggtcc ctccggagct 3420
   tctggtcccg ctggtccccg aggtccccct ggctctgctg gtgctcctgg caaagatgga 3480
   ctcaacggtc tccccggccc catcggtccc cctgggcctc gtggtcgcac tggtgatgct 3540
   ggccctgttg gtcctcccgg ccctcctgga ccccccggtc cccctggtcc tcccagcggc 3600
   ggtttcgact tcagcttctt gccccagcca cctcaagaga aggctcacga tggtggccgc 3660
   tactaccggg ccgatgatgc caatgtggtc cgcgaccgtg acctcgaggt ggacaccacc 3720
   ctcaagagcc tgagccagca gatcgagaac atccggagcc ccgaaggcag ccgcaagaac 3780
   cccgcccgca cctgccgcga cctcaagatg tgccactccg actggaagag cggagaatac 3840
   tggattgacc ccaaccaagg ctgcaacctg gacgccatca aagtcttctg caacatggag 3900
   acaggcgaga cctgcgtgta ccccactcag cccagcgtgc cccagaagaa ctggtacatc 3960
   agcaagaacc ccaaggacaa gaggcacgtc tggtacggcg agagcatgac cgacggattc 4020
   cagttcgagt acggcggcga gggctccgat cctgctgacg tggccatcca gctgaccttc 4080
   ctgcgcctga tgtccactga ggcttcccag aacatcacct accactgcaa gaacagcgtg 4140
   gcctacatgg accagcagac tggcaacctc aagaaggccc tgctcctcca gggctccaac 4200
   gagatcgaga tccgggccga gggcaacagc cgcttcacct acagcgtgat ctacgacggc 4260
   tgcacgagtc acaccggagc ctggggcaag acagtgatcg aatacaaaac caccaagacc 4320
   tcccgcctgc ccatcatcga tgtggccccc ttggacgttg gcgcccccga ccaagaattc 4380
   ggcatcgacc ttagccctgt ctgcttcctg taaactcctg aattc 4425
<210> 8
   <211> 1449
   <212> PRT
   <213> porcine
<400> 8
   Met Phe Ser Phe Val Asp Leu Arg Leu Leu Leu Leu Leu Ala Ala Thr
   1               5                   10                  15
   Ala Leu Leu Thr His Gly Gln Glu Glu Gly Gln Glu Glu Gly Gln Gln
               20                  25                  30
   Gly Gln Glu Glu Asp Ile Pro Pro Val Thr Cys Val Gln Asn Gly Leu
           35                  40                  45
   Arg Tyr His Asp Arg Asp Val Trp Lys Pro Val Pro Cys Gln Ile Cys
       50                  55                  60
   Val Cys Asp Asn Gly Asn Val Leu Cys Asp Asp Val Ile Cys Asp Glu
   65                  70                  75                  80
   Ile Lys Asn Cys Pro Ser Ala Arg Val Pro Ala Gly Glu Cys Cys Pro
                   85                  90                  95
   Val Cys Pro Glu Gly Glu Val Ser Pro Thr Asp Gln Glu Thr Thr Gly
               100                 105                 110
   Val Glu Gly Pro Lys Gly Asp Thr Gly Pro Arg Gly Pro Arg Gly Pro
           115                 120                 125
   Ser Gly Pro Pro Gly Arg Asp Gly Ile Pro Gly Gln Pro Gly Leu Pro
       130                 135                 140
   Gly Pro Pro Gly Pro Pro Gly Pro Pro Gly Pro Pro Gly Leu Gly Gly
   145                 150                 155                 160
   Asn Phe Ala Pro Gln Leu Ser Tyr Gly Tyr Asp Glu Lys Ser Ala Gly
                   165                 170                 175
   Ile Ser Val Pro Gly Pro Met Gly Pro Ser Gly Pro Arg Gly Leu Ser
               180                 185                 190
   Gly Pro Pro Gly Ala Pro Gly Pro Gln Gly Phe Gln Gly Pro Pro Gly
           195                 200                 205
   Glu Pro Gly Glu Pro Gly Ala Ser Gly Pro Met Gly Pro Arg Gly Pro
       210                 215                 220
   Pro Gly Pro Pro Gly Lys Asn Gly Asp Asp Gly Glu Ala Gly Lys Pro
   225                 230                 235                 240
   Gly Arg Pro Gly Glu Arg Gly Pro Pro Gly Pro Gln Gly Ala Arg Gly
                   245                 250                 255
   Leu Pro Gly Thr Ala Gly Leu Pro Gly Met Lys Gly His Arg Gly Phe
               260                 265                 270
   Ser Gly Leu Asp Gly Ala Lys Gly Asp Ala Gly Pro Ala Gly Pro Lys
   275 280 285
   Gly Glu Pro Gly Ser Pro Gly Glu Asn Gly Ala Pro Gly Gln Met Gly
   290 295 300
   Pro Arg Gly Leu Pro Gly Glu Arg Gly Arg Pro Gly Pro Pro Gly Pro
   305 310 315 320
   Ala Gly Ala Arg Gly Asn Asp Gly Ala Thr Gly Ala Ala Gly Pro Pro
   325 330 335
   Gly Pro Thr Gly Pro Ala Gly Pro Pro Gly Phe Pro Gly Ala Val Gly
   340 345 350
   Ala Lys Gly Glu Ala Gly Pro Gln Gly Ala Arg Gly Ser Glu Gly Pro
   355 360 365
   Gln Gly Val Arg Gly Glu Pro Gly Pro Pro Gly Pro Ala Gly Ala Ala
   370 375 380
   Gly Pro Ala Gly Asn Pro Gly Ala Asp Gly Gln Pro Gly Gly Lys Gly
   385 390 395 400
   Ala Asn Gly Ala Pro Gly Ile Ala Gly Ala Pro Gly Phe Pro Gly Ala
   405 410 415
   Arg Gly Pro Ser Gly Pro Gln Gly Pro Ser Gly Pro Pro Gly Pro Lys
   420 425 430
   Gly Asn Ser Gly Glu Pro Gly Ala Pro Gly Ser Lys Gly Asp Thr Gly
   435 440 445
   Ala Lys Gly Glu Pro Gly Pro Thr Gly Val Gln Gly Pro Pro Gly Pro
   450 455 460
   Ala Gly Glu Glu Gly Lys Arg Gly Ala Arg Gly Glu Pro Gly Pro Ala
   465 470 475 480
   Gly Leu Pro Gly Pro Pro Gly Glu Arg Gly Gly Pro Gly Ser Arg Gly
   485 490 495
   Phe Pro Gly Ala Asp Gly Val Ala Gly Pro Lys Gly Pro Ala Gly Glu
   500 505 510
   Arg Gly Ser Pro Gly Pro Ala Gly Pro Lys Gly Ser Pro Gly Glu Ala
   515 520 525
   Gly Arg Pro Gly Glu Ala Gly Leu Pro Gly Ala Lys Gly Leu Thr Gly
   530 535 540
   Ser Pro Gly Ser Pro Gly Pro Asp Gly Lys Thr Gly Pro Pro Gly Pro
   545 550 555 560
   Ala Gly Gln Asp Gly Arg Pro Gly Pro Pro Gly Pro Pro Gly Ala Arg
   565 570 575
   Gly Gln Ala Gly Val Met Gly Phe Pro Gly Pro Lys Gly Ala Ala Gly
   580 585 590
   Glu Pro Gly Lys Ala Gly Glu Arg Gly Val Pro Gly Pro Pro Gly Ala
   595 600 605
   Val Gly Pro Ala Gly Lys Asp Gly Glu Ala Gly Ala Gln Gly Pro Pro
   610 615 620
   Gly Pro Ala Gly Pro Ala Gly Glu Arg Gly Glu Gln Gly Pro Ala Gly
   625 630 635 640
   Ser Pro Gly Phe Gln Gly Leu Pro Gly Pro Ala Gly Pro Pro Gly Glu
   645 650 655
   Ala Gly Lys Pro Gly Glu Gln Gly Val Pro Gly Asp Leu Gly Ala Pro
   660 665 670
   Gly Pro Ser Gly Ala Arg Gly Glu Arg Gly Phe Pro Gly Glu Arg Gly
   675 680 685
   Val Gln Gly Pro Pro Gly Pro Ala Gly Pro Arg Gly Ala Asn Gly Ala
   690 695 700
   Pro Gly Asn Asp Gly Ala Lys Gly Asp Ala Gly Ala Pro Gly Ala Pro
   705 710 715 720
   Gly Ser Gln Gly Ala Pro Gly Leu Gln Gly Met Pro Gly Glu Arg Gly
   725 730 735
   Ala Ala Gly Leu Pro Gly Pro Lys Gly Asp Arg Gly Asp Ala Gly Pro
   740 745 750
   Lys Gly Ala Asp Gly Ala Pro Gly Lys Asp Gly Val Arg Gly Leu Thr
   755 760 765
   Gly Pro Ile Gly Pro Pro Gly Pro Ala Gly Ala Pro Gly Asp Lys Gly
   770 775 780
   Glu Thr Gly Pro Ser Gly Pro Ala Gly Pro Thr Gly Ala Arg Gly Ala
   785 790 795 800
   Pro Gly Asp Arg Gly Glu Pro Gly Pro Pro Gly Pro Ala Gly Phe Ala
   805 810 815
   Gly Pro Pro Gly Ala Asp Gly Gln Pro Gly Ala Lys Gly Gly Pro Thr
   820 825 830
   Gly Pro Pro Gly Pro Ile Gly Ser Val Gly Ala Pro Gly Pro Lys Gly
   835 840 845
   Ala Arg Gly Ser Ala Gly Pro Pro Gly Ala Thr Gly Phe Pro Gly Ala
   850 855 860
   Ala Gly Arg Val Gly Pro Pro Gly Pro Ser Gly Asn Ala Gly Pro Pro
   865 870 875 880
   Gly Pro Pro Gly Pro Ala Gly Lys Glu Gly Ser Lys Gly Pro Arg Gly
   885 890 895
   Glu Thr Gly Pro Ala Gly Arg Pro Gly Glu Ala Gly Pro Pro Gly Pro
   900 905 910
   Pro Gly Pro Ala Gly Glu Lys Gly Ser Pro Gly Ala Asp Gly Pro Ala
   915 920 925
   Gly Ala Pro Gly Thr Pro Gly Pro Gln Gly Ile Ala Gly Gln Arg Gly
   930 935 940
   Val Val Gly Leu Pro Gly Gln Arg Gly Glu Arg Gly Phe Pro Gly Leu
   945 950 955 960
   Pro Gly Pro Ser Gly Glu Pro Gly Lys Gln Gly Pro Ser Gly Pro Ser
   965 970 975
   Gly Glu Arg Gly Pro Pro Gly Pro Met Gly Pro Pro Gly Leu Ala Gly
   980 985 990
   Pro Pro Gly Glu Ser Gly Arg Glu Gly Ala Pro Gly Ala Glu Gly Ser
   995 1000 1005
   Pro Gly Arg Asp Gly Ala Pro Gly Pro Lys Gly Asp Arg Gly Glu Ser
   1010 1015 1020
   Gly Pro Ala Gly Pro Pro Gly Ala Pro Gly Ala Pro Gly Ala Pro Gly
   1025 1030 1035 1040
   Pro Val Gly Pro Ala Gly Lys Ser Gly Asp Arg Gly Glu Thr Gly Pro
   1045 1050 1055
   Ala Gly Pro Ala Gly Pro Val Gly Pro Val Gly Ala Arg Gly Pro Ala
   1060 1065 1070
   Gly Pro Gln Gly Pro Arg Gly Asp Lys Gly Glu Thr Gly Glu Gln Gly
   1075 1080 1085
   Asp Arg Gly Ile Lys Gly His Arg Gly Phe Ser Gly Leu Gln Gly Pro
   1090 1095 1100
   Pro Gly Pro Pro Gly Ser Pro Gly Glu Gln Gly Pro Ser Gly Ala Ser
   1105 1110 1115 1120
   Gly Pro Ala Gly Pro Arg Gly Pro Pro Gly Ser Ala Gly Ala Pro Gly
   1125 1130 1135
   Lys Asp Gly Leu Asn Gly Leu Pro Gly Pro Ile Gly Pro Pro Gly Pro
   1140 1145 1150
   Arg Gly Arg Thr Gly Asp Ala Gly Pro Val Gly Pro Pro Gly Pro Pro
   1155 1160 1165
   Gly Pro Pro Gly Pro Pro Gly Pro Pro Ser Gly Gly Phe Asp Phe Ser
   1170 1175 1180
   Phe Leu Pro Gln Pro Pro Gln Glu Lys Ala His Asp Gly Gly Arg Tyr
   1185 1190 1195 1200
   Tyr Arg Ala Asp Asp Ala Asn Val Val Arg Asp Arg Asp Leu Glu Val
   1205 1210 1215
   Asp Thr Thr Leu Lys Ser Leu Ser Gln Gln Ile Glu Asn Ile Arg Ser
   1220 1225 1230
   Pro Glu Gly Ser Arg Lys Asn Pro Ala Arg Thr Cys Arg Asp Leu Lys
   1235 1240 1245
   Met Cys His Ser Asp Trp Lys Ser Gly Glu Tyr Trp Ile Asp Pro Asn
   1250 1255 1260
   Gln Gly Cys Asn Leu Asp Ala Ile Lys Val Phe Cys Asn Met Glu Thr
   1265 1270 1275 1280
   Gly Glu Thr Cys Val Tyr Pro Thr Gln Pro Ser Val Pro Gln Lys Asn
   1285 1290 1295
   Trp Tyr Ile Ser Lys Asn Pro Lys Asp Lys Arg His Val Trp Tyr Gly
   1300 1305 1310
   Glu Ser Met Thr Asp Gly Phe Gln Phe Glu Tyr Gly Gly Glu Gly Ser
   1315 1320 1325
   Asp Pro Ala Asp Val Ala Ile Gln Leu Thr Phe Leu Arg Leu Met Ser
   1330 1335 1340
   Thr Glu Ala Ser Gln Asn Ile Thr Tyr His Cys Lys Asn Ser Val Ala
   1345 1350 1355 1360
   Tyr Met Asp Gln Gln Thr Gly Asn Leu Lys Lys Ala Leu Leu Leu Gln
   1365 1370 1375
   Gly Ser Asn Glu Ile Glu Ile Arg Ala Glu Gly Asn Ser Arg Phe Thr
   1380 1385 1390
   Tyr Ser Val Ile Tyr Asp Gly Cys Thr Ser His Thr Gly Ala Trp Gly
   1395 1400 1405
   Lys Thr Val Ile Glu Tyr Lys Thr Thr Lys Thr Ser Arg Leu Pro Ile
   1410 1415 1420
   Ile Asp Val Ala Pro Leu Asp Val Gly Ala Pro Asp Gln Glu Phe Gly
   1425 1430 1435 1440
   Ile Asp Leu Ser Pro Val Cys Phe Leu
   1445
<210> 9
   <211> 4498
   <212> DNA
   <213> porcine
<400> 9
   gaattcaggg acatgctcag ctttgtggat acgcggactt tgttgctgct tgcagtaact 60
   tcgtgcctag caacatgcca atctttacaa gaggcaactg caagaaaggg cccaactgga 120
   gatagaggac cacgcggaga aaggggtcca ccaggcccac caggcagaga tggtgatgat 180
   ggtatcccag gccctcctgg tccacctggt cctcctggcc cccctggtct tggcgggaac 240
   tttgctgctc agtatgatgg aaaaggagtt ggagctggcc ctggaccaat gggtttgatg 300
   ggacctaggg gccctcctgg ggcagttgga gcccctggcc ctcaaggttt ccaaggacct 360
   gctggtgagc ctggcgaacc tggtcagact ggtcctgctg gtgctcgtgg tccacctggc 420
   cctcctggca aggctggtga ggatggtcac cctggaaaac ccggacgacc tggtgagaga 480
   ggagttgttg gaccacaggg tgctcgtggt ttccctggaa ctcctggact tcctggcttc 540
   aagggcatta ggggtcacaa cggtctggat ggattgaagg gacagcccgg tgctccaggt 600
   gtgaagggcg aacctggtgc ccccggcgaa aatggaactc caggtcaaac aggagctcgc 660
   gggcttcctg gtgagagagg acgtgtcggt gctcctggcc cagctggtgc ccgtggaaat 720
   gatggaagtg tgggtcctgt gggtcctgct ggtcccattg ggtctgctgg ccctccaggc 780
   ttcccaggtg ctcctggccc caagggtgaa cttggacctg ttggtaaccc tggtcctgca 840
   ggtcctgcgg gtccccgtgg tgaagtgggt cttccaggtg tttctggccc tgttggacct 900
   cctggcaacc ctggagccaa cggccttcct ggtgctaaag gtgctgctgg cctgcttggt 960
   gttgctgggg ctcctggcct ccctgggcct cgaggtattc ctggccctgc tggtgctgct 1020
   ggtgctactg gtgccagagg tcttgttggt gagcctggtc cagctggttc caaaggagag 1080
   agcggcaaca agggcgagcc tggtgctgct gggccccaag gtcctcctgg tcccagtggt 1140
   gaagaaggaa agagaggccc caatggagaa gttggatctg ctggcccccc aggacctcct 1200
   gggctgaggg gaaatcctgg ttctcgtggt ctccctggag ctgatggcag agctggtgtc 1260
   atgggccctc ctggtagtcg tggtccaact ggccctgctg gtgttcgagg tcccaatgga 1320
   gattctggtc gccctggaga gcctggcctt atgggacccc gaggtttccc tggatcccct 1380
   ggaaatgttg gtccagctgg taaagaaggt cctgcgggcc tccctggtat tgatggcagg 1440
   cctggaccaa ttggcccagc tggagcaaga ggagagcctg gcaacattgg attccctgga 1500
   cccaaaggcc ccactggtga tcctggcaaa aatggtgaaa aaggtcatgc tggtctggct 1560
   ggtgctcggg gtgccccagg tcctgatgga aacaatggtg ctcagggacc tcctggacca 1620
   cagggtgttc aaggtggaaa aggtgaacaa ggtcccgctg gtcctccagg cttccagggt 1680
   ctccctggcc ccgcaggtac agctggtgaa gttggcaaac caggagaaag gggtatccct 1740
   ggtgaatttg gtctccctgg tcctgctggt ccaagagggg agcgtggtcc cccaggtgaa 1800
   agtggtgctg ctggtcctgc tggtcctatt ggaagccgag gtccttctgg acccccgggg 1860
   cctgatggca acaagggcga acctggtgtg cttggtgctc caggcactgc tggtccatct 1920
   ggtcctagtg gactcccagg agagaggggt gctgctggca tacctggagg caagggagaa 1980
   aagggtgaaa ctggtctcag aggtgacgtt ggtagccctg gcagagatgg tgctcgtggt 2040
   gctcctggtg ctgtaggtgc ccctggtcct gctggagcca atggggaccg gggtgaagct 2100
   ggccctgctg gccctgctgg ccctgctggt cctcgtggta gtcctggtga acgtggtgag 2160
   gttggtcctg ctggccccaa tggatttgct ggtcctgctg gtgctgccgg tcaacctggt 2220
   gctaaaggag agagaggaac caaagggccc aaaggtgaaa atggtcctgt tggtcccaca 2280
   ggccctgttg gagctgctgg cccagctggt ccaaatggtc ctcctggtcc tgctggcagt 2340
   cgtggtgatg gcggcccccc tggtgctact ggtttccctg gtgctgctgg acggattggt 2400
   cctcctggac cttctggtat ctctgggccc cctggacccc ctggtcctgc tgggaaagaa 2460
   ggacttcgtg ggcctcgtgg tgaccaaggt ccagttggtc gaactggaga aacaggtgca 2520
   tctggccccc ctggctttgc tggtgagaaa ggtccctctg gagagcctgg tactgctgga 2580
   cctcctggta ccccaggtcc tcaaggtatt cttggtgctc ctggttttct gggtctccct 2640
   ggctctagag gtgaacgtgg tctaccaggt gttgctggat cagtgggtga acctggcccc 2700
   ctcggcattg caggcccacc tggggcccgt ggtccccctg gtgctgtggg taatcctggt 2760
   gtcaatggtg ctcctggtga agctggtcgt gatggcaacc ctggaagcga tggtccccca 2820
   ggccgagatg gtcaagctgg acacaagggc gagcgtggtt accctggtaa tcctggtcct 2880
   gctggtgctg caggagcacc tggtcctcaa ggtgctgtgg gtcccgctgg caaacatgga 2940
   aaccgtggtg aacctggtcc tgctggttct gttggtcctg ctggtgctgt tggtccaaga 3000
   ggtcctagtg gcccacaagg tattcgaggt gagaagggag agcctggtga taaggggccc 3060
   agaggtcttc ctggcttgaa gggacacaac ggattgcaag gtcttcctgg tcttgctggt 3120
   catcatggtg atcaaggtgc tcctggccct gtgggtcctg ctggtcctag gggtccagct 3180
   ggtccttctg gccctgctgg caaagatggt cgcactggac aacctggtgc agttggacct 3240
   gctggcattc gtggctctca aggaagccaa ggtcctgctg gtcctcctgg tcctcctggc 3300
   cctcctggac cacctggccc aagtggtggt ggttatgatt ttggatatga aggagacttc 3360
   tacagggctg accagcctcg ctcaccacct tctctcagac ccaaggatta tgaagttgat 3420
   gctactctga aatctctcaa caaccagatt gagactctac ttactccaga aggctctagg 3480
   aagaacccag ctcgcacatg ccgtgacttg agactcagcc acccagaatg gagtagtggt 3540
   tactactgga ttgaccctaa ccaaggatgt actatggatg ctatcaaagt atactgtgat 3600
   ttctctactg gtgaaacctg cattcgggct caacctgaaa acatcccagc caaaaactgg 3660
   tacagaaact ccaaggtcaa gaagcacgtc tggttaggag aaactatcaa tggtggtacc 3720
   cagtttgaat ataatatgga aggagttacc accaaggaaa tggctacaca acttgccttc 3780
   atgcgcctgc tggccaacca tgcctcccaa aacatcacct accattgcaa gaacagcatt 3840
   gcatacatgg atgaagagac tggcaacctg aaaaaggctg tcattctgca aggatccaat 3900
   gatgttgaac ttgttgccga gggcaacagc agattcacct acactgttct tgtagatggc 3960
   tgttctaaaa aaacaaatga atggagaaaa acaatcattg aatataaaac aaataagcca 4020
   tctcgcctgc ctatccttga tattgcacct ttggacatcg gtgatgctga ccaagaagtc 4080
   agtgtggacg ttggcccagt ctgtttcaaa taaatgaact caacctaaat taaagaaaaa 4140
   ggaaatctga aaaatttctc tctttgccat ttctttttct tctttttaac tgaaagctga 4200
   atcattccat ttcttctgca catctacttg cttaaattgt gggcaaaaga gaaggagaag 4260
   gattgatcag agcatcgtgc aatacaatta attcgttccc tgtccctctt cccctcccca 4320
   aaagatttgg aatttttttc aacattctaa cacctgttgt ggaaaatgtc aacctttgta 4380
   agaaaaccaa aaataaaaat tgaaaaataa aataaaaacc atgaacattt gcaccacttg 4440
   tggcttttga atatcttcca cagagggaag tttaaaaccc aaacttccac ctgaattc   4498
<210> 10
   <211> 1366
   <212> PRT
   <213> porcine
<400> 10
   Met Leu Ser Phe Val Asp Thr Arg Thr Leu Leu Leu Leu Ala Val Thr
    1               5                  10                  15
   Ser Cys Leu Ala Thr Cys Gln Ser Leu Gln Glu Ala Thr Ala Arg Lys
               20                  25                  30
   Gly Pro Thr Gly Asp Arg Gly Pro Arg Gly Glu Arg Gly Pro Pro Gly
           35                  40                  45
   Pro Pro Gly Arg Asp Gly Asp Asp Gly Ile Pro Gly Pro Pro Gly Pro
       50                  55                  60
   Pro Gly Pro Pro Gly Pro Pro Gly Leu Gly Gly Asn Phe Ala Ala Gln
   65                  70                  75                  80
   Tyr Asp Gly Lys Gly Val Gly Ala Gly Pro Gly Pro Met Gly Leu Met
                   85                  90                  95
   Gly Pro Arg Gly Pro Pro Gly Ala Val Gly Ala Pro Gly Pro Gln Gly
               100                 105                 110
   Phe Gln Gly Pro Ala Gly Glu Pro Gly Glu Pro Gly Gln Thr Gly Pro
           115                 120                 125
   Ala Gly Ala Arg Gly Pro Pro Gly Pro Pro Gly Lys Ala Gly Glu Asp
       130                 135                 140
   Gly His Pro Gly Lys Pro Gly Arg Pro Gly Glu Arg Gly Val Val Gly
   145                 150                 155                 160
   Pro Gln Gly Ala Arg Gly Phe Pro Gly Thr Pro Gly Leu Pro Gly Phe
                   165                 170                 175
   Lys Gly Ile Arg Gly His Asn Gly Leu Asp Gly Leu Lys Gly Gln Pro
               180                 185                 190
   Gly Ala Pro Gly Val Lys Gly Glu Pro Gly Ala Pro Gly Glu Asn Gly
           195                 200                 205
   Thr Pro Gly Gln Thr Gly Ala Arg Gly Leu Pro Gly Glu Arg Gly Arg
       210                 215                 220
   Val Gly Ala Pro Gly Pro Ala Gly Ala Arg Gly Asn Asp Gly Ser Val
   225                 230                 235                 240
   Gly Pro Val Gly Pro Ala Gly Pro Ile Gly Ser Ala Gly Pro Pro Gly
                   245                 250                 255
   Phe Pro Gly Ala Pro Gly Pro Lys Gly Glu Leu Gly Pro Val Gly Asn
               260                 265                 270
   Pro Gly Pro Ala Gly Pro Ala Gly Pro Arg Gly Glu Val Gly Leu Pro
           275                 280                 285
   Gly Val Ser Gly Pro Val Gly Pro Pro Gly Asn Pro Gly Ala Asn Gly
       290                 295                 300
   Leu Pro Gly Ala Lys Gly Ala Ala Gly Leu Leu Gly Val Ala Gly Ala
   305                 310                 315                 320
   Pro Gly Leu Pro Gly Pro Arg Gly Ile Pro Gly Pro Ala Gly Ala Ala
                   325                 330                 335
   Gly Ala Thr Gly Ala Arg Gly Leu Val Gly Glu Pro Gly Pro Ala Gly
               340                 345                 350
   Ser Lys Gly Glu Ser Gly Asn Lys Gly Glu Pro Gly Ala Ala Gly Pro
           355                 360                 365
   Gln Gly Pro Pro Gly Pro Ser Gly Glu Glu Gly Lys Arg Gly Pro Asn
       370                 375                 380
   Gly Glu Val Gly Ser Ala Gly Pro Pro Gly Pro Pro Gly Leu Arg Gly
   385                 390                 395                 400
   Asn Pro Gly Ser Arg Gly Leu Pro Gly Ala Asp Gly Arg Ala Gly Val
                   405                 410                 415
   Met Gly Pro Pro Gly Ser Arg Gly Pro Thr Gly Pro Ala Gly Val Arg
               420                 425                 430
   Gly Pro Asn Gly Asp Ser Gly Arg Pro Gly Glu Pro Gly Leu Met Gly
           435                 440                 445
   Pro Arg Gly Phe Pro Gly Ser Pro Gly Asn Val Gly Pro Ala Gly Lys
       450                 455                 460
   Glu Gly Pro Ala Gly Leu Pro Gly Ile Asp Gly Arg Pro Gly Pro Ile
   465                 470                 475                 480
   Gly Pro Ala Gly Ala Arg Gly Glu Pro Gly Asn Ile Gly Phe Pro Gly
                   485                 490                 495
   Pro Lys Gly Pro Thr Gly Asp Pro Gly Lys Asn Gly Glu Lys Gly His
               500                 505                 510
   Ala Gly Leu Ala Gly Ala Arg Gly Ala Pro Gly Pro Asp Gly Asn Asn
           515                 520                 525
   Gly Ala Gln Gly Pro Pro Gly Pro Gln Gly Val Gln Gly Gly Lys Gly
       530                 535                 540
   Glu Gln Gly Pro Ala Gly Pro Pro Gly Phe Gln Gly Leu Pro Gly Pro
   545                 550                 555                 560
   Ala Gly Thr Ala Gly Glu Val Gly Lys Pro Gly Glu Arg Gly Ile Pro
                   565                 570                 575
   Gly Glu Phe Gly Leu Pro Gly Pro Ala Gly Pro Arg Gly Glu Arg Gly
               580                 585                 590
   Pro Pro Gly Glu Ser Gly Ala Ala Gly Pro Ala Gly Pro Ile Gly Ser
           595                 600                 605
   Arg Gly Pro Ser Gly Pro Pro Gly Pro Asp Gly Asn Lys Gly Glu Pro
       610                 615                 620
   Gly Val Leu Gly Ala Pro Gly Thr Ala Gly Pro Ser Gly Pro Ser Gly
   625                 630                 635                 640
   Leu Pro Gly Glu Arg Gly Ala Ala Gly Ile Pro Gly Gly Lys Gly Glu
                   645                 650                 655
   Lys Gly Glu Thr Gly Leu Arg Gly Asp Val Gly Ser Pro Gly Arg Asp
               660                 665                 670
   Gly Ala Arg Gly Ala Pro Gly Ala Val Gly Ala Pro Gly Pro Ala Gly
           675                 680                 685
   Ala Asn Gly Asp Arg Gly Glu Ala Gly Pro Ala Gly Pro Ala Gly Pro
       690                 695                 700
   Ala Gly Pro Arg Gly Ser Pro Gly Glu Arg Gly Glu Val Gly Pro Ala
   705                 710                 715                 720
   Gly Pro Asn Gly Phe Ala Gly Pro Ala Gly Ala Ala Gly Gln Pro Gly
                   725                 730                 735
   Ala Lys Gly Glu Arg Gly Thr Lys Gly Pro Lys Gly Glu Asn Gly Pro
               740                 745                 750
   Val Gly Pro Thr Gly Pro Val Gly Ala Ala Gly Pro Ala Gly Pro Asn
           755                 760                 765
   Gly Pro Pro Gly Pro Ala Gly Ser Arg Gly Asp Gly Gly Pro Pro Gly
       770                 775                 780
   Ala Thr Gly Phe Pro Gly Ala Ala Gly Arg Ile Gly Pro Pro Gly Pro
   785                 790                 795                 800
   Ser Gly Ile Ser Gly Pro Pro Gly Pro Pro Gly Pro Ala Gly Lys Glu
                   805                 810                 815
   Gly Leu Arg Gly Pro Arg Gly Asp Gln Gly Pro Val Gly Arg Thr Gly
               820                 825                 830
   Glu Thr Gly Ala Ser Gly Pro Pro Gly Phe Ala Gly Glu Lys Gly Pro
           835                 840                 845
   Ser Gly Glu Pro Gly Thr Ala Gly Pro Pro Gly Thr Pro Gly Pro Gln
       850                 855                 860
   Gly Ile Leu Gly Ala Pro Gly Phe Leu Gly Leu Pro Gly Ser Arg Gly
   865                 870                 875                 880
   Glu Arg Gly Leu Pro Gly Val Ala Gly Ser Val Gly Glu Pro Gly Pro
                   885                 890                 895
   Leu Gly Ile Ala Gly Pro Pro Gly Ala Arg Gly Pro Pro Gly Ala Val
               900                 905                 910
   Gly Asn Pro Gly Val Asn Gly Ala Pro Gly Glu Ala Gly Arg Asp Gly
           915                 920                 925
   Asn Pro Gly Ser Asp Gly Pro Pro Gly Arg Asp Gly Gln Ala Gly His
       930                 935                 940
   Lys Gly Glu Arg Gly Tyr Pro Gly Asn Pro Gly Pro Ala Gly Ala Ala
   945                 950                 955                 960
   Gly Ala Pro Gly Pro Gln Gly Ala Val Gly Pro Ala Gly Lys His Gly
                   965                 970                 975
   Asn Arg Gly Glu Pro Gly Pro Ala Gly Ser Val Gly Pro Ala Gly Ala
               980                 985                 990
   Val Gly Pro Arg Gly Pro Ser Gly Pro Gln Gly Ile Arg Gly Glu Lys
           995                 1000                1005
   Gly Glu Pro Gly Asp Lys Gly Pro Arg Gly Leu Pro Gly Leu Lys Gly
       1010                1015                1020
   His Asn Gly Leu Gln Gly Leu Pro Gly Leu Ala Gly His His Gly Asp
   1025                1030                1035                1040
   Gln Gly Ala Pro Gly Pro Val Gly Pro Ala Gly Pro Arg Gly Pro Ala
                   1045                1050                1055
   Gly Pro Ser Gly Pro Ala Gly Lys Asp Gly Arg Thr Gly Gln Pro Gly
               1060                1065                1070
   Ala Val Gly Pro Ala Gly Ile Arg Gly Ser Gln Gly Ser Gln Gly Pro
           1075                1080                1085
   Ala Gly Pro Pro Gly Pro Pro Gly Pro Pro Gly Pro Pro Gly Pro Ser
       1090                1095                1100
   Gly Gly Gly Tyr Asp Phe Gly Tyr Glu Gly Asp Phe Tyr Arg Ala Asp
   1105                1110                1115                1120
   Gln Pro Arg Ser Pro Pro Ser Leu Arg Pro Lys Asp Tyr Glu Val Asp
                   1125                1130                1135
   Ala Thr Leu Lys Ser Leu Asn Asn Gln Ile Glu Thr Leu Leu Thr Pro
               1140                1145                1150
   Glu Gly Ser Arg Lys Asn Pro Ala Arg Thr Cys Arg Asp Leu Arg Leu
           1155                1160                1165
   Ser His Pro Glu Trp Ser Ser Gly Tyr Tyr Trp Ile Asp Pro Asn Gln
       1170                1175                1180
   Gly Cys Thr Met Asp Ala Ile Lys Val Tyr Cys Asp Phe Ser Thr Gly
   1185                1190                1195                1200
   Glu Thr Cys Ile Arg Ala Gln Pro Glu Asn Ile Pro Ala Lys Asn Trp
                   1205                1210                1215
   Tyr Arg Asn Ser Lys Val Lys Lys His Val Trp Leu Gly Glu Thr Ile
               1220                1225                1230
   Asn Gly Gly Thr Gln Phe Glu Tyr Asn Met Glu Gly Val Thr Thr Lys
           1235                1240                1245
   Glu Met Ala Thr Gln Leu Ala Phe Met Arg Leu Leu Ala Asn His Ala
       1250                1255                1260
   Ser Gln Asn Ile Thr Tyr His Cys Lys Asn Ser Ile Ala Tyr Met Asp
   1265                1270                1275                1280
   Glu Glu Thr Gly Asn Leu Lys Lys Ala Val Ile Leu Gln Gly Ser Asn
                   1285                1290                1295
   Asp Val Glu Leu Val Ala Glu Gly Asn Ser Arg Phe Thr Tyr Thr Val
               1300                1305                1310
   Leu Val Asp Gly Cys Ser Lys Lys Thr Asn Glu Trp Arg Lys Thr Ile
           1315                1320                1325
   Ile Glu Tyr Lys Thr Asn Lys Pro Ser Arg Leu Pro Ile Leu Asp Ile
       1330                1335                1340
   Ala Pro Leu Asp Ile Gly Asp Ala Asp Gln Glu Val Ser Val Asp Val
   1345                1350                1355                1360
   Gly Pro Val Cys Phe Lys
                   1365
<210> 11
   <211> 4428
   <212> DNA
   <213> porcine
<400> 11
   gaattcaggg acatgatgag ctttgtgcaa aaggggacct ggttactttt tgctctactt 60
   catcccactg ttattttggc acaacaacag gaagctattg aaggaggatg ctcccatctt 120
   ggtcagtcct atgcggatag agatgtctgg aagccagaac catgtcaaat atgcgtctgt 180
   gactcaggat ctgttctctg cgatgatata atatgtgatg atcaagaatt agactgtccc 240
   aaccctgaga tcccatttgg agaatgttgt gcagtttgtc cacaacctcc aacagctccc 300
   acccgccctc ccaatggtca tggacctcaa ggccccaagg gagatccagg ccctcctggt 360
   attcctggga gaaatggaga ccctggtctt ccaggacaac caggttcccc tggttctcct 420
   gggcctcctg gaatctgtga atcatgccct actggtggcc agaactattc tccccagtat 480
   gagtcatatg atgtcaaggc tggagtagca ggaggaggaa tcggaggcta tcctgggcca 540
   gcaggtcccc ctggcccacc tggtccccct ggtgtatctg gtcatcctgg tgcccctggt 600
   tctccaggat accaagggcc ccctggtgaa cctgggcaag ctggtcctgc aggtcctcca 660
   gggcctcctg gtgctatagg tccatctggt cctgccggaa aagatgggga gtcaggaaga 720
   cccggacgac ctggagaacg aggattgcct ggccctccag gtctcaaagg tccagctggc 780
   atgcctggat tccctggtat gaaagggcat agaggctttg atggacgaaa tggagaaaaa 840
   ggtgatacag gtgctcctgg gctgaagggt gaaaatggcc ttccaggtga aaatggagct 900
   cctggaccca tgggtccaag aggggctcct ggtgagcgag gacggccagg acttcctgga 960
   gctgcagggg ctcgaggtaa tgatggtgcc cgaggaagtg atggacaacc aggtccccct 1020
   ggtccccctg gaactgcagg attccctggt tcccctggtg ctaagggtga agttggaccc 1080
   gcgggatctc ctggtccaag tggatcccct ggacaaagag gagaacctgg acctcaggga 1140
   catgccggtg ctgcaggtcc tcctggccct cctgggagta atggtagtcc tggtggcaaa 1200
   ggtgaaatgg gtcctgctgg catccctgga gctcctggat tgatgggagc ccgtggtcct 1260
   ccaggaccac ctggtaccaa tggtgctcct gggcaacgag gtgcagcagg tgaacctggt 1320
   aaaaatgggg ccaaaggaga gccaggacca cgtggtgaac gtggggaagc tggttctccg 1380
   ggtattccag gacccaaggg tgaagatggc aaagatggtt ctcctggaga acctggtgca 1440
   aatggacttc caggagctgc aggagaaagg ggtatgcctg gattccgagg agctcctgga 1500
   gcaaatggcc ttccaggaga aaagggtccc gctggcgagc gcggtggtcc aggccccgca 1560
   ggccccagag gagttgccgg agaacctggc cgagatggtg ttcctggagg tccaggattg 1620
   aggggcatgc ccggtagccc cggaggacca ggcagtgatg ggaaaccagg acctcctgga 1680
   agtcagggag aaagtggtcg accaggtcct ccaggctcac ctggtccccg aggtcagcct 1740
   ggagtcatgg gcttccctgg tcctaaagga aatgacggtg ctcctggaaa gaatggagaa 1800
   agaggtggcc ctggaggtcc cggccttccg ggtcctcctg gaaagaatgg tgagacagga 1860
   cctcagggtc ccccaggacc tactgggcca ggtggtgaca aaggagacac aggaccccct 1920
   ggtcaacaag gattacaagg cttgcctgga accagtggtc ctccaggaga aaatggaaaa 1980
   cctggtgaac ccggcccaaa aggtgaagct ggtgcacctg gaattccagg aggcaagggt 2040
   gattctggtg cccccggtga acgtggacct cctggtgcag taggtccctc aggacctaga 2100
   ggtggagctg gcccccctgg tcccgaagga ggaaagggcc ctgctggtcc ccctgggccg 2160
   cctggtgccg ctggtacacc tggtctgcaa gggatgcctg gagaaagagg aggttctgga 2220
   ggccccggcc caaagggtga caagggtgac cctggcggtt caggtgctga tggtgctcca 2280
   ggaaaagatg gtccaagggg tcctactggt cccattggtc cccctggtcc agctggtcag 2340
   cctggagata agggtgaaag tggtgcccct ggacttcctg gtatagctgg tcctcgtggt 2400
   ggccctggtg agagaggtga acatgggcca ccaggacctg ccggcttccc tggtgctcct 2460
   ggccagaacg gtgagcctgg tgccaaagga gaaagaggcg ctcctggtga gaaaggtgaa 2520
   ggaggacctc ctgggattgc aggacagccc ggaggcactg ggcctcctgg tccccctggt 2580
   ccccaaggtg tcaaaggtga acgtggcagt cctggtggtc ctggtgctgc tgggttcccc 2640
   ggtggtcgtg gtcttcctgg tcctcctggc agtaacggta acccaggccc ccctggctcc 2700
   agtggtcctc caggcaaaga tggtccccca ggtccacctg gtagcagtgg tgctcctggc 2760
   agccctggag tatctggacc gaaaggtgat gccggtcaac caggtgaaaa aggatcacct 2820
   ggcccccagg gccctccggg agctccaggc ccaggtggaa tttcagggat tactggagca 2880
   cgaggtctcg caggcccacc aggcatgcca ggtgctaggg gaagccctgg cccacagggc 2940
   gtcaagggtg aaaatggaaa accaggacct agtggtctca atggagaacg tggtcctcct 3000
   ggaccccagg gtcttcctgg tctggctggt gcagctggtg aacctggacg agatggaaac 3060
   cctggatcag atggtctgcc aggccgagac ggagctcccg gtagcaaggg cgatcgtggt 3120
   gaaaatggct ctcctggtgc ccctggtgct cctggtcacc caggcccacc tggccctgtt 3180
   ggtcctgctg gaaagaatgg tgacagagga gaaactggcc ctgctggtcc tgctggtgct 3240
   ccaggtcctg ctggttcaag aggtgctcct ggtccccaag gcccacgcgg tgacaaaggt 3300
   gaaaccggtg aacgtggtgc taatggcatc aaaggacatc gaggattccc tggtaatcca 3360
   ggtgccccag gttctccagg tcccgctggt caccaaggtg cagtaggtag cccaggacct 3420
   gcaggcccca gaggacctgt tggaccgagt gggccccctg gcaaagatgg agcaagtgga 3480
   caccctggtc ccattggacc accagggcct cgaggtaaca gaggtgaaag aggatctgag 3540
   ggctccccag gccatccagg acaaccaggc cctcctggac cccctggtgc ccctggtcca 3600
   tgttgtggtg gtggggctgc tgccatcgct ggtgttggag gtgaaaaagc tggtggtttt 3660
   gccccatatt atggagatga accaatggat ttcaaaatca acaccgacga gattatgact 3720
   tcacttaaat ccgtcaacgg acaaatagaa agcctcatta gtcccgatgg ttctcgtaaa 3780
   aaccctgctc gtaactgcag agacctaaaa ttctgccatc ctgagctcaa gagcggagaa 3840
   tattgggttg atcctaacca aggctgcaaa atggatgcta ttaaagtatt ttgtaacatg 3900
   gaaactgggg aaacatgcat aagtgccagt ccttctactg ttccacgtaa gaactggtgg 3960
   acagattctg gtgctgagaa gaaatatgtt tggtttggag aatccatgaa tggtggtttt 4020
   cagtttagct atggcaatcc tgaacttcct gaagatgtcc ttgatgtcca gttggcattc 4080
   cttcgacttc tctctagccg agcttcccag aacatcacat atcactgcaa gaatagcatt 4140
   gcgtacatgg aacatgccag tgggaatgta aagaaagcct tgaggctgat gggatcaaat 4200
   gaaggtgaat tcaaggctga aggaaatagc aaattcacat acaccgttct ggaggatggt 4260
   tgcactaaac acactgggga atggggcaag acagtcttcg aatatcgaac acgcaaggct 4320
   gtgagactac ctattgtaga tattgcaccc tatgatattg gtggtcctga tcaagaattt 4380
   ggtgcggaca ttggccctgt ttgcttttta taaaccaaac ctgaattc             4428
<210> 12
   <211> 1466
   <212> PRT
   <213> porcine
<400> 12
   Met Met Ser Phe Val Gln Lys Gly Thr Trp Leu Leu Phe Ala Leu Leu
   1               5                   10                  15
   His Pro Thr Val Ile Leu Ala Gln Gln Gln Glu Ala Ile Glu Gly Gly
               20                  25                  30
   Cys Ser His Leu Gly Gln Ser Tyr Ala Asp Arg Asp Val Trp Lys Pro
           35                  40                  45
   Glu Pro Cys Gln Ile Cys Val Cys Asp Ser Gly Ser Val Leu Cys Asp
       50                  55                  60
   Asp Ile Ile Cys Asp Asp Gln Glu Leu Asp Cys Pro Asn Pro Glu Ile
   65                  70                  75                  80
   Pro Phe Gly Glu Cys Cys Ala Val Cys Pro Gln Pro Pro Thr Ala Pro
                   85                  90                  95
   Thr Arg Pro Pro Asn Gly His Gly Pro Gln Gly Pro Lys Gly Asp Pro
               100                 105                 110
   Gly Pro Pro Gly Ile Pro Gly Arg Asn Gly Asp Pro Gly Leu Pro Gly
           115                 120                 125
   Gln Pro Gly Ser Pro Gly Ser Pro Gly Pro Pro Gly Ile Cys Glu Ser
       130                 135                 140
   Cys Pro Thr Gly Gly Gln Asn Tyr Ser Pro Gln Tyr Glu Ser Tyr Asp
   145                 150                 155                 160
   Val Lys Ala Gly Val Ala Gly Gly Gly Ile Gly Gly Tyr Pro Gly Pro
                   165                 170                 175
   Ala Gly Pro Pro Gly Pro Pro Gly Pro Pro Gly Val Ser Gly His Pro
               180                 185                 190
   Gly Ala Pro Gly Ser Pro Gly Tyr Gln Gly Pro Pro Gly Glu Pro Gly
           195                 200                 205
   Gln Ala Gly Pro Ala Gly Pro Pro Gly Pro Pro Gly Ala Ile Gly Pro
       210                 215                 220
   Ser Gly Pro Ala Gly Lys Asp Gly Glu Ser Gly Arg Pro Gly Arg Pro
   225                 230                 235                 240
   Gly Glu Arg Gly Leu Pro Gly Pro Pro Gly Leu Lys Gly Pro Ala Gly
                   245                 250                 255
   Met Pro Gly Phe Pro Gly Met Lys Gly His Arg Gly Phe Asp Gly Arg
               260                 265                 270
   Asn Gly Glu Lys Gly Asp Thr Gly Ala Pro Gly Leu Lys Gly Glu Asn
           275                 280                 285
   Gly Leu Pro Gly Glu Asn Gly Ala Pro Gly Pro Met Gly Pro Arg Gly
       290                 295                 300
   Ala Pro Gly Glu Arg Gly Arg Pro Gly Leu Pro Gly Ala Ala Gly Ala
   305 310 315 320
   Arg Gly Asn Asp Gly Ala Arg Gly Ser Asp Gly Gln Pro Gly Pro Pro
   325 330 335
   Gly Pro Pro Gly Thr Ala Gly Phe Pro Gly Ser Pro Gly Ala Lys Gly
   340 345 350
   Glu Val Gly Pro Ala Gly Ser Pro Gly Pro Ser Gly Ser Pro Gly Gln
   355 360 365
   Arg Gly Glu Pro Gly Pro Gln Gly His Ala Gly Ala Ala Gly Pro Pro
   370 375 380
   Gly Pro Pro Gly Ser Asn Gly Ser Pro Gly Gly Lys Gly Glu Met Gly
   385 390 395 400
   Pro Ala Gly Ile Pro Gly Ala Pro Gly Leu Met Gly Ala Arg Gly Pro
   405 410 415
   Pro Gly Pro Pro Gly Thr Asn Gly Ala Pro Gly Gln Arg Gly Ala Ala
   420 425 430
   Gly Glu Pro Gly Lys Asn Gly Ala Lys Gly Glu Pro Gly Pro Arg Gly
   435 440 445
   Glu Arg Gly Glu Ala Gly Ser Pro Gly Ile Pro Gly Pro Lys Gly Glu
   450 455 460
   Asp Gly Lys Asp Gly Ser Pro Gly Glu Pro Gly Ala Asn Gly Leu Pro
   465 470 475 480
   Gly Ala Ala Gly Glu Arg Gly Met Pro Gly Phe Arg Gly Ala Pro Gly
   485 490 495
   Ala Asn Gly Leu Pro Gly Glu Lys Gly Pro Ala Gly Glu Arg Gly Gly
   500 505 510
   Pro Gly Pro Ala Gly Pro Arg Gly Val Ala Gly Glu Pro Gly Arg Asp
   515 520 525
   Gly Val Pro Gly Gly Pro Gly Leu Arg Gly Met Pro Gly Ser Pro Gly
   530 535 540
   Gly Pro Gly Ser Asp Gly Lys Pro Gly Pro Pro Gly Ser Gln Gly Glu
   545 550 555 560
   Ser Gly Arg Pro Gly Pro Pro Gly Ser Pro Gly Pro Arg Gly Gln Pro
   565 570 575
   Gly Val Met Gly Phe Pro Gly Pro Lys Gly Asn Asp Gly Ala Pro Gly
   580 585 590
   Lys Asn Gly Glu Arg Gly Gly Pro Gly Gly Pro Gly Leu Pro Gly Pro
   595 600 605
   Pro Gly Lys Asn Gly Glu Thr Gly Pro Gln Gly Pro Pro Gly Pro Thr
   610 615 620
   Gly Pro Gly Gly Asp Lys Gly Asp Thr Gly Pro Pro Gly Gln Gln Gly
   625 630 635 640
   Leu Gln Gly Leu Pro Gly Thr Ser Gly Pro Pro Gly Glu Asn Gly Lys
   645 650 655
   Pro Gly Glu Pro Gly Pro Lys Gly Glu Ala Gly Ala Pro Gly Ile Pro
   660 665 670
   Gly Gly Lys Gly Asp Ser Gly Ala Pro Gly Glu Arg Gly Pro Pro Gly
   675 680 685
   Ala Val Gly Pro Ser Gly Pro Arg Gly Gly Ala Gly Pro Pro Gly Pro
   690 695 700
   Glu Gly Gly Lys Gly Pro Ala Gly Pro Pro Gly Pro Pro Gly Ala Ala
   705 710 715 720
   Gly Thr Pro Gly Leu Gln Gly Met Pro Gly Glu Arg Gly Gly Ser Gly
   725 730 735
   Gly Pro Gly Pro Lys Gly Asp Lys Gly Asp Pro Gly Gly Ser Gly Ala
   740 745 750
   Asp Gly Ala Pro Gly Lys Asp Gly Pro Arg Gly Pro Thr Gly Pro Ile
   755 760 765
   Gly Pro Pro Gly Pro Ala Gly Gln Pro Gly Asp Lys Gly Glu Ser Gly
   770 775 780
   Ala Pro Gly Leu Pro Gly Ile Ala Gly Pro Arg Gly Gly Pro Gly Glu
   785 790 795 800
   Arg Gly Glu His Gly Pro Pro Gly Pro Ala Gly Phe Pro Gly Ala Pro
   805 810 815
   Gly Gln Asn Gly Glu Pro Gly Ala Lys Gly Glu Arg Gly Ala Pro Gly
   820 825 830
   Glu Lys Gly Glu Gly Gly Pro Pro Gly Ile Ala Gly Gln Pro Gly Gly
   835 840 845
   Thr Gly Pro Pro Gly Pro Pro Gly Pro Gln Gly Val Lys Gly Glu Arg
   850 855 860
   Gly Ser Pro Gly Gly Pro Gly Ala Ala Gly Phe Pro Gly Gly Arg Gly
   865 870 875 880
   Leu Pro Gly Pro Pro Gly Ser Asn Gly Asn Pro Gly Pro Pro Gly Ser
   885 890 895
   Ser Gly Pro Pro Gly Lys Asp Gly Pro Pro Gly Pro Pro Gly Ser Ser
   900 905 910
   Gly Ala Pro Gly Ser Pro Gly Val Ser Gly Pro Lys Gly Asp Ala Gly
   915 920 925
   Gln Pro Gly Glu Lys Gly Ser Pro Gly Pro Gln Gly Pro Pro Gly Ala
   930 935 940
   Pro Gly Pro Gly Gly Ile Ser Gly Ile Thr Gly Ala Arg Gly Leu Ala
   945 950 955 960
   Gly Pro Pro Gly Met Pro Gly Ala Arg Gly Ser Pro Gly Pro Gln Gly
   965 970 975
   Val Lys Gly Glu Asn Gly Lys Pro Gly Pro Ser Gly Leu Asn Gly Glu
   980 985 990
   Arg Gly Pro Pro Gly Pro Gln Gly Leu Pro Gly Leu Ala Gly Ala Ala
   995 1000 1005
   Gly Glu Pro Gly Arg Asp Gly Asn Pro Gly Ser Asp Gly Leu Pro Gly
   1010 1015 1020
   Arg Asp Gly Ala Pro Gly Ser Lys Gly Asp Arg Gly Glu Asn Gly Ser
   1025 1030 1035 1040
   Pro Gly Ala Pro Gly Ala Pro Gly His Pro Gly Pro Pro Gly Pro Val
   1045 1050 1055
   Gly Pro Ala Gly Lys Asn Gly Asp Arg Gly Glu Thr Gly Pro Ala Gly
   1060 1065 1070
   Pro Ala Gly Ala Pro Gly Pro Ala Gly Ser Arg Gly Ala Pro Gly Pro
   1075 1080 1085
   Gln Gly Pro Arg Gly Asp Lys Gly Glu Thr Gly Glu Arg Gly Ala Asn
   1090 1095 1100
   Gly Ile Lys Gly His Arg Gly Phe Pro Gly Asn Pro Gly Ala Pro Gly
   1105 1110 1115 1120
   Ser Pro Gly Pro Ala Gly His Gln Gly Ala Val Gly Ser Pro Gly Pro
   1125 1130 1135
   Ala Gly Pro Arg Gly Pro Val Gly Pro Ser Gly Pro Pro Gly Lys Asp
   1140 1145 1150
   Gly Ala Ser Gly His Pro Gly Pro Ile Gly Pro Pro Gly Pro Arg Gly
   1155 1160 1165
   Asn Arg Gly Glu Arg Gly Ser Glu Gly Ser Pro Gly His Pro Gly Gln
   1170 1175 1180
   Pro Gly Pro Pro Gly Pro Pro Gly Ala Pro Gly Pro Cys Cys Gly Gly
   1185 1190 1195 1200
   Gly Ala Ala Ala Ile Ala Gly Val Gly Gly Glu Lys Ala Gly Gly Phe
   1205 1210 1215
   Ala Pro Tyr Tyr Gly Asp Glu Pro Met Asp Phe Lys Ile Asn Thr Asp
   1220 1225 1230
   Glu Ile Met Thr Ser Leu Lys Ser Val Asn Gly Gln Ile Glu Ser Leu
   1235 1240 1245
   Ile Ser Pro Asp Gly Ser Arg Lys Asn Pro Ala Arg Asn Cys Arg Asp
   1250 1255 1260
   Leu Lys Phe Cys His Pro Glu Leu Lys Ser Gly Glu Tyr Trp Val Asp
   1265                1270                1275                1280
   Pro Asn Gln Gly Cys Lys Met Asp Ala Ile Lys Val Phe Cys Asn Met
                   1285                1290                1295
   Glu Thr Gly Glu Thr Cys Ile Ser Ala Ser Pro Ser Thr Val Pro Arg
               1300                1305                1310
   Lys Asn Trp Trp Thr Asp Ser Gly Ala Glu Lys Lys Tyr Val Trp Phe
           1315                1320                1325
   Gly Glu Ser Met Asn Gly Gly Phe Gln Phe Ser Tyr Gly Asn Pro Glu
       1330                1335                1340
   Leu Pro Glu Asp Val Leu Asp Val Gln Leu Ala Phe Leu Arg Leu Leu
   1345                1350                1355                1360
   Ser Ser Arg Ala Ser Gln Asn Ile Thr Tyr His Cys Lys Asn Ser Ile
                   1365                1370                1375
   Ala Tyr Met Glu His Ala Ser Gly Asn Val Lys Lys Ala Leu Arg Leu
               1380                1385                1390
   Met Gly Ser Asn Glu Gly Glu Phe Lys Ala Glu Gly Asn Ser Lys Phe
           1395                1400                1405
   Thr Tyr Thr Val Leu Glu Asp Gly Cys Thr Lys His Thr Gly Glu Trp
       1410                1415                1420
   Gly Lys Thr Val Phe Glu Tyr Arg Thr Arg Lys Ala Val Arg Leu Pro
   1425                1430                1435                1440
   Ile Val Asp Ile Ala Pro Tyr Asp Ile Gly Gly Pro Asp Gln Glu Phe
                   1445                1450                1455
   Gly Ala Asp Ile Gly Pro Val Cys Phe Leu
               1460                1465
<210> 13
   <211> 20
   <212> DNA
   <213> human
<400> 13
   ccggctcctg ctcctcttag 20
<210> 14
   <211> 20
   <212> DNA
   <213> human
<400> 14
   gccaggagca ccagcaatac 20
<210> 15
   <211> 20
   <212> DNA
   <213> human
<400> 15
   gctgatggac agcctggtgc 20
<210> 16
   <211> 20
   <212> DNA
   <213> human
<400> 16
   gccctggaag accagctgca 20
<210> 17
   <211> 20
   <212> DNA
   <213> human
<400> 17
   cctggcctta agggaatgcc 20
<210> 18
   <211> 20
   <212> DNA
   <213> human
<400> 18
   gcgccaggag aaccgtctcg 20
<210> 19
   <211> 20
   <212> DNA
   <213> human
<400> 19
   ccgaaggttc ccctggacga 20
<210> 20
   <211> 20
   <212> DNA
   <213> human
<400> 20
   cggtcatgct ctcgccgaac 20
<210> 21
   <211> 22
   <212> DNA
   <213> bovine
<400> 21
   ccccagttgt cttacggcta tg 22
<210> 22
   <211> 22
   <212> DNA
   <213> bovine
<400> 22
   catagccgta agacaactgg gg 22
<210> 23
   <211> 19
   <212> DNA
   <213> bovine
<400> 23
   ggtagccccg gtgaaaatg 19
<210> 24
   <211> 19
   <212> DNA
   <213> bovine
<400> 24
   cattttcacc ggggctacc 19
<210> 25
   <211> 20
   <212> DNA
   <213> bovine
<400> 25
   gccccaaggg taacagcggt 20
<210> 26
   <211> 20
   <212> DNA
   <213> bovine
<400> 26
   accgctgtta cccttggggc 20
<210> 27
   <211> 22
   <212> DNA
   <213> bovine
<400> 27
   tcctggccct gctggcccca aa 22
<210> 28
   <211> 22
   <212> DNA
   <213> bovine
<400> 28
   tttggggcca gcagggccag ga 22
<210> 29
   <211> 22
   <212> DNA
   <213> bovine
<400> 29
   tggacctaaa ggtgctgctg ga 22
<210> 30
   <211> 22
   <212> DNA
   <213> bovine
<400> 30 22
   tccagcagca cctttaggtc ca 22
<210> 31
   <211> 20
   <212> DNA
   <213> bovine
<400> 31 20
   gaacagggtg ttcctggaga 20
<210> 32
   <211> 20
   <212> DNA
   <213> bovine
<400> 32 20
   tctccaggaa caccctgttc 20
<210> 33
   <211> 18
   <212> DNA
   <213> bovine
<400> 33 18
   ggcaaagatg gcgtccgt 18
<210> 34
   <211> 18
   <212> DNA
   <213> bovine
<400> 34 18
   acggacgcca tctttgcc 18
<210> 35
   <211> 20
   <212> DNA
   <213> bovine
<400> 35 20
   gctaaaggcg aacctggcga 20
<210> 36
   <211> 20
   <212> DNA
   <213> bovine
<400> 36 20
   tcgccaggtt cgcctttagc 20
<210> 37
   <211> 21
   <212> DNA
   <213> bovine
<400> 37
   gccggcaaga gcggtgatcg t 21
<210> 38
   <211> 21
   <212> DNA
   <213> bovine
<400> 38
   acgatcaccg ctcttgccgg c 21
<210> 39
   <211> 19
   <212> DNA
   <213> bovine
<400> 39
   cgatggtggc cgctactac 19
<210> 40
   <211> 19
   <212> DNA
   <213> bovine
<400> 40
   gtagtagcgg ccaccatcg 19
<210> 41
   <211> 23
   <212> DNA
   <213> bovine
<400> 41
   agagcatgac cgaagggcga att 23
<210> 42
   <211> 23
   <212> DNA
   <213> bovine
<400> 42
   aattcgccct tcggtcatgc tct 23
<210> 43
   <211> 39
   <212> DNA
   <213> human
<400> 43
   ttaattccta ggatgttcag ctttgtggac ctccggctc 39
<210> 44
   <211> 32
   <212> DNA
   <213> human
<400> 44
   tgccactctg actggaagag tggagagtac tg 32
<210> 45
   <211> 45
   <212> DNA
   <213> human
<400> 45
   ttttcctttt gcggccgctt acaggaagca gacagggcca acgtc 45
<210> 46
   <211> 30
   <212> DNA
   <213> bovine
<400> 46
   gtcatggtac ctgaggccgt tctgtacgca 30
<210> 47
   <211> 29
   <212> DNA
   <213> bovine
<400> 47
   acgtcatcgc acagcacgtt gccgttgtc 29
<210> 48
   <211> 34
   <212> DNA
   <213> bovine
<400> 48
   aggacagtcc ttaagttcgt cgcagatcac gtca 34
<210> 49
   <211> 26
   <212> DNA
   <213> bovine
<400> 49
   agggaggcca gctgttccag gcaatc 26
<210> 50
   <211> 27
   <212> DNA
   <213> bovine
<400> 50
   ccgaaggttc ccctggacga gatggtt 27
<210> 51
   <211> 29
   <212> DNA
   <213> bovine
<400> 51
   cgtggtgaca agggtgagac aggcgaaca 29
<210> 52
   <211> 27
   <212> DNA
   <213> bovine
<400> 52
   cgggctgatg atgccaatgt ggtccgt 27
<210> 53
   <211> 32
   <212> DNA
   <213> bovine
<400> 53
   aacatggaaa ccggtgagac ctgtgtatac cc 32
<210> 54
   <211> 25
   <212> DNA
   <213> human
<400> 54
   gacatgatga gctttgtgca aaagg 25
<210> 55
   <211> 27
   <212> DNA
   <213> bovine
<400> 55
   tttggtttat aaaaagcaaa cagggcc 27
<210> 56
   <211> 24
   <212> DNA
   <213> human
<400> 56
   tctcatgtct gatatttaga catg 24
<210> 57
   <211> 26
   <212> DNA
   <213> bovine
<400> 57
   ggactaatga ggctttctat ttgtcc 26
<210> 58
   <211> 24
   <212> DNA
   <213> bovine
<400> 58
   ggcaccattc ttaccaggct cacc 24
<210> 59
   <211> 22
   <212> DNA
   <213> bovine
<400> 59
   tgggtcccgc tggcattcct gg 22
<210> 60
   <211> 23
   <212> DNA
   <213> bovine
<400> 60
   ccaggacaac caggccctcc tgg 23
<210> 61
   <211> 24
   <212> DNA
   <213> human
<400> 61
   gacatgttca gctttgtgga cctc 24
<210> 62
   <211> 20
   <212> DNA
   <213> porcine
<400> 62
   agtttacagg aagcagacag 20
<210> 63
   <211> 24
   <212> DNA
   <213> porcine
<400> 63
   ctacatgtct agggtctaga catg 24
<210> 64
   <211> 24
   <212> DNA
   <213> porcine
<400> 64
   aggcgccagg ctcgccaggc tcac 24
<210> 65
   <211> 23
   <212> DNA
   <213> porcine
<400> 65
   agttgtctta tggctatgat gag 23
<210> 66
   <211> 24
   <212> DNA
   <213> human
<400> 66
   gacatgctca gctttgtgga tacg 24
<210> 67
   <211> 23
   <212> DNA
   <213> porcine
<400> 67
   agctggacca ggctcaccaa caa 23
<210> 68
   <211> 24
   <212> DNA
   <213> porcine
<400> 68
   tggtgctaag ggtgctgctg gcct 24
<210> 69
   <211> 25
   <212> DNA
   <213> porcine
<400> 69
   aggttcaccc actgatccag caaca 25
<210> 70
   <211> 25
   <212> DNA
   <213> porcine
<400> 70
   tccctctgga gagcctggta ctgct 25
<210> 71
   <211> 25
   <212> DNA
   <213> porcine
<400> 71
   tggaagtttg ggttttaaac ttccc 25
<210> 72
   <211> 21
   <212> DNA
   <213> porcine
<400> 72
   acacaaggag tctgcatgtc t 21

## Description

### FIELD OF THE INVENTION

The present invention relates to the recombinant synthesis of collagens and gelatins derived from animal sequences. The present invention in particular relates to novel polynucleotide sequences encoding bovine collagens, and to the encoded polypeptide sequences, and to the use of such sequences in the recombinant production of animal collagens and gelatins.

### BACKGROUND OF THE INVENTION

The most abundant component of the extracellular matrix is collagen. Collagens are a a large family of fibrous proteins, characterized by the presence of triple-stranded helical domains. Collagen molecules are generally the result of the trimeric assembly of polypeptide chains containing (-Gly-X-Y-)ₙ repeats which allow for the formation of triple helical domains (van der Rest et al. (1991) FASEB J. 5:2814-2823).

### Collagen

Presently, about twenty distinct collagen types have been identified in vertebrates, including bovine, ovine, porcine, chicken, and human collagens. Generally, the collagen types are numbered by Roman numerals, and the chains found in each collagen type are identified by Arabic numerals. Detailed descriptions of structure and biological functions of the various different types of naturally occurring collagens are generally available in the art. (See, e.g., Ayad et al. (1998) The Extracellular Matrix Facts Book, Academic Press, San Diego, CA; Burgeson, R E., and Nimmi (1992) "Collagen types: Molecular Structure and Tissue Distribution" in Clin. Orthop. 282:250-272; Kielty, C. M. et al. (1993) "The Collagen Family: Structure, Assembly And Organization In The Extracellular Matrix," Connective Tissue And Its Heritable Disorders, Molecular Genetics, And Medical Aspects, Royce, P. M. and B. Steinmann eds., Wiley-Liss, NY, pp. 103-147; and Prockop, D.J- and K.I. Kivirikko (1995) "Collagens: Molecular Biology, Diseases, and Potentials for Therapy," Annu. Rev. Biochem., 64:403-434.)

Type I collagen is the major fibrillar collagen of bone and skin, comprising approximately 80-90% of an organism's total collagen. Type I collagen is the major structural macromolecule present in the extracellular matrix of multicellular organisms and comprises approximately 20% of total protein mass. Type I collagen is a heterotrimeric molecule comprising two α1(I) chains and one α2(I) chain, encoded by the COL1A1 and COL1A2 genes, respectively. Other collagen types are less abundant than type I collagen, and exhibit different distribution patterns. For example, type II collagen is the predominant collagen in cartilage and vitreous humor, while type III collagen is found at high levels in blood vessels and to a lesser extent in skin.

Type II collagen is a homotrimeric collagen comprising three identical al(II) chains encoded by the COL2A1 gene. Purified type II collagen may be prepared from tissues by, methods known in the art, for example, by procedures described in Miller and Rhodes (1982) Methods In Enzymology 82:33-64.

Type III collagen is a major fibrillar collagen found in skin and vascular tissues. Type III collagen is a homotrimeric collagen comprising three identical α1(III) chains encoded by the COL3A1 gene. Methods for purifying type III collagen from tissues can be found in, for example, Byers et al. (1974) Biochemistry 13:5243-5248; and Miller and Rhodes, *supra*.

Type IV collagen is found in basement membranes in the form of sheets rather than fibrils. Most commonly, type IV collagen contains two α1(IV) chains and one α2(IV) chain. The particular chains comprising type IV collagen are tissue-specific. Type IV collagen may be purified using, for example, the procedures described in Furuto and Miller (1987) Methods in Enzymology, 144:41-61, Academic Press.

Type V collagen is a fibrillar collagen found in, primarily, bones, tendon, cornea, skin, and blood vessels. Type V collagen exists in both homotrimeric and heterotrimeric forms. One form of type V collagen is a heterotrimer of two α1(V) chains and one α2(V) chain. Another form of type V collagen is a heterotrimer of α1(V), α2(V), and α3(V) chains. A further form of type V collagen is a homotrimer of α1(V). Methods for isolating type V collagen from natural sources can be found, for example, in Elstow and Weiss (1983) Collagen Rel. Res. 3:181-193, and Abedin et al. (1982) Biosci. Rep. 2:493-502.

Type VI collagen has a small triple helical region and two large non-collagenous remainder portions. Type VI collagen is a heterotrimer comprising α1(VI), α2(VI), and α3(VI) chains. Type VI collagen is found in many connective tissues. Descriptions of how to purify type VI collagen from natural sources can be found, for example, in Wu et al. (1987) Biochem. J. 248:373-381, and Kielty et al. (1991) J. Cell Sci. 99:797-807.

Type VII collagen is a fibrillar collagen found in particular epithelial tissues. Type VII collagen is a homotrimeric molecule of three α1(VII) chains. Descriptions of how to purify type VII collagen from tissue can be found in, for example, Lunstrum et al. (1986) J. Biol. Chem. 261:9042-9048, and Bentz et al. (1983) Proc. Natl. Acad. Sci. USA 80:3168-3172.

Type VIII collagen can be found in Descemet's membrane in the cornea. Type VIII collagen is a heterotrimer comprising two α1(VIII) chains and one α2(VIII) chain, although other chain compositions have been reported. Methods for the purification of type VIII collagen from nature can be found, for example, in Benya and Padilla (1986) J. Biol. Chem. 261:4160-4169, and Kapoor et al. (1986) Biochemistry 25:3930-3937.

Type IX collagen is a fibril-associated collagen found in cartilage and vitreous humor. Type IX collagen is a heterotrimeric molecule comprising α1(IX), α2(IX), and α3 (IX) chains. Type IX collagen has been classified as a FACIT (Fibril Associated Collagens with Interrupted Triple Helices) collagen, possessing several triple helical domains separated by non-triple helical domains. Procedures for purifying type IX collagen can be found, for example, in Duance, et al. (1984) Biochem. J. 221:885-889; Ayad et al. (1989) Biochem. J. 262:753-761; and Grant et al. (1988) The Control of Tissue Damage, Glauert, A. M., ed., Elsevier Science Publishers, Amsterdam, pp. 3-28.

Type X collagen is a homotrimeric compound of α1(X) chains. Type X collagen has been isolated from, for example, hypertrophic cartilage found in growth plates. (See, e.g., Apte et al. (1992) Eur J Biochem 206 (1):217-24.)

Type XI collagen can be found in cartilaginous tissues associated with type II and type IX collagens, and in other locations in the body. Type XI collagen is a heterotrimeric molecule comprising α1(XI), α2(XI), and α3(XI) chains. Methods for purifying type XI collagen can be found, for example, in Grant et al., *supra*.

Type XII collagen is a FACIT collagen found primarily in association with type I collagen. Type XII collagen is a homotrimeric molecule comprising three α1(XII) chains. Methods for purifying type XII collagen and variants thereof can be found, for example, in Dublet et al. (1989) J. Biol. Chem. 264:13150-13156; Lunstrum et al. (1992) J. Biol. Chem. 267:20087-20092; and Watt et al. (1992) J. Biol. Chem. 267:20093-20099.

Type XIII is a non-fibrillar collagen found, for example, in skin, intestine, bone, cartilage, and striated muscle. A detailed description of type XIII collagen may be found, for example, in Juvonen et al. (1992) J. Biol. Chem. 267:24700-24707.

Type XIV is a FACIT collagen characterized as a homotrimeric molecule comprising α1(XIV) chains. Methods for isolating type XIV collagen can be found, for example, in Aubert-Foucher et al. (1992) J. Biol. Chem. 267:15759-15764, and Watt et al., *supra*.

Type XV collagen is homologous in structure to type XVIII collagen. Information about the structure and isolation of natural type XV collagen can be found, for example, in Myers et al. (1992) Proc. Natl. Acad. Sci. USA 89:10144-10148; Huebner et al. (1992) Genomics 14:220-224; Kivirikko et al. (1994) J. Biol. Chem. 269:4773-4779; and Muragaki, J. (1994) Biol. Chem. 264:4042-4046.

Type XVI collagen is a fibril-associated collagen, found, for example, in skin, lung fibroblast, and keratinocytes. Information on the structure of type XVI collagen and the gene encoding type XVI collagen can be found, for example, in Pan et al. (1992) Proc. Natl. Acad. Sci. USA 89:6565-6569; and Yamaguchi et al. (1992) J. Biochem. 112:856-863.

Type XVII collagen is a hemidesmosal transmembrane collagen, also known at the bullous pemphigoid antigen. Information on the structure of type XVII collagen and the gene encoding type XVII collagen can be found, for example, in Li et al. (1993) J. Biol. Chem. 268(12):8825-8834; and McGrath et al. (1995) Nat. Genet. 11(1):83-86.

Type XVIII collagen is similar in structure to type XV collagen and can be isolated from the liver. Descriptions of the structures and isolation of type XVIII collagen from natural sources can be found, for example, in Rehn and Pihlajaniemi (1994) Proc. Natl. Acad. Sci USA 91:4234-4238; Oh et al. (1994) Proc. Natl. Acad. Sci USA 91:4229-4233; Rehn et al. (1994) J. Biol. Chem. 269:13924-13935; and Oh et al. (1994) Genomics 19:494-499.

Type XIX collagen is believed to be another member of the FACIT collagen family, and has been found in mRNA isolated from rhabdomyosarcoma cells. Descriptions of the structures and isolation of type XIX collagen can be found, for example, in Inoguchi et al. (1995) J. Biochem. 117:137-146; Yoshioka et al. (1992) Genomics 13:884-886; and Myers et al., J. Biol. Chem. 289:18549-18557 (1994).

Type XX collagen is a newly found member of the FACIT collagenous family, and has been identified in chick cornea. (See, e.g., Gordon et al. (1999) FASEB Journal 13:A1119; and Gordon et al. (1998), IOVS 39:S1128.)

### Gelatin

Gelatin is a derivative of collagen, a principal structural and connective protein in animals. Gelatin is derived from denaturation of collagen and contains polypeptide sequences having Gly-X-Y repeats, where X and Y are most often proline and hydroxyproline residues. These sequences contribute to triple helical structure and affect the gelling ability of gelatin polypeptides. Currently available gelatin is extracted through processing of animal hides and bones, typically from bovine and porcine sources. The biophysical properties of gelatin make it a versatile material, widely used in a variety of applications and industries. Gelatin is used, for example, in numerous pharmaceutical and medical, photographic, industrial, cosmetic, and food and beverage products and processes of manufacture. Gelatin is thus a commercially valuable and versatile product.

Gelatin is typically manufactured from naturally occurring collagen in bovine and porcine sources, in particular, from hides and bones. In some instances, gelatin can be extracted from, for example, piscine, chicken, or equine sources. Raw materials of typical gelatin production, such as bovine hides and bones, originate from animals subject to government-certified inspection and passed as fit for human consumption. There is concern over the infectivity of this raw material, due to the presence of contaminating agents such as transmissible spongiform encephalopathies (TSEs), particularly bovine spongiform encephalopathy (BSE), and scrapie, etc. (See, e.g., Rohwer, R.G. (1996), Dev Biol Stand 88:247-256.) Such issues are especially critical to gelatin used in pharmaceutical and medical applications.

Recently, concern about the safety of these materials, a significant portion of which are derived from bovine sources, has increased, causing various gelatin-containing products to become the focus of several regulatory measures to reduce the potential risk of transmission of bovine spongiform encephalopathy (BSE), linked to new variant Creutzfeldt-Jakob disease (nvCJD), a fatal neurological disease in humans. There is concern that purification steps currently used in the process of extracting gelatin from animal tissues and bones may not be sufficient to remove the likelihood of infectivity due to contaminating SE-carrying tissue (i.e., brain tissue, etc.). U.S. and European manufacturers specify that raw material for gelatin to be included in animal or human food products or in pharmaceutical, medical, or cosmetic applications must not be obtained from a growing number of BSE countries. In addition, regulations specify that certain materials, e.g., bovine brain tissues, are not used in the production of gelatin.

Current production processes involve several purification and cleansing steps, and can require harsh and lengthy modes of extraction. The animal hides and bones are treated in a rendering process, and the extracted material is subjected to various chemical treatments, including prolonged exposure to highly acidic or alkaline solutions. Numerous purification steps can involve washing and filtration and various heat treatments. Acid demineralization and lime treatments are used to remove impurities such as non-collagenous proteins. Bones must be degreased. Additional washing and filtration steps, ion exchanges, and other chemical and sterilizing treatments are added to the process to further purify the material. Furthermore, contaminants and impurities can still remain after processing, and the resultant gelatin product must thus typically be clarified, purified, and often further concentrated before being ready for use.

Commercial gelatin is generally classified as type A or type B. These classifications reflect the pre-treatment extraction sources receive as part of the extraction process. Type A is generally derived from acid-processed materials, usually porcine hides, and type B is generally derived from alkaline- or lime-processed materials, usually bovine bones (ossein) and hides. In both type A and B extraction processes, the resultant gelatin product typically comprises a mixture of gelatin molecules, in sizes of from a few thousand up to several hundred thousand Daltons.

Fish gelatin, classified as gelling or non-gelling types, and typically processed as Type A gelatin, is also used in certain commercial applications. Gelling types are usually derived from the skins of warm water fish, while non-gelling types are typically derived from cold water fish. Fish gelatins have widely varying amino acid compositions, and differ from animal gelatins in having typically lower proportions of proline and hydroxyproline residues. In contrast to other animal gelatins, fish gelatins typically remain liquid at much lower temperatures, even at comparable average molecular weights. As with animal gelatin, fish gelatin is extracted by treatment and subsequent hydrolyzation of fish skin. Again, as with animal extraction processes, the process of extracting fish gelatin results in a product that lacks homogeneity.

Current methods of extraction thus result in a gelatin product that is a heterogeneous mixture of proteins, containing polypeptides with molecular weight distributions of varying ranges. It is sometimes necessary to blend various lots of product in order to obtain a gelatin mixture with the physical properties appropriate for use in a desired application. There is thus a need for a reliable and reproducible means of gelatin production that provides a homogenous product with controlled characteristics.

In addition, in the pharmaceutical, cosmetic, and food and beverage industries, especially, there is a need for a source of gelatin other than that obtained through extraction from animal sources, e.g., bovine, porcine bones and tissues. Further, as currently available gelatin is manufactured from animal sources such as bones and tissues, there are concerns relating to the undesirable immunogenicity and infectivity of gelatin-containing products. (See, e.g., Sakaguchi, M. et al. (1999) J. Aller. Clin. Immunol. 104:695-699; Miyazawa et al. (1999) Vaccine 17:2176-2180; Sakaguchi et al. (1999) Immunology 96:286-290; Kelso (1999) J Aller. Clin Immunol. 103:200-202; Asher (1999) Dev Biol Stand 99:41-44; and Verdrager (1999) Lancet 354:1304-1305.) In addition, the availability of a substitute material that does not undergo extraction from animal sources, e.g., tissues and bones, will address various ethical, religious, and social dictates. A recombinant material that does not require extraction from animal sources, such as tissues and bones, could be used, for example, in the manufacture of foods and other ingested products, including encapsulated medicines, that are appropriate for use by people with dietary restrictions, for example, those who follow Kosher and Halal law.

### Post-translational Enzymes

Post-translational enzymes are important to the biosynthesis of collagens and collagenous proteins. For example, prolyl 4-hydroxylase is required to hydroxylate prolyl residues in the Y-position of the repeating -Gly-X-Y- sequences to 4-hydroxyproline. (See, e.g., Prockop et al. (1984) N. Engl. J. Med. 311:376-386.) Hydroxyproline plays a critical role for stabilization of the collagen triple helix.

Vertebrate prolyl 4-hydroxylase is an α₂β₂ tetramer. (See, e.g. Berg and Prockop. (1973) J. Biol. Chem. 248:1175-1192; and Tuderman et al. (1975) Eur. J. Biochem. 52:9-16.) The α subunits (63 kDa) contain the catalytic sites involved in the hydroxylation of prolyl residues, and are insoluble in the absence of β subunits. The β subunits (55 kDa), identical to protein disulfide isomerase, catalyze thiol/disulfide interchange protein substrate, leading to the formation of a set of disulfide bonds essential to establishing a stable protein. The β subunits retain 50% of protein disulfide isomerase activity when part of the prolyl 4-hydroxylase tetramer. (See, e.g., Pihlajaniemi et al. (1987) Embo J. 6:643-649; Parkkonen et al. (1988) Biochem. J. 256:1005-1011; and Koivu et al. (1987) J. Biol. Chem. 262:6447-6449.) Active recombinant human prolyl 4-hydroxylase has been produced in insect cells by simultaneously expressing the α and β subunits. (See, e.g., Vuori et al. (1992) Proc. Natl. Acad. Sci. USA 89:7467-7470.)

In addition to prolyl 4-hydroxylase, other collagen post-translational enzymes have been identified and reported in the literature, including, for example, C-proteinase, N-proteinase, lysyl oxidase, and lysyl hydroxylase. (See, e.g., Olsen et al. (1991) Cell Biology of Extracellular Matrix, 2nd ed., Hay editor, Plenum Press, New York.)

Expression of many exogenous genes is readily obtained in a variety of recombinant host-vector systems. However, expression becomes difficult if the final formation of the protein requires extensive post-translational processing. For example, prolyl 4-hydroxylase activity is clearly an essential requirement for hydroxylation in nature of collagenous domains. Supplementation of prolyl 4-hydroxylase activity is required in expression systems deficient of prolyl 4-hydroxylase endogenous activity, in order to provide hydroxylation systems as found in nature.

Failure to obtain reliable and stable recombinant expression of genes for collagens has prevented the production of collagens and gelatins that have a number of useful applications. In addition, many types of collagen are only available in trace quantities present in tissues, and cannot be obtained in significant quantities from these sources. Furthermore, non-collagenous impurities can be left over after or introduced during the extraction and purification processes.

### Summary

In summary, although the characteristics of commercially available animal collagens and gelatins are suitable for many products, the variability in these currently available materials, and the difficulties associated with optimizing these materials for use in various applications, provide little flexibility. As a result, there is a need in the art for an efficient system that allows the starting material to be modified at the genetic and molecular levels, providing the potential for producing recombinant collagens and gelatins, specifically tailored and standardized for different applications and markets. Furthermore, existing concern over the risks of immunogenicity and infectivity associated with the use of the extracted materials currently available has established a need for a pure and safe substitute material.

WO 97/04123 discloses a method of producing a collagen compound in transgenic plants. The compound is preferably procollagen or collagen or fragments thereof of human, animal or fish origin. The compound may be recovered from the plant as collagen or gelatin.

FR-A-2757874 discloses the use of a recombinant nucleotide sequence containing a cDNA coding for one or several mammal collagen chains or derived proteins and elements enabling a plant cell to produce the collagen chain(s) or derived proteins, coded by said cDNA, particularly a transcription promoter and terminator identified by the transcription machinery of the plant cells, for transforming the plant cells so as to obtain from these cells, or plants obtained from them, the collagen chain(s) or derived proteins. The invention also concerns the resulting proteins and transformed plant material as well as their uses.

### SUMMARY OF THE INVENTION

The present invention provides animal collagens and gelatins, and methods of producing these animal collagens and gelatins. Therefore, in one aspect, the present invention provides a recombinant bovine collagen polypeptide comprising the amino acid sequence of SEQ ID NO: 2.

In certain embodiments, the polypeptide is single-chain, or homotrimeric, or heterotrimeric. In one aspect, the polypeptide consists of the amino acid sequence of SEQ ID NO:2. A composition comprising the polypeptide is also provided.

In a further embodiment, the present invention encompasses an isolated and purified polynucleotide encoding SEQ ID NO:2, and an isolated and purified polynucleotide that is complementary to the polynucleotide encoding SEQ ID NO: 2. Compositions, expression vectors, and host cells comprising the polynucleotide are also provided. In various embodiments, the host cell is a prokaryotic cell or a eukaryotic cell, specifically, an animal, yeast, plant, insect, or fungal cell. In some embodiments, the present invention provides transgenic non-human animals and transgenic plants comprising the polynucleotide. In one aspect, the present invention encompasses a method for producing a bovine α1(I) collagen, the method comprising culturing the host cell comprising the polynucleotide under conditions suitable for expression of the polypeptide of the invention and recovering the polypeptide from the host cell culture.

Methods for producing recombinant animal gelatins are also provided. In one aspect, the method comprises providing the recombinant bovine collagen polypeptides of the invention and deriving recombinant animal gelatin therefrom. In another aspect, the method comprises producing recombinant animal gelatin directly from a polynucleotide encoding SEQ ID NO:2.

### BRIEF DESCRIPTION OF THE FIGURES

Figures 1A, 1B, and 1C show a nucleic acid sequence (SEQ NO:1) encoding a bovine α1(I) collagen.
Figures 2A, 2B, 2C, and 2D show the amino acid sequence (SEQ ID NO:2) of a bovine α1(I) collagen.

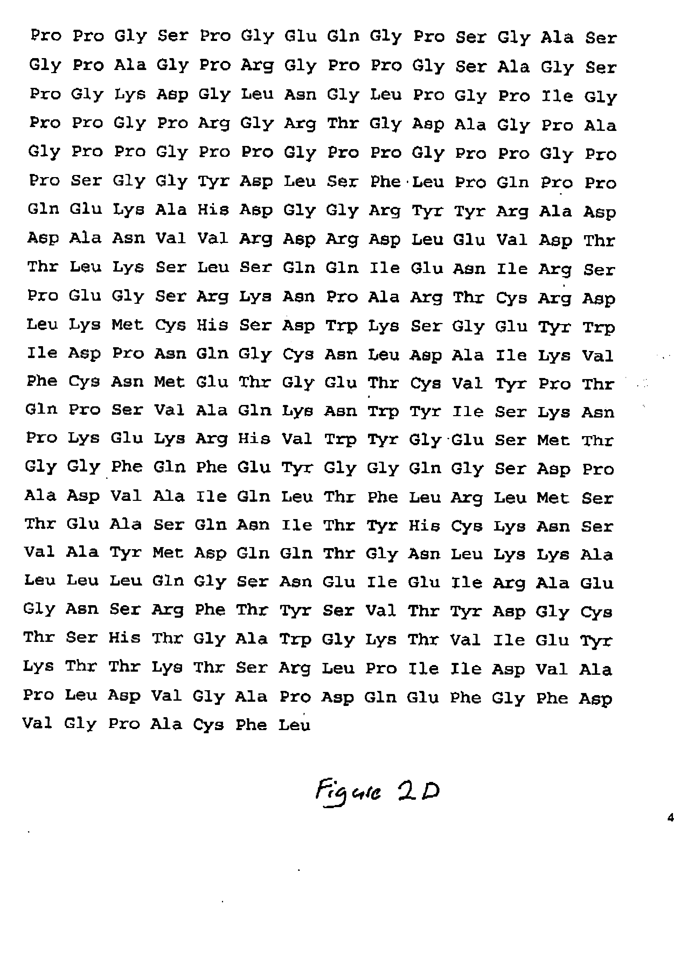

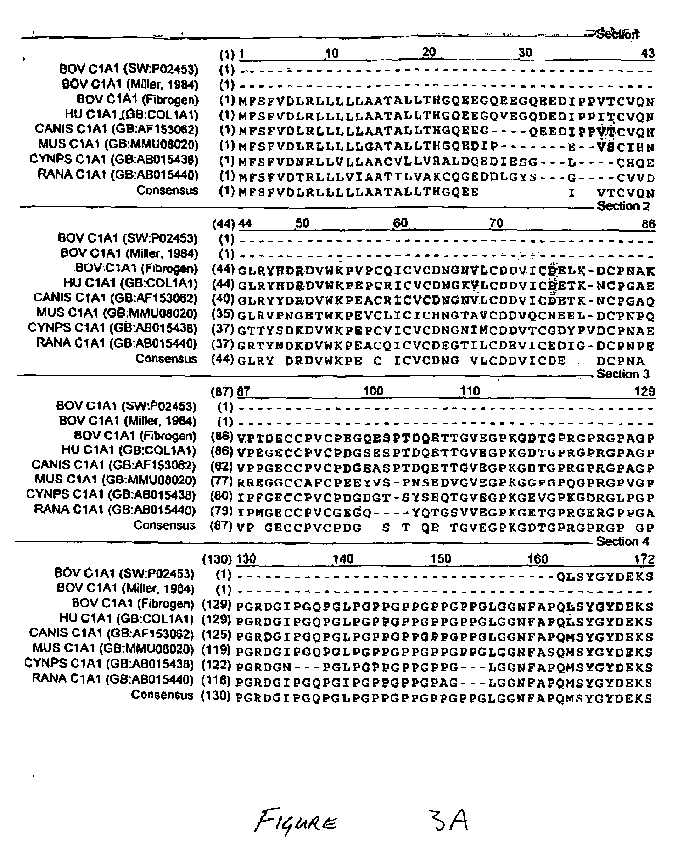

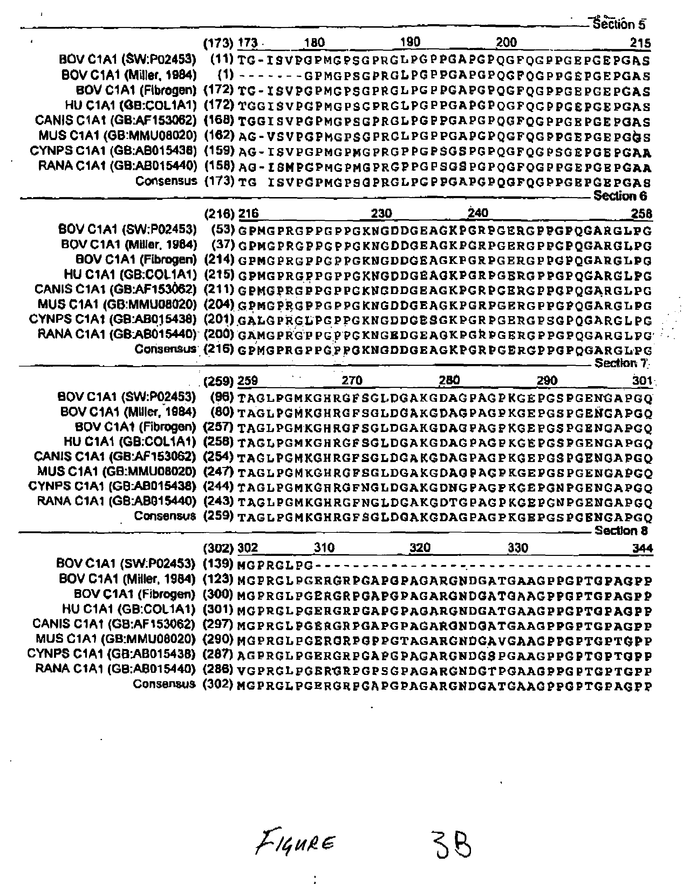

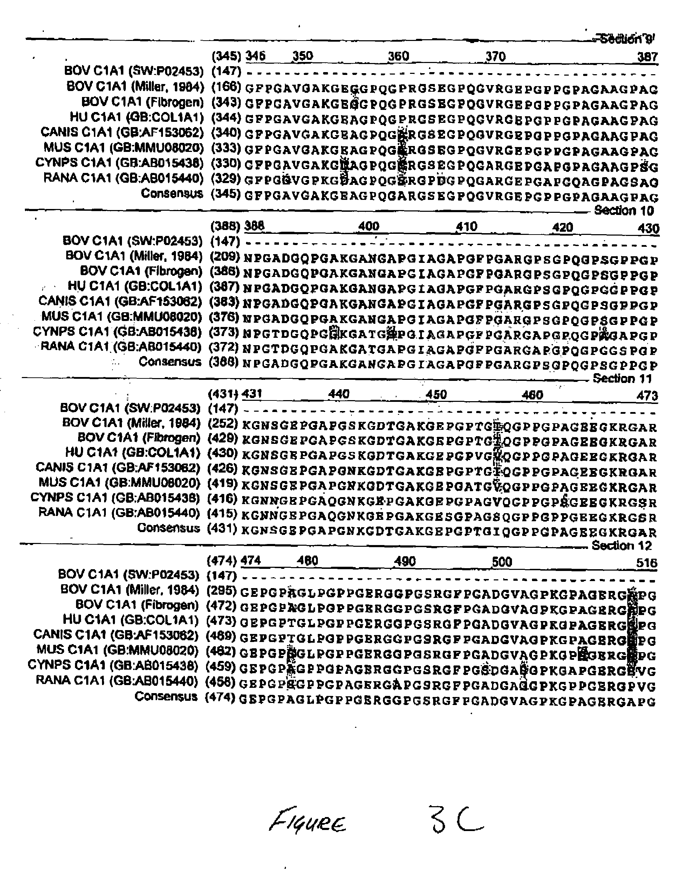

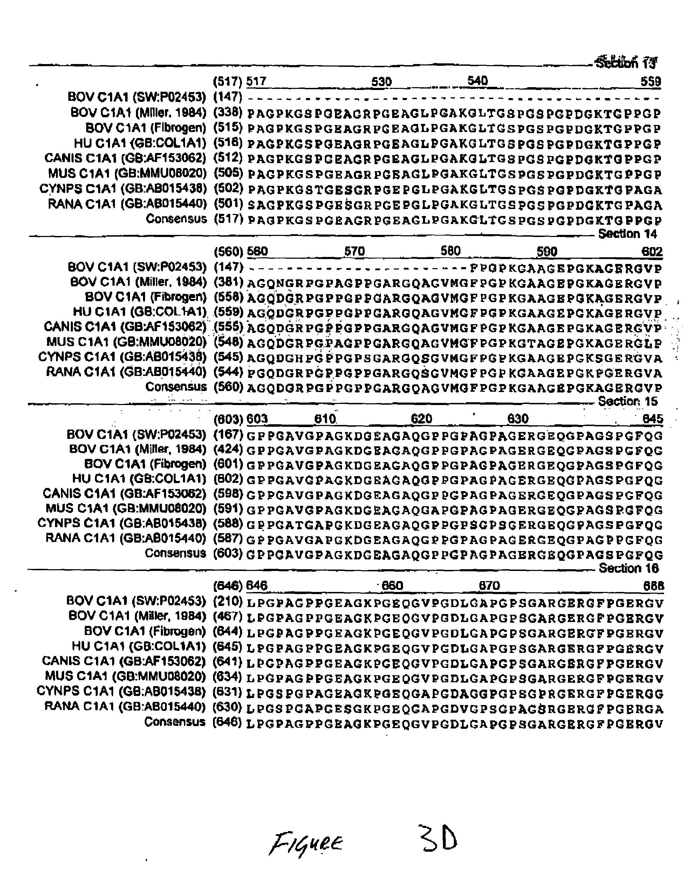

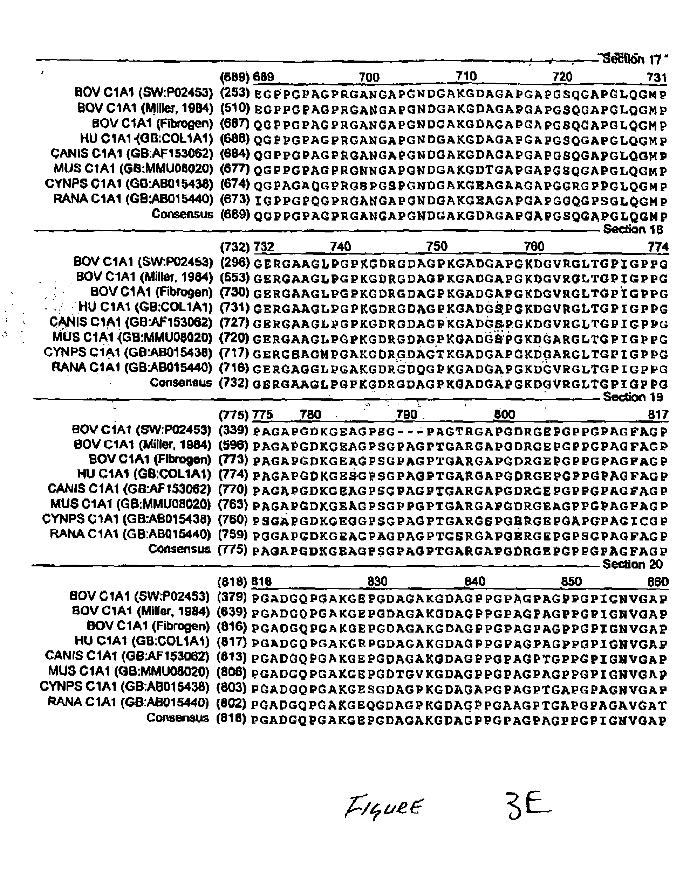

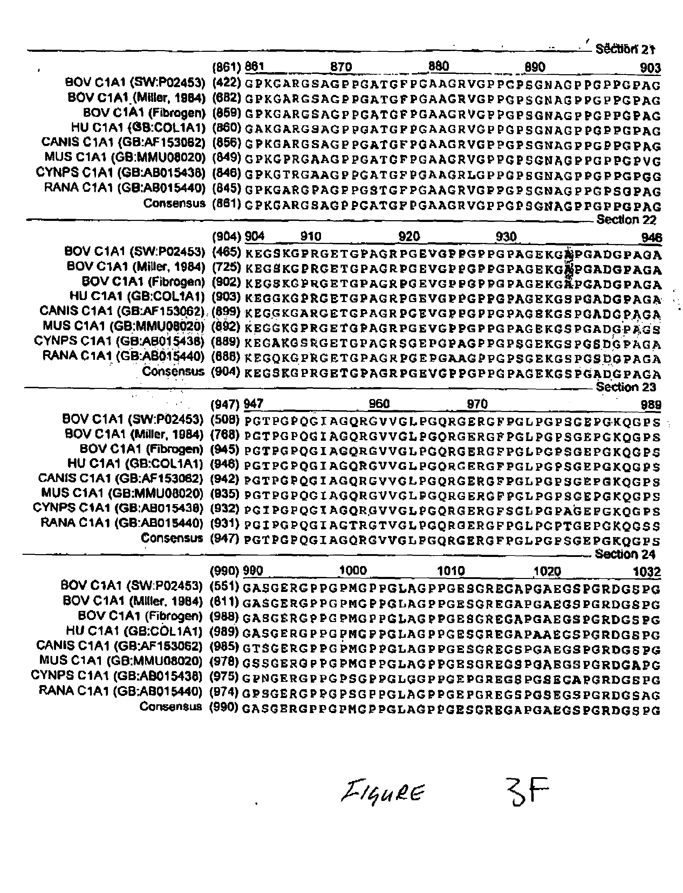

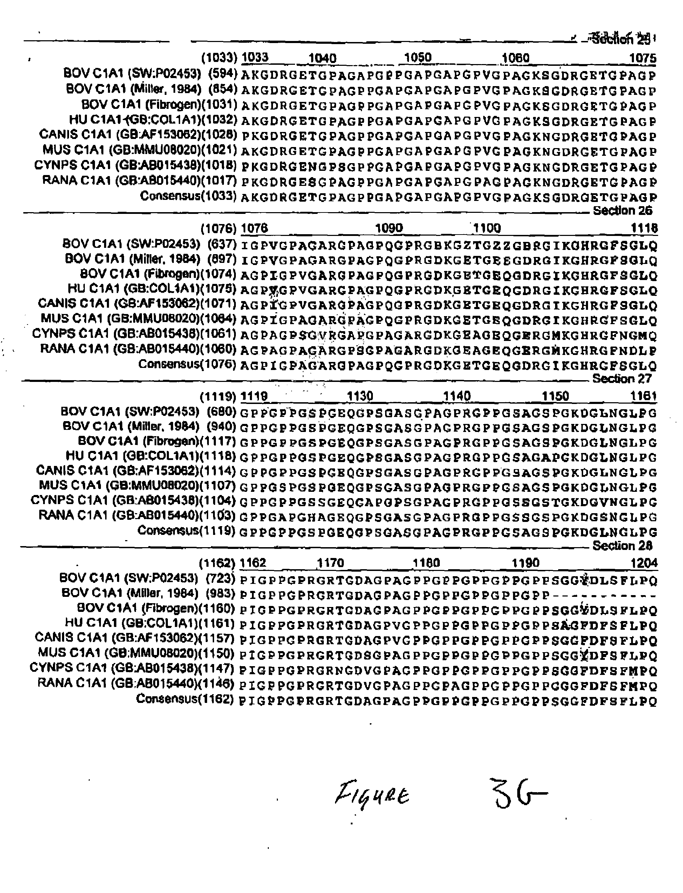

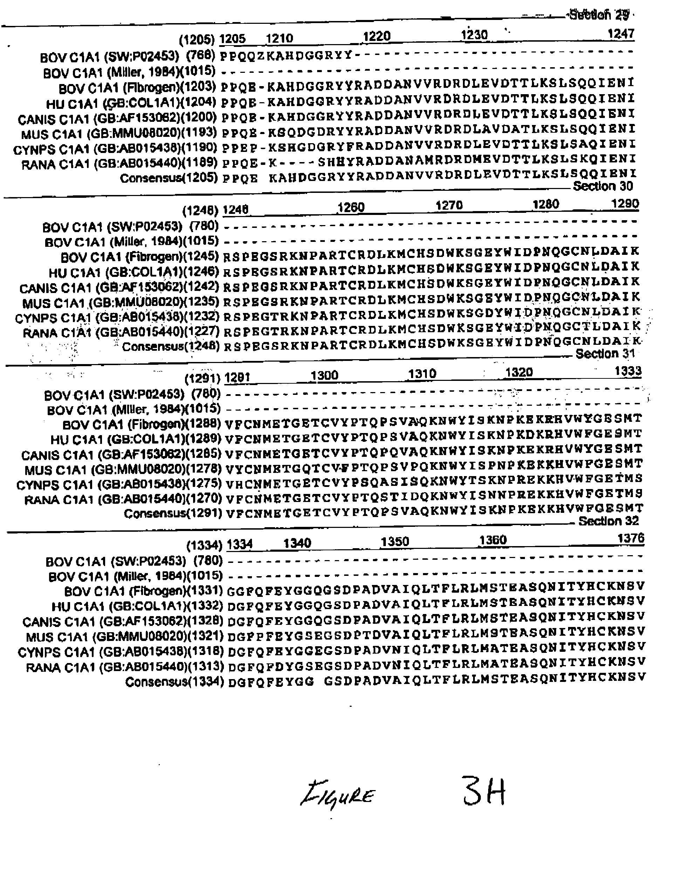

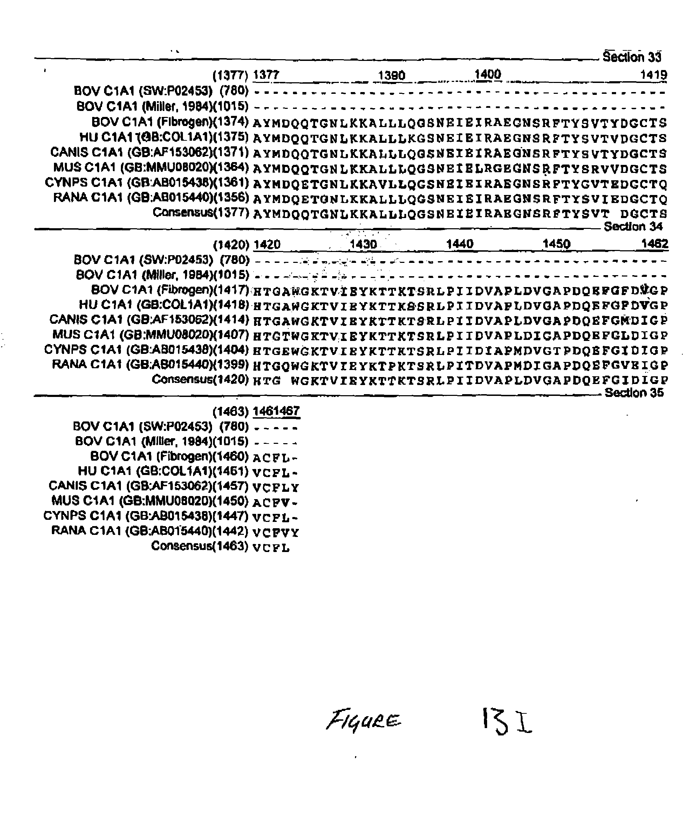

Figures 3A, 3B, 3C, 3D, 3E, 3F, 3G, 3H, and 3I depict the translated bovine α1(I) collagen open reading frame sequences aligned with known human (HU), mouse (MUS), dog (CANIS), bullfrog (RANA), and Japanese newt (CYNPS) collagen sequences.

### DETAILED DESCRIPTION OF THE INVENTION

Before the present proteins, nucleotide sequences, and methods are described, it is understood that this invention is not limited to the particular methodology, protocols, cell lines, vectors, and reagents described, as these may vary. It is also to be understood that the terminology used herein is for the purpose of describing particular embodiments only, and is not intended to limit the scope of the present invention.

It must be noted that at used herein, and in the appended claims, the singular forms "a," "an," and "the" include plural reference unless the context clearly dictate otherwise. Thus, for example, reference to "a host cell" is reference to one or more of such host cells and equivalents thereof known to those skilled in the art, and reference to "an antibody" is a reference to one or more antibodies and equivalents thereof known to those skilled in the art, and so forth.

Unless defined otherwise, all technical and scientific terms used herein have the meanings as commonly understood by one of ordinary skill in the art to which the invention belongs. Although any methods and materials similar or equivalent to those described herein can be used in the practice or testing of the present invention, the preferred methods, devices, and materials are now described. All publications mentioned herein are for the purpose of describing and disclosing the cell lines, vectors, and methodologies, etc., which are reported in the publications which might be used in connection with the invention. Nothing herein is to be construed as an admission that the invention is not entitled to antedate such disclosure by virtue of prior invention.

The practice of the present invention will employ, unless otherwise indicated, conventional methods of chemistry, biochemistry, molecular biology, immunology and pharmacology, within the skill of the art. Such techniques are explained fully in the literature. See, e.g., Gennaro, A.R., ed. (1990) Remington's Pharmaceutical Sciences, 18th ed., Mack Publishing Co.; Colowick, S. et al., eds., Methods In Enzymology, Academic Press, Inc.; Handbook of Experimental Immunology, Vols. I-IV (D.M. Weir and C.C. Blackwell, eds., 1986, Blackwell Scientific Publications); Maniatis, T. et al., eds. (1989) Molecular Cloning: A Laboratory Manual, 2nd edition, Vols. I-III, Cold Spring Harbor Laboratory Press; Ausubel, F. M. et al., eds. (1999) Short Protocols in Molecular Biology, 4th edition, John Wiley & Sons; Ream et al., eds. (1998) Molecular Biology Techniques: An Intensive Laboratory Course, Academic Press); PCR (Introduction to Biotechniques Series), 2nd ed. (Newton & Graham eds., 1997, Springer Verlag).

### DEFINITIONS

The term "collagen" refers to any one of the known collagen types, including collagen types I through XX, as well as to any other collagens, whether natural, synthetic, semi-synthetic, or recombinant. The term also encompasses procollagens. The term collagen encompasses any single-chain polypeptide encoded by a single polynucleotide, as well as homotrimeric and heterotrimeric assemblies of collagen chains. The term "collagen" specifically encompasses variants and fragments thereof, and functional equivalents and derivatives thereof, which preferably retain at least one structural or functional characteristic of collagen, for example, a (Gly-X-Y)ₙ domain.

So, for example, the term "bovine α1(I) collagen" refers to a single-chain bovine α1(I) collagen encoded by a single polynucleotide sequence, and to any corresponding procollagen, or to any fragment, variant, functional equivalent, or derivative thereof. The term "bovine type I collagen" refers to a homotrimeric or heterotrimeric collagen comprising bovine type I collagen chains, and to any corresponding procollagen, or to any fragment, variant, functional equivalent, or derivative thereof.

The term "procollagen" refers to a procollagen corresponding to any one of the collagen types I through XX, as well as to a procollagen corresponding to any other collagens, whether natural, synthetic, semi-synthetic, or recombinant, that possesses additional C-terminal and/or N-terminal propeptides or telopeptides that assist in trimer assembly, solubility, purification, or any other function, and that then are subsequently cleaved by N-proteinase, C-proteinase, or other enzymes, e.g., proteolytic enzymes, associated with collagen production. The term procollagen specifically encompasses variants and fragments thereof, and functional equivalents and derivatives thereof, which preferably retain at least one structural or functional characteristic of collagen, for example, a (Gly-X-Y)ₙ domain.

The term "bovine α 1 (I)" refers to a bovine α1(I) collagen or functional equivalent thereof, and to fragments and variants thereof, and to polynucleotides encoding such polypeptides from any source whether natural, synthetic, semi-synthetic, or recombinant.

"Gelatin" as used herein refers to any gelatin, whether extracted by traditional methods or recombinant or biosynthetic in origin, or to any molecule having at least one structural and/or functional characteristic of gelatin. Gelatin is currently obtained by extraction from collagen derived from animal (e.g., bovine, porcine rodent, chicken, equine, piscine) sources, e.g., bones and tissues. The term gelatin encompasses both the composition of more than one polypeptide included in a gelatin product, as well as an individual polypeptide contributing to the gelatin material, Thus, the term recombinant gelatin as used in reference to the present invention encompasses both a recombinant gelatin material comprising the present gelatin polypeptides, as well as an individual gelatin polypeptide of the present invention.

Polypeptides from which gelatin can be derived are polypeptides such as Collagens, procollagens, and other polypeptides having at least one structural and/or functional characteristic of collagen. Such a polypeptide could include a single collagen chain, or a collagen homotrimer or heterotrimer, or any fragments, derivatives, oligomers, polymers, or subunits thereof, containing at least one collagenous domain (a Gly-X-Y region). The term specifically contemplates engineered sequences not found in nature, such as altered collagen constructs, etc. An altered collagen constructs is a polynucleotide comprising a sequence that is altered, through deletions, additions, substitutions, or other changes, from the naturally occurring collagen gene.

An "adjuvant" is any agent added to a drug or vaccine to increase, improve, or otherwise aid its effect. An adjuvant used in a vaccine formulation might be an immunological agent that improves the immune response by producing a non-specific stimulator of the immune response. Adjuvants are often used in non-living vaccines.

The terms "allele" or "allelic sequence" refer to alternative forms of genetic sequences. Alleles may result from at least one mutation in the nucleic acid sequence and may result in altered mRNAs or polypeptides whose structure or function may or may not be altered. Any given natural or recombinant gene may have none, one, or many allelic forms. Common mutational changes which give rise to alleles are generally ascribed to natural deletions, additions, or substitutions of nucleotides. Each of these types of changes may occur alone, or in combination with the others, one or more times in a given sequence.

"Altered" polynucleotide sequences include those with deletions, insertions, or substitutions of different nucleotides resulting in a polynucleotide that encodes the same or a functionally equivalent polypeptide. Included within this definition are sequences displaying polymorphisms that may or may not be readily detectable using particular oligonucleotide probes or through deletion of improper or unexpected hybridization to alleles, with a locus other than the normal chromosomal locus for the subject polynucleotide sequence.

"Altered" polypeptides may contain deletions, insertions, or substitutions of amino acid residues which produce a silent change and result in a functionally equivalent polypeptide. Deliberate amino acid substitutions may be made on the basis of similarity in polarity, charge, solubility, hydrophobicity, hydrophilicity, and/or the amphipathic nature of the residues as long as the biological or immunological activity of the encoded polypeptide is retained. For example, negatively charged amino acids may include aspartic acid and glutamic acid; positively charged amino acids may include lysine and arginine; and amino acids with uncharged polar head groups having similar hydrophilicity values may include leucine, isoleucine, and valine, glycine and alanine, asparagine and glutamine, serine and threonine, and phenylalanine and tyrosine.

"Amino acid" or "polypeptide" sequences or "polypeptides," as these terms are used herein, refer to oligopeptide, peptide, polypeptide, or protein sequences, and fragments thereof, and to naturally occurring or synthetic molecules. Polypeptide or amino acid fragments are any portion of a polypeptide which retains at least one structural and/or functional characteristic of the polypeptide. In at least one embodiment, polypeptide fragments are those retaining at least one (Gly-X-Y)ₙ region.

The term "animal" as it is used in reference, for example, to "animal collagens"encompasses any collagens, whether natural, synthetic, semi-synthetic, or recombinant. Animal sources include, for example, mammalian sources, including, but not limited to, bovine, porcine, equine, rodent, and ovine sources, and other animal sources, including, but not limited to, chicken and piscine sources, and non-vertebrate sources.

"Antigenicity" relates to the ability of a substance to, when introduced into the body, stimulate the immune response and the production of an antibody. An agent displaying the property of antigenicity is referred to as being antigenic. Antigenic agents can include, but are not limited to, a variety of macromolecules such as, for example, proteins, lipoproteins, polysaccharides, nucleic acids, bacteria and bacterial components, and viruses and viral components.

The terms "complementary" or "complementarity," as used herein, refer to the natural binding of polynucleotides by base-pairing. For example, the sequence "A-G-T" binds to the complementary sequence "T-C-A." Complementarity between two single-stranded molecules may be "partial," when only some of the nucleic acids bind, or may be complete, when total complementarity exists between the single stranded molecules. The degree of complementarity between nucleic acid strands has significant effects on the efficiency and strength of hybridization between nucleic acid strands. This is of particular importance in amplification reactions, which depend upon binding between nucleic acids strands, and in the design and use, for example, of peptide nucleic acid (PNA) molecules.

A "deletion" is a change in an amino acid or nucleotide sequence that results in the absence of one or more amino acid residues or nucleotides.

The term "derivative," as applied to polynucleotides, refers to the chemical modification of a polynucleotide encoding a particular polypeptide or complementary to a polynucleotide encoding a particular polypeptide. Such modifications include, for example, replacement of hydrogen by an alkyl, acyl, or amino group. As used herein to refer to polypeptides, the term "derivative" refers to a polypeptide which is modified, for example, by hydroxylation, glycosylation, pegylation, or by any similar process. The term "derivatives" encompasses those molecules containing at least one structural and/or functional characteristic of the molecule from which it is derived.

A molecule is said to be a "chemical derivative" of another molecule when it contains additional chemical moieties not normally a part of the molecule. Such moieties can improve the molecule's solubility, absorption, biological half-life, and the like. The moieties can alternatively decrease the toxicity of the molecule, eliminate or attenuate any undesirable side effect of the molecule, and the like. Moieties capable of mediating such effects are generally available in the art and can be found for example, in Remington's Pharmaceutical Sciences, *supra.* Procedures for coupling such moieties to a molecule are well known in the art.

An "excipient" as the term is used herein is any inert substance used as a diluent or vehicle in the formulation of a drug, a vaccine, or other pharmaceutical composition, in order to confer a suitable consistency or form to the drug, vaccine, or pharmaceutical composition.

The term "functional equivalent" as it is used herein refers to a polypeptide or polynucleotide that possesses at least one functional and/or structural characteristic of a particular polypeptide or polynucleotide. A functional equivalent may contain modifications that enable the performance of a specific function. The term "functional equivalent" is intended to include fragments, mutants, hybrids, variants, analogs, or chemical derivatives of a molecule.

A "fusion protein" is a protein in which peptide sequences from different proteins are operably linked.

The term "hybridization" refers to the process by which a nucleic acid sequence binds to a complementary sequence through base pairing. Hybridization conditions can be defined by, for example, the concentrations of salt or formamide in the prehybridization and hybridization solutions, or by the hybridization temperature, and are well known in the art. Hybridization can occur under conditions of various stringency.

In particular, stringency can be increased by reducing the concentration of salt, increasing the concentration of formamide, or raising the hybridization temperature. For example, for purposes of the present invention, hybridization under high stringency conditions occurs in about 50% formamide at about 37°C to 42°C, and under reduced stringency conditions in about 35% to 25% formamide at about 30°C to 35°C. In particular, hybridization occurs in conditions of highest stringency at 42°C in 50% formamide, 5X SSPE, 0.3% SDS, and 200 µg/ml sheared and denatured salmon sperm DNA.

The temperature range corresponding to a particular level of stringency can be further narrowed by methods known in the art, for example, by calculating the purine to pyrimidine ratio of the nucleic acid of interest and adjusting the temperature accordingly. To remove nonspecific signals, blots can be sequentially washed, for example, at room temperature under increasingly stringent conditions of up to 0.1X SSC and 0.5% SDS. Variations on the above ranges and conditions are well known in the art.

"Immunogenicity" relates to the ability to evoke an immune response within an organism. An agent displaying the property of immunogenicity is referred to as being immunogenic. Agents can include, but are not limited to, a variety of macromolecules such as, for example, proteins, lipoproteins, polysaccharides, nucleic acids, bacteria and bacterial components, and viruses and viral components. Immunogenic agents often have a fairly high molecular weight (usually greater than 10 kDa).

"Infectivity" refers to the ability to be infective or the ability to produce infection, referring to the invasion and multiplication of microorganisms, such as bacteria or viruses within the body.

The terms "insertion" or "addition" refer to a change in a polypeptide or polynucleotide sequence resulting in the addition of one or more amino acid residues or nucleotides, respectively, as compared to the naturally occurring molecule.

The term "isolated" as used herein refers to a molecule separated not only from proteins, etc., that are present in the natural source of the protein, but also from other components in general, and preferably refers to a molecule found in the presence of, if anything, only a solvent, buffer, ion, or other component normally present in a solution of the same. As used herein, the terms "isolated" and "purified" do not encompass molecules present in their natural source.

The term "microarray" refers to any arrangement of nucleic acids, amino acids, antibodies, etc., on a substrate. The substrate can be any suitable support, e.g., beads, glass, paper, nitrocellulose, nylon, or any appropriate membrane, etc. A substrate can be any rigid or semi-rigid support including, but not limited to, membranes, filters, wafers, chips, slides, fibers, beads, including magnetic or nonmagnetic beads, gels, tubing, plates, polymers, microparticles, capillaries, etc. The substrate can provide a surface for coating and/or can have a variety of surface forms, such as wells, pins, trenches, channels, and pores, to which the nucleic acids, amino acids, etc., may be bound.

The term "microorganism" can include, but is not limited to, viruses, bacteria, Chlamydia, rickettsias, mycoplasmas, ureaplasmas, fungi, and parasites, including infectious parasites such as protozoans.

The terms "nucleic acid" or "polynucleotide" sequences or "polynucleotides" refer to oligonucleotides, nucleotides, or polynucleotides, or any fragments thereof, and to DNA or RNA of natural or synthetic origin which may be single- or double-stranded and may represent the sense or antisense strand, to peptide nucleic acid (PNA), or to any DNA-like or RNA-like material, natural or synthetic in origin. Polynucleotide fragments are any portion of a polynucleotide sequence that retains at least one structural or functional characteristic of the polynucleotide. In one embodiment polynucleotide fragments are those that encode at least one (Gly-X-Y)ₙ region. Polynucleotide fragments can be of variable length, for example, greater than 60 nucleotides in length, at least 100 nucleotides in length, at least 1000 nucleotides in length, or at least 10,000 nucleotides in length.

The phrase "percent similarity" (% similarity) refers to the percentage of sequence similarity found in a comparison of two or more polypeptide or polynucleotide sequences. Percent similarity can be determined by methods well-known in the art. For example, percent similarity between amino acid sequences can be calculated using the Clustal method. (See, e.g., Higgins, D. G. and P. M. Sharp (1988) Gene 73:237-244.) The Clustal algorithm groups sequences into clusters by examining the distances between all pairs. The clusters are aligned pairwise and then in groups. The percentage similarity between two amino acid sequences, e.g., sequence A and sequence B, is calculated by dividing the length of sequence A, minus the number of gap residues in sequence A, minus the number of gap residues in sequence B, into the sum of the residue matches between sequence A and sequence B, times one hundred. Gaps of low or of no homology between the two amino acid sequences are not included in determining percentage similarity. Percent similarity can be calculated by other methods known in the art, for example, by varying hybridization conditions, and can be calculated electronically using programs such as the MEGALIGN program (DNASTAR Inc., Madison, Wisconsin).

As used herein, the term "plant" includes reference to one or more plants, i.e., any eukaryotic autotrophic organisms, such as angiosperms and gymnosperms, monotyledons and dicotyledons, etc., including, but not limited to, soybean, cotton, alfalfa, flax, tomato, sugar, beet, sunflower, potato, tobacco, maize, wheat, rice, lettuce, banana, cassava, safflower, oilseed, rape, mustard, canola, hemp, algae, kelp, etc. The term "plant" also encompasses one or more plant cells. The term "plant cells" includes, but is not limited to, vegetative tissues and organs such as seeds, suspension cultures, embryos, meristematic regions, callus tissue, leaves, roots, shoots, gametophytes, sporophytes, pollen, tubers, corms, bulbs, flowers, fruits, cones, microspores, etc.

The term "post-translational enzyme" refers to any enzyme that catalyzes post-translational modification of, for example, any collagen or procollagen. The term encompasses, but is not limited to, for example, prolyl hydroxylase, peptidyl prolyl isomerase, collagen galactosyl hydroxylysyl glucosyl transferase, hydroxylysyl galactosyl transferase, C-proteinase, N-proteinase, lysyl hydroxylase, and lysyl oxidase.

As used herein, the term "promoter" generally refers to a regulatory region of nucleic acid sequence capable of initiating, directing, and mediating the transcription of a polynucleotide sequence. Promoters may additionally comprise recognition sequences, such as upstream or downstream promoter elements, which may influence the transcription rate.

The term "non-constitutive promoters" refers to promoters that induce transcription via a specific tissue, or may be otherwise under environmental or developmental controls, and includes repressible and inducible promoters such as tissue-preferred, tissue-specific, and cell type-specific promoters. Such promoters include, but are not limited to, the AdH1 promoter, inducible by hypoxia or cold stress, the Hsp70 promoter, inducible by heat stress, and the PPDK promoter, inducible by light.

Promoters which are "tissue-preferred" are promoters that preferentially initiate transcription in certain tissues. Promoters which are "tissue-specific" are promoters that initiate transcription only in certain tissues. "Cell type-specific" promoters are promoters which primarily drive expression in certain cell types in at least one organ, for example, vascular cells.

"Inducible" or "repressible" promoters are those under control of the environment, such that transcription is effected, for example, by an environmental condition such as anaerobic conditions, the presence of light, biotic stresses, etc., or in response to internal, chemical, or biological signals, e.g., glyceraldehyde phosphate dehydrogenase, AOX1 and AOX2 methanol-inducible promoters, or to physical damage.

As used herein, the term "constitutive promoters" refers to promoters that initiate, direct, or mediate transcription, and are active under most environmental conditions and states of development or cell differentiation. Examples of constitutive promoters, include, but are not limited to, the cauliflower mosaic virus (CaMv) 35S, the 1'- or 2'- promoter derived from T-DNA of *Agrobacteriuam tumefaciens*, the ubiquitin 1 promoter, the Smas promoter, the cinnamyl alcohol dehydrogenase promoter, glyceraldehyde dehydrogenase promoter, and the *Nos* promoter, etc.

The term "purified" as it is used herein denotes that the indicated molecule is present in the substantial absence of other biological macromolecules, e.g., polynucleotides, proteins, and the like. The term preferably contemplates that the molecule of interest is present in a solution or composition at least 80% by weight; preferably, at least 85% by weight; more preferably, at least 95% by weight; and, most preferably, at least 99.8% by weight. Water, buffers, and other small molecules, especially molecules having a molecular weight of less than about one kDa, can be present.

The term "substantially purified", as used herein, refers to nucleic or amino acid sequences that are removed from their natural environment, isolated or separated, and are at least 60% free, preferably 75% free, and most preferably 90% free from other components with which they are naturally associated.

A "substitution" is the replacement of one or more amino acids or nucleotides by different amino acids or nucleotides, respectively.

The term "transfection" as used herein refers to the process of introducing an expression vector into a cell. Various transfection techniques are known in the art, for example, microinjection, lipofection, or the use of a gene gun.

"Transformation", as defined herein, describes a process by which exogenous nucleic acid sequences, e.g., DNA, enters and changes a recipient cell. Transformation may occur under natural or artificial conditions using various methods well known in the art. Transformation may rely on any known method for the insertion of foreign nucleic acid sequences into a prokaryotic or eukaryotic host cell. The method is selected based on the type of host cell being transformed and may include, but is not limited to, viral infection, electroporation, heat shock, lipofection, and particle bombardment. Such "transformed" cells include stably transformed cells in which the inserted DNA is capable of replication either as an autonomously replicating plasmid or as part of the host chromosome, and also include cells which transiently express the inserted nucleic acid for limited periods of time.

As used herein, the term "vaccine" refers to a preparation of killed or modified microorganisms, living attenuated organisms, or living fully virulent organisms, or any other agent, including, but not limited to peptides, proteins, biological macromolecules, or nucleic acids, natural, synthetic, or semi-synthetic, administered to produce or artificially increase immunity to a particular disease, in order to prevent future infection with a similar entity. Vaccines can be live or inactivated microorganisms or agents, including viruses and bacteria, as well as subunit, synthetic, semi-synthetic, or recombinant DNA-based.

Vaccines can be monovalent (a single strain/microorganism/disease vaccine) consisting of one microorganism or agent (e.g., poliovirus vaccine) or the antigens of one microorganism or agent. Vaccines can also be multivalent, e.g., divalent, trivalent, etc. (a combined vaccine), consisting of more than one microorganism or agent (e.g., a measles-mumps-rubella (MMR) vaccine) or the antigens of more than one microorganism or agent.

Live vaccines are prepared from living microorganisms. Attenuated vaccines are live vaccines prepared from microorganisms which have undergone physical alteration (such as radiation or temperature conditioning) or serial passage in laboratory animal hosts or infected tissue/cell cultures, such treatments producing avirulent strains or strains of reduced virulence, but maintaining the capability of inducing protective immunity. Examples of live attenuated vaccines include measles, mumps, rubella, and canine distemper. Inactivated vaccines are vaccines in which the infectious microbial components have been destroyed, e.g., by chemical or physical treatment (such as formalin, beta-propiolactone, or gamma radiation), without affecting the antigenicity or immunogenicity of the viral coat or bacterial outer membrane proteins. Examples of inactivated or subunit vaccines include influenza, Hepatitis A, and poliomyelitis (IPV) vaccines.

Subunit vaccines are composed of key macromolecules from, e.g., the viral, bacterial, or other agent responsible for eliciting an immune response. These components can be obtained in a number of ways, for example, through purification from microorganisms, generation using recombinant DNA technology, etc. Subunit vaccines can contain synthetic mimics of any infective agent. Subunit vaccines can include macromolecules such as bacterial protein toxins (e.g., tetanus, diphtheria), viral proteins (e.g., from influenza virus), polysaccharides from encapsulated bacteria (e.g., from *Haemophilus influenzae* and *Streptococcus pneumonia*), and viruslike particles produced by recombinant DNA technology (e.g., hepatitis B surface antigen), etc.

Synthetic vaccines are vaccines made up of small synthetic peptides that mimic the surface antigens of pathogens and are immunogenic, or may be vaccines manufactured with the aid of recombinant DNA techniques, including whole viruses whose nucleic acids have been modified.

Semi-synthetic vaccines, or conjugate vaccines, consist of polysaccharide antigens from microorganisms attached to protein carrier molecules.

DNA vaccines contain recombinant DNA vectors encoding antigens, which, upon expression of the encoded antigen in host cells having taken up the DNA, induce humoral and cellular immune responses against the encoded antigens.

Vaccines have been developed for a variety of infectious agents. The present invention is directed to recombinant gelatins that can be used in vaccine formulations regardless of the agent involved, and are thus not limited to use in the vaccines specifically described herein by way of example. Vaccines include, but are not limited to, vaccines for vacinnia virus (small pox), polio virus (Salk and Sabin), mumps, measles, rubella, diphtheria, tetanus, Varicella-Zoster (chicken pox/shingles), pertussis (whopping cough), Bacille Calmette-Guerin (BCG, tuberculosis), haemophilus influenzae meningitis, rabies, cholera, Japanese encephalitis virus, salmonella typhi, shigella, hepatitis A, hepatitis B, adenovirus, yellow fever, foot-and-mouth disease, herpes simplex virus, respiratory syncytial virus, rotavirus, Dengue, West Nile virus, Turkey herpes virus (Marek's Disease), influenza, and anthrax. The term vaccine as used herein includes reference to vaccines to various infectious and autoimmune diseases and cancers that have been or that will be developed, for example, vaccines to various infectious and autoimmune diseases and cancers, e.g., vaccines to HIV, HCV, malaria, and vaccines to breast, lung, colon, renal, bladder, and ovarian cancers.

A polypeptide or amino acid "variant" is an amino acid sequence that is altered by one or more amino acids from a particular amino acid sequence. A polypeptide variant may have conservative changes, wherein a substituted amino acid has similar structural or chemical properties to the amino acid replaced, e.g., replacement of leucine with isoleucine. A variant may also have nonconservative changes, in which the substituted amino acid has physical properties different from those of the replaced amino acid, e.g., replacement of a glycine with a tryptophan. Analogous minor variations may also include amino acid deletions or insertions, or both. Preferably, amino acid variants retain certain structural or functional characteristics of a particular polypeptide. Guidance in determining which amino acid residues may be substituted, inserted, or deleted may be found, for example, using computer programs well known in the art, such as LASERGENE software (DNASTAR Inc., Madison, WI).

A polynucleotide variant is a variant of a particular polynucleotide sequence that preferably has at least about 80%, more preferably at least about 90%, and most preferably at least about 95% polynucleotide sequence similarity to the particular polynucleotide sequence. It will be appreciated by those skilled in the art that as a result of the degeneracy of the genetic code, a multitude of variants polynucleotide sequences encoding a particular protein, some bearing minimal homology to the polynucleotide sequences of any known and naturally occurring gene, may be produced.

### Invention

The present invention provides for the production of recombinant animal collagens and gelatins. These animal collagens and gelatins provide advantages over currently available materials in that they are produced as well-characterized and pure proteins. Methods for producing these animal collagens and gelatins are also provided. The animal collagens and gelatins are derived from bovine type I collagen. Specifically, bovine α1(I) collagens and gelatins are provided.

The present invention provides for production of relatively large amounts of single types of animal collagen, synthesized in recombinant cell culture systems that do not make any other collagen types. The present invention provides animal collagen type I that is substantially free from any other collagen type. Using methods of the present invention, purification of collagen is greatly facilitated.

The present invention is further directed to vectors and plasmids used in the methods of the invention. These vectors and/or plasmids are comprised of a polynucleotide encoding the desired collagen, necessary promoters, and other sequences necessary for the proper expression of such polypeptides. The polynucleotide encoding a collagen is preferably obtained from animal sources. Animal sources include non-human mammalian sources, such as bovine, ovine, and porcine sources. In one embodiment, the vectors and plasmids further include at least one polynucleotides encoding one or more post-translational enzymes or functional equivalents thereof. The polynucleotide encoding one or more post-translational enzymes may be derived from any of the above-mentioned species. In a preferred embodiment, the collagen-encoding polynucleotide is derived from the same species as the polynucleotide encoding the post-translational enzyme.

In a further embodiment, at least one polynucleotide encoding a post-translational enzyme, such as prolyl 4-hydroxylase, C-proteinase, N-proteinase, lysyl oxidase, or lysyl hydroxylase, is inserted into cells that do not naturally produce post-translational enzymes, such as yeast cells, or may not naturally produce sufficient amounts of post-translational enzymes, such as some mammalian and insect cells. In a preferred embodiment, the post-translational enzyme is prolyl 4-hydroxylase, wherein the polynucleotides encoding an α subunit of prolyl 4-hydroxylase and the polynucleotides encoding a β subunit of prolyl 4-hydroxylase are inserted into a cell to produce a biologically active prolyl 4-hydroxylase enzyme.

The present invention specifically contemplates the use of any compound, biological or chemical, that confers hydroxylation, e.g., proline hydroxylation and/or lysine hydroxylation, etc., as desired, to the present recombinant animal collagens and gelatins. This includes, for example, prolyl 4-hydroxylase from any species, endogenously or exogenously supplied, including various isoforms of prolyl 4-hydroxylase and any variants or fragments or subunits of prolyl 4-hydroxylase having the desired activity, whether native, synthetic, or semi-synthetic, and other hydroxylases such as prolyl 3-hydroxylase, etc. (See, e.g., U.S. Patent No. 5,928,922). In one embodiment, the prolyl hydroxylase activity is conferred by a prolyl hydroxylase derived from the same species as the polynucleotide encoding recombinant Collagen or gelatin, or encoding a polypeptides from which recombinant gelatin can be derived. In a further embodiment, the prolyl 4-hydroxylase is from an animal and the encoding polynucleotide is derived from sequence from the same animal.

The present invention provides a method for producing recombinant animal collagens and gelatins. It is to be noted that while, for clarity, the present methods of production are directed generally to the production of collagens, the production methods can be applied to the production of gelatins directly from altered collagen constructs, and the production of polypeptides from which gelatins can be derived. In one embodiment, the method comprises introducing into a host cell, under conditions suitable for expression, an expression vector encoding the animal collagen of the invention and a second expression vector encoding a post-translational enzymes, and isolating the collagen. In a preferred embodiment, the post translational enzyme is prolyl hydroxylase. (See, e.g., U.S. Patent No. 5,593,859).

In one aspect, the collagen compositions of the present invention are less than fully glycosolated or less than fully hydroxylated. For example, the collagen of the present invention may be deglycosolated, unglycosolated, partially glycosolated, and partially hydroxylated. In a further aspect of the present invention, the collagen compositions are comprised of one type of collagen, and are substantially free from any other type of collagen.

Recombinant polypeptides are disclosed, including fusion products produced from chimeric genes wherein, for example, relevant epitopes of collagen can be manufactured for therapeutic and other uses. Furthermore, any modifications made to the collagens or gelatins or compositions thereof or any degradation products thereof are disclosed. Such modifications include, for example, processing of animal collagens or collagenous proteins and gelatin.

The present invention further provides gelatin compositions. Specifically, the present invention provides gelatin compositions derived from the collagen of the invention. In another aspect of the present invention, the composition is composed of a gelatin derived from a collagen type substantially free from any other collagen type. In a further aspect of the present invention, the gelatin composition is comprised of denatured triple helices.

The present invention further provides methods of producing a gelatin by expressing the collagen of the invention and deriving gelatin therefrom. The present invention further provides for direct expression of recombinant animal gelatin from a polynucleotide encoding SEQ ID NO: 2 (See, e.g., commonly owned, co-pending application U.S. Application US 2005/224264) More specfically, the process involves inserting into a cell an expression vector comprising at least one polynucleotide encoding an animal collagen of the invention and an expression vector comprising at least one polynucleotide encoding a collagen post-translational enzyme or subunit thereof, recovering the collagen, and deriving gelatin from the collagen.

In some embodiments of the present invention, the gelatin compositions may be obtained directly from the isolated collagen or from biomass or culture media. Methods, processes, and techniques of producing gelatin compositions from collagen include denaturing the triple helical structure of the collagen utilizing detergents, heat or denaturing agents. Additionally, these methods, processes, and techniques include, but are not limited to, treatments with strong alkali or strong acids, heat extraction in aqueous solution, ion exchange chromatography, cross-flow filtration and heat drying, and other methods known in the art that may be applied to collagen to produce the gelatin compositions. The same methods, processes, and techniques may be applied to biomass or culture media to produce the gelatin compositions of the present invention.

The present invention further relates to animal collagens. The present invention provides a bovine type I collagen, specifically a bovine α1(I) collagen is provided.

The present invention also provides polynucleotides encoding the bovine α1(I) collagen of the invention. The invention further provides polynucleotides complementary to the encoding polynucleotides. The present invention also provides methods of producing the recombinant bovine type I collagens of the invention.

In another aspect of the present invention, the expression vectors comprising the polynucleotides of the present invention may be inserted into host cells to produce the bovine type I collagens or gelatins. In one method, an expression vector comprising a polynucleotide of the present invention is co-expressed in host cells with an expression vector comprising a polynucleotide encoding a polypeptide of the present invention with an expression vector comprising a polynucleotide encoding a post-translational enzyme. In one embodiment, the post-translational enzyme is prolyl 4-hydroxylase, comprising an α subunit and a β subunit.

The recombinant animal collagens and gelatins of the present invention limit human exposure to various contaminants that may be present in animal tissues currently used as raw material in the manufacture of collagens and collagen-derived materials such as gelatin. Moreover, the collagens and gelatins of the present invention are more reproducible than collagens or gelatins currently obtained from raw animal sources.

In accordance with the invention, encoding polynucleotide sequences, as well as being well-characterized proteins with predictable performance may be used to generate recombinant molecules that direct the expression of the present polypeptides in appropriate host cells.

Nucleic acid sequences encoding collagens have been generally described in the art. (See, e.g., Fuller and Boedtker (1981) Biochemistry 20:996-1006; Sandell et al. (1984) J Biol Chem. 259:7826-34; Kohno et al. (1984) J Biol Chem. 259:13668-13673; French et al. (1985) Gene 39:311-312; Metsaranta et al. (1991) J Biol Chem. 266:16862-16869; Metsaranta et al, (1991) Biochim Biophys Acta 1089:241-243; Wood et al. (1987) Gene 61:225-230; Glumoff et al. (1994) Biochim Biophys Acta 1217:41-48; Shirai et al. (1998) Matrix Biology 17:85-88; Tromp et al. (1988) Biochem J. 253:919-912; Kuivaniemi et al. (1988) Biochem J. 252:633-640; and Ala-Kokko et al. (1989) Biochem J. 260:509-516.)

In one embodiment, the polynucleotide sequence encodes the bovine α1(I) collagen amino acid sequence of SEQ ID NO:2.

Altered polynucleotide sequences which may be used include deletions, additions, or substitutions of different nucleotide residues resulting in a sequence that encodes the same or a functionally equivalent gene product. The gene product itself may contain deletions, additions, or substitutions of amino acid residues still resulting in a functionally equivalent polypeptide.

The nucleic acid sequences of the invention may be engineered in order to alter the coding sequence for a variety of ends including, but not limited to, alterations which modify processing and expression of the gene product. For examples, alternative secretory signals may be substituted for the native secretory signal and/or mutations may be introduced using techniques which are well known in the art, e.g., site-directed mutagenesis, to insert new restriction sites, to alter glycosylation patterns, phosphorylation, etc. In one embodiment, the polynucleotides of the present invention may be modified in the silent position of any triplet amino acid codon so as to better conform to the codon preference of the particular host organism.

In another embodiment a polynucleotide of the present invention may be ligated to a heterologous sequence to encode a fusion protein. For example, a fusion protein may be engineered to contain a cleavage site located between an α1(I) bovine collagen sequence of the present invention and the heterologous protein sequence, so that the α1(I) collagen may be cleaved away from the heterologous moiety.

Polynucleotide variants can also be generated according to methods well-known in the art. In one method polynucleotides are changed via site-directed mutagenesis. This method uses oligonucleotide sequences that encode the polynucleotide sequence of the desired amino acid variant, as well as a sufficient adjacent nucleotide on both sides of the changed amino acid to form a stable duplex on either side of the site of being changed. In general, the techniques of site-directed mutagenesis are well known to those of skill in the art and this technique is exemplified by publications such as, for example, Edelman et al. (1983) DNA 2:183. A versatile and efficient method for producing site-specific changes in a polynucleotide sequence is described in, e.g., by Zoller and Smith (1982) Nucleic Acids Res. 10:6487-6500.

As known in the art, nucleic acid mutations do not necessarily alter the amino acid sequence encoded by a polynucleotide sequence while providing unique restriction sites useful for manipulation of the molecule. Thus, the modified molecule can be made up of a number of discrete regions, or D-regions, flanked by unique restriction sites. These discrete regions of the molecule are herein referred to as cassettes. Molecules formed of multiple copies of a cassette are encompassed by the present disclosure. Recombinant or mutant nucleic acid molecules or cassettes, which provide desired characteristics, such as resistance to endogenous enzymes such as collagenase, are also encompassed by the present disclosure. (See, e.g., Maniatis et al., *supra*; and Ausubel et al., *supra*.)

It will be appreciated by those skilled in the art that, as a result of the degeneracy of the genetic code, a multitude of polynucleotide sequences encoding the polypeptides of the present invention, some bearing minimal homology to the nucleotide sequences of any known and naturally occurring gene, may be produced. Thus, the invention contemplates each and every possible variation of nucleotide sequence that could be made by selecting combinations based on possible codon choices. These combinations are made in accordance with the standard triplet genetic code.

The invention also encompasses production of polynucleotide sequences encoding the polypeptides of the present invention entirely by synthetic chemistry. After production, the synthetic sequence may be inserted into any of the many available expression vectors and cell systems using reagents that are well known in the art. Moreover, synthetic chemistry may be used to introduce mutations into a polynucleotide sequence encoding the collagen of the invention.

PCR may also be used to create variants of the present invention. When small amounts of template nucleic acid are used as starting material, primer(s) that differs slightly in sequence from the correspondig region in the template nucleic acid can generate the desired amino acid variant. PCR amplification results in a population of product polynucleotide fragments that differ from the polynucleotide template encoding the collagen at the position specified by the primer, The product fragments replace the corresponding region in the plasmid, creating the desired nucleic acid or amino acid variant.

Due to the inherent degeneracy of the genetic code, other polynucleotide sequences which encode substantially the same or functionally equivalent polypeptide sequences are encompassed by the present disclosure, and all degeneration variants and codon-optimized sequences are specifically contemplated. Encoding polynucleotide sequences that are natural, synthetic, semi-synthetic, or recombinant may be used in the practice of the claimed invention.

As naturally produced, collagens are structural proteins comprised of one or more collagen subunits which together form at least one triple-helical domain. A variety of enzymes are utilized in order to transform the collagen subunits into procollagen or other precursor molecules, and then into mature collagen. Such enzymes include, for example, prolyl-4-hydroxylase, C-proteinase, N-prateinase, lysyl oxidase, lysyl hydroxylase, etc.

Prolyl 4-bydroxylase is a α₂β₂ tetramer, and plays a central role in the biosynthesis of all collagens, 4-hydroxyproline residues stabilize the folding of the newly synthesized polypeptide chains into stable triple-helical molecules. (See, e.g., Prockop et al. (1995) Annu. Rev. Biochem. 64:403-434; Kivirikko et al. (1992) "Post-Translational Modifications of Proteins," pp. 1-5 1; and Kivirikko et al. (1989) FASEB J. 3:1609-1617.) Additionally, the level of expression of type In collagen was lower in the absence of recombinant prolyl 4-hydroxylase than in its presence. Human isoforms of prolyl 4-hydroxylase have been cloned and characterized. (See, e.g., Helaakoski et al. (1995) Proc. Natl. Acad. Sci. 92:4427-4431; U.S. Patent No. 5,928,922.)

Lysyl hydroxylase, an α2 homodimer, catalyzes the post-translational modification of collagen to form hydroxylysine in collagens. See generally, Kivirikko et al. (1992) Post-Translational Modifications of Proteins, Harding, J.J., and Crabbe, M.J.C., eds., CRC Press, Boca Raton, FL; and Kivirikko (1995) Principles of Medical Biology, Vol. 3 Cellular Organelles and the Extracellular Matrix, Bittar, E.E., and Bittar, N., eds., JAI Press, Greenwich, Great Britain. Isoforms of lysyl hydroxylase have been cloned and identified. (See, e.g. Passoja et al. (1998) Proc. Natl. Acad. Sci. 95(18):10482-10486; and Valtavaara et al. (1997) J. Biol. Chem. 272(11):6831-6834.)

C-proteinase processes the assembled procollagen by cleaving off the C-terminal ends of the procollagens that assist in assembly of, but are not part of, the triple helix of the collagen molecule. (See, e.g., Kadler et al. (1987) J. Biol. Chem. 262:15969-15701; and Kadler et al. (1990) Ann. NY Acad. Sci. 580:214-224.)

N-proteinase processes the assembled procollagen by cleaving off the N-terminal ends of the procollagens that assist in the assembly of, but are not part of, the collagen triple helix. (See, e.g., Hojima et al. (1994) J. Biol. Chem. 269:11381-11390.)

Lysyl oxidase is an extracellular copper enzyme that catalyzes the oxidative deamination of the α-amino group in certain lysine and hydroxylysine residues to form a reactive aldehyde. These aldehydes then undergo an aldol condensation to form aldols, which cross links collagen fibrils. Information on the DNA and protein sequence of lysyl oxidase can found, for example, in Kivirikko (1995), *supra;* Kagan (1994) Path. Res. Pract. 190: 910-919; Kenyon et al. (1993) J. Biol. Chem. 268(25):18435-18437; Wu et al. (1992) J. Biol. Chem. 267(34):24199-24206; Mariani et al. (1992) Matrix 12(3):242-248; and Hamalainen et al. (1991) Genomics 11(3):508-516.

The nucleic acid sequences encoding a number of these post-translational enzymes have been reported. (See, e.g., Vuori et al. (1992) Proc. Natl. Acad. Sci. USA 89:7467-7470; and Kessler et al. (1996) Science 271:360-362. The nucleic acid sequences encoding various post-translational enzymes may also be determined according to the methods generally described above and include use of appropriate probes and nucleic acid libraries.

The recombinant animal gelatins may be derived from the animal collagens of the invention using a variety of procedures known in the art. (See, e.g., Veis, A. (1965) International Review of Connective Tissue Research, 3:113-200.) For example, a common feature of current processes is the denaturation of the secondary structure of the collagen protein, and in the majority of instances, an alteration in either the primary or tertiary structure of the collagen. Thus, the animal collagens of the present invention can be processed using different procedures depending on the type of gelatin desired.

Recombinant animal gelatins can be derived from the recombinantly produced collagen of the invention by a variety of methods known in the art. For example, gelatin may be derived directly from cell mass or culture media by taking advantage of gelatin's solubility at elevated temperatures and its stability conditions of low or high pH, low or high salt concentration and high temperatures. Methods, processes, and techniques of producing gelatin compositions from collagen include denaturing the triple helical structure of the collagen utilizing detergents, heat, or various denaturing agents well known in the art. In addition, various steps involved in the extraction of gelatin from animal or slaughterhouse sources, including treatment with lime or acids, beat extraction in aqueous solution, ion exchange chromatography, cross-flow filtration and various methods of drying can be used to derive the gelatin from recombinant collagen.

### Expression

The present methods of producing animal collagens and gelatins can be applied in a variety of recombinant systems available to those in the art. A number of these recombinant systems are described herein, although it is to be understood that applications of the present methods is not to be limited to the systems illustrated for example below.

In order to express the recombinant animal collagens and gelatins of the present invention, or polypeptides from which the recombinant gelatins can be derived, the encoding polynucleotide is inserted into an appropriate expression vector, i.e., a vector which contains the necessary elements for the transcription and translation of the inserted coding sequence, or in the case of an RNA viral vector, the necessary elements for replication and translation.

Methods which are well known to those skilled in the art can be used to construct expression vectors containing the polynucleotides of the invention and appropriate transcriptional/translational control signals. These methods include standard DNA cloning techniques, e.g., *in vitro* recombinant techniques, synthetic techniques and *in vivo* recombination/genetic recombination. (See, for example, the techniques described in Maniatis et al., *supra;* and Ausubel et al., *supra*.)

The expression elements of different systems vary in their strength and specificities. Depending on the host/vector system utilized, any of a number of suitable transcription and translation elements, including constitutive and inducible promoters, may be used in the expression vector. For example, when cloning in bacterial systems, inducible promoters such as pL of bacteriophage γ plac, ptrp, ptac (ptrp-lac hybrid promoter) and the like may be used; when cloning in insect cell systems, promoters such as the baculovirus polyhedron promoter may be used; when cloning in plant cell systems, promoters derived from the genome of plant cells (e.g., heat shock promoters; the promoter for the small subunit of RUBISCO; the promoter for the chlorophyll a/b binding protein) or from plant viruses (e.g., the 35S RNA promoter of CaMV; the coat protein promoter of TMV) may be used; when cloning in mammalian cell systems, promoters derived from the genome of mammalian cells (e.g., metallothionein promoter) or from mammalian viruses (e.g., the adenovirus late promoter; the vaccinia virus 7.5 K promoter) may be used; when generating cell lines that contain multiple copies of a collagen DNA, SV40-, BPV- and EBV-based vectors may be used with an appropriate selectable marker.

Specific initiation signals may also be required for efficient translation of inserted sequences. These signals include the ATG initiation codon and adjacent sequences. In cases where the entire collagen gene, including its own initiation codon and adjacent sequences, is inserted into the appropriate expression vector, no additional translational control signals may be needed. However, in cases where only a portion of a collagen coding sequence is inserted, exogenous translational control signals, including the ATG initiation codon, must be provided. Furthermore, the initiation codon must be in phase with the reading frame of the collagen coding sequence to ensure translation of the entire insert. These exogenous translational control signals and initiation codons can be of a variety of origins, both natural and synthetic. The efficiency of expression may be enhanced by the inclusion of appropriate transcription enhancer elements, transcription terminators, etc. (See, e.g., Bittner et al. (1987) Methods in Enzymol. 153:516-544).

The polypeptides of the invention may be expressed as secreted proteins. When the engineered cells used for expression of the proteins are non-human host cells, it is often advantageous to replace the secretory signal peptide of the collagen protein with an alternative secretory signal peptide which is more efficiently recognized by the host cell's secretory targeting machinery. The appropriate secretory signal sequence is particularly important in obtaining optimal fungal expression of mammalian genes. For example, see, e.g., Brake et al. (1984) Proc. Natl. Acad. Sci. USA 81:4642. Other signal sequences for prokaryotic, yeast, fungi, insect or mammalian cells are well known in the art, and one of ordinary skill could easily select a signal sequence appropriate for the host cell of choice.

The vectors of this invention may autonomously replicate in the host cell, or may integrate into the host chromosome. Suitable vectors with autonomously replicating sequences are well known for a variety of bacteria, yeast, and various viral replications sequences for both prokaryotes and eukaryotes. Vectors may integrate into the host cell genome when they have a nucleic acid sequence homologous to a sequence found in the genomic DNA of the host cell.

In one embodiment, the expression vectors of the present invention comprise a selectable marker, which encodes a product necessary for the host cell to grow and survive under certain conditions. Typical selection genes include genes encoding proteins that confer resistance to an antibiotic or other toxin (e.g., tetracycline, ampicillin, neomycin, methotrexate, etc.), proteins that complement an auxotrophic requirement of the host cell, etc. Other examples of selection genes include the herpes simplex virus thymidine kinase (Wigler et al. (1977) Cell 11:223), hypoxanthine-guanine phosphoribosyltransferase (Szybalska et al. (1962) Proc. Natl. Acad. Sci. USA 48:2026), and adenine phosphoribosyltransferase (Lowy et al. (1980) Cell 22:817) genes, which can be employed in *tk, hgprt*; or *aprt* cells, respectively.

Antimetabolite resistance can be used as the basis of selection, such as with the use of *dhfr* which confers resistance to methotrexate; *gpt*, which confers resistance to mycophenolic acid; *neo*, which confers resistance to the aminoglycoside G-418; and *hygro*, which confers resistance to hygromycin. (See, e.g., Wigler et al. (1980) Proc. Natl. Acad. Sci. USA 77:3567; O'Hare et al. (1981) Proc. Natl. Acad. Sci. USA 78:1527; Mulligan et al. (1981) Proc. Natl. Acad. Sci. USA 78:2072; Colberre-Garapin et al. (1981) J. Mol. Biol. 150:1; and Santerre et al. (1984) Gene 30:147.) Additional selectable genes include *trpB*, which allows cells to utilize indole in place of tryptophan; *hisD*, which allows cells to utilize histinol in place of histidine; and *odc* (ornithine decarboxylase) which confers resistance to the ornithine decarboxylase inhibitor, 2-(difluoromethyl)-DL-ornithine, DFMO. (See, e.g., Hartman et al. (1988) Proc. Natl. Acad. Sci. USA 85:8047 and McConlogue L., In: Current Communications in Molecular Biology, Cold Spring Harbor Laboratory, Ed. (1987)).

Elements necessary for the expression vectors of the invention include sequences for initiating transcription, e.g., promoters and enhancers. Promoters are untranslated sequences located upstream from the start codon of the structural gene that control the transcription of the nucleic acid under its control. Inducible promoters are promoters that alter their level of transcription initiation in response to a change in culture conditions, e.g., the presence or absence of a nutrient. One of skill in the art would know of a large number of promoters that would be recognized in host cells suitable for the present invention. These promoters are operably linked to the DNA encoding the collagen by removing the promoter from its native gene and placing the collagen encoding DNA 3' of the promoter sequence.

Promoters useful in the present invention include, but are not limited to, the lactose promoter, the alkaline phosphatase promoter, the tryptophan promoter, hybrid promoters such as the tac promoter, promoter for 3-phosphoglycerate kinase, other glycolytic enzyme promoters (hexokinase, pyruvate decarboxylase, phophofructosekinase, glucose-6-phosphate isomerase, etc.), the promoter for alcohol dehydrogenase, the metallothionein promoter, the maltose promoter, the galactose promoter, promoters from the viruses polyoma, fowlpox, adenovirus, bovine papilloma virus, avian sarcoma virus, cytomegalovirus, retroviruses, SV40, and promoters from target eukaryotes including the glucoamylase promoter from *Aspergillus*, the actin promoter or an immunoglobin promoter from a mammal, and native collagen promoters. (See, e.g., de Boer et al. (1983) Proc. Natl. Acad. Sci. USA 80:21-25; Hitzeman et al. (1980) J. Biol. Chem. 255:2073; Fiers et al. (1978) Nature 273:113; Mulligan and Berg (1980) Science 209:1422-1427; Pavlakis et al. (1981) Proc. Natl. Acad. Sci. USA 78:7398-7402; Greenway et al. (1982) Gene 18:355-360; Gray et al. (1982) Nature 295:503-508; Reyes et al. (1982) Nature 297:598-601; Canaani and Berg (1982) Proc. Natl. Acad. Sci. USA 79:5166-5170; Gorman et al. (1982) Proc. Natl. Acad. Sci. USA 79:6777-6781; and Nunberg et al. (1984) Mol. and Cell. Biol. 11(4):2306-2315.)

Transcription of the coding sequence from the promoter is often increased by inserting an enhancer sequence in the vector. Enhancers are cis-acting elements, usually about from 10 to 300 bp, that act to increase the rate of transcription initiation at a promoter. Many enhancers are known for both eukaryotes and prokaryotes, and one of ordinary skill could select an appropriate enhancer for the host cell of interest. (See, e.g., Yaniv (1982) Nature 297:17-18.)

In addition, a host cell strain may be chosen which modulates the expression of the inserted sequences, or modifies and processes the gene product in the specific fashion desired. Such modifications (e.g., glycosylation) and processing (e.g., cleavage) of protein products may be important for the function of the protein. Different host cells have characteristic and specific mechanisms for the post-translational processing and modification of proteins. Appropriate cells lines or host systems can be chosen to ensure the correct modification and processing of the foreign protein expressed. To this end, eukaryotic host cells which possess the cellular machinery for proper processing of the primary transcript, glycosylation, and phosphorylation of the gene product may be used. Such mammalian host cells include, but are not limited to, CHO, VERO, BHK, HeLa, COS, MDCK, 293, WI38, etc. Additionally, host cells may be engineered to express various enzymes to ensure the proper processing of the encoded polypeptide. For example, the gene for prolyl 4-hydroxylase may be co-expressed with a polynucleotide encoding a collagen or fragments or variants thereof to achieve proper hydroxylation.

For long-term, high-yield production of recombinant proteins, stable expression is preferred. For example, cell lines which stably express the collagens of the invention may be engineered. Rather than using expression vectors which contain viral origins of replication, host cells can be transformed with collagen encoding DNA controlled by appropriate expression control elements (e.g., promoter, enhancer, sequences, transcription terminators, polyadenylation sites, etc.), and a selectable marker. Following the introduction of foreign DNA, engineered cells may be allowed to grow for 1-2 days in an enriched media, and then are switched to a selective media. The selectable marker in the recombinant plasmid confers resistance to the selection and allows cells to stably integrate the plasmid into their chromosomes and grow to form foci which in turn can be cloned and expanded into cell lines. Thus, the present methods may advantageously be used to engineer cell lines which express a desired animal collagen or fragments or variants thereof.

For example, expression of the present polypeptides driven by the galactose promoters can be induced by growing the culture on a non-repressing, non-inducing sugar so that very rapid induction follows addition of galactose; by growing the culture in glucose medium and then removing the glucose by centrifugation and washing the cells before resuspension in galactose medium; and by growing the cells in medium containing both glucose and galactose so that the glucose is preferentially metabolized before galactose-induction can occur.

The vectors expressing the polypeptides of the present invention, and the vectors expressing polynucleotides encoding any post-translational enzymes desired may be introduced into host cells to produce the encoded polypeptides, using techniques known to one of skill in the art. For example, host cells are transfected or infected or transformed with the above-described expression vectors, and cultured in nutrient media appropriate for selecting transductants or transformants containing the collagen encoding vector. Cell transfection can be carried out by a variety of methods available to those of skill in the art, such as, for example, by calcium phosphate precipitation, electroporation, and lipofection techniques. (See, e.g., Maniatis et al., *supra*, Ohta T. (1996) Nippon Rinsho 54(3):757-764; Totter and Wood (1996) Mol Biotechnol 6(3):329-334; Mann and King (1989) J Gen Virol 70:3501-3505; and Hartig et al. (1991) Biotechniques 11(3):310.)

In one embodiment, a method is contemplated in which more than one of the expression vectors encoding for the polypeptides of the present invention are inserted into cells, so that, e.g., trimeric collagens can be synthesized.

In another method of the present invention, production of homotrimeric collagen is contemplated.

Host cells containing coding sequence and expressing the biologically active gene product may be identified by any number of techniques known in the art. Such techniques include, for example, detecting the formation of nucleic acid hybridization complexes, detecting the presence or absence of marker gene functions assessing the level of transcription as measured by the expression ofmRNA transcripts in the host cell, and detecting gene product as measured by immunoassay or by biological activity.

In the first approach, the presence of the present polynucleotide can be detected by, for example, detection of DNA-DNA or DNA-RNA hybridization complexes, or by amplification using probes comprising nucleotide sequences homologous to the animal collagen coding sequence, or portions, or derivatives thereof. Amplification-based assays involve the use of oligonucleotides or oligomers based on sequences homologous to the coding sequence of interest to detect transformants containing the encoding polynucleotides.

In the second approach, the recombinant expression vector/host system is identified and selected based upon the presence or absence of certain marker gene functions (e.g., thymidine kinase activity, resistance to antibiotics, resistance to methotrexate, transformation phenotype, occlusion body formation in baculovirus, etc.). For example, if the coding sequence is inserted within a marker gene sequence of the vector, recombinant cells containing coding sequence can be identified by the absence of the marker gene function. Alternatively, a marker gene can be placed in tandem with the coding sequence under the control of the same or different promoter used to control the expression of the coding sequence. Expression of the marker in response to induction or selection indicates expression of the coding sequence.

In the third approach, transcriptional activity of the coding region can be assessed by hybridization assays. For example, RNA can be isolated and analyzed by northern blot using a probe homologous to the coding sequence or particular portions thereof. Alternatively, total nucleic acids of the host cell may be extracted and assayed for hybridization to such probes.

In the fourth approach, the expression of a protein product can be assessed immunologically, for example by Western blots, immunoassays such as radioimmuno-precipitation, enzyme-linked immunoassays, and the like.

In one embodiment, the animal collagens of the present invention are secreted into the culture medium, and can be purified to homogeneity by various methods known in the art, for example, by chromatography. In one embodiment, recombinant animal collagens of the present invention are purified by size exclusion chromatography. However, other purification techniques known in the art can also be used, including ion exchange chromatography, and reverse-phase chromatography. (See, e.g., Maniatis et al., *supra,* Ausubel et al., *supra,* and Scopes (1994) Protein Purification: Principles and Practice, Springer-Verlag New York, Inc., NY.)

The present methods can be used in, although are not limited in application to, the expression systems listed below.

### Prokaryotic

In prokaryotic systems, such as bacterial systems, a number of expression vectors may be advantageously selected depending upon the use intended for the expressed polypeptide. For example, when large quantities of the animal collagens and gelatins of the invention are to be produced, such as for the generation of antibodies, vectors which direct the expression of high levels of fusion protein products that are readily purified may be desirable. Such vectors include, but are not limited to, the *E. coli* expression vector pUR278 (Ruther et al. (1983) EMBO J. 2:1791), in which the coding sequence may be ligated into the vector in frame with the lac Z coding region so that a hybrid AS-lac Z protein is produced; pIN vectors (Inouye et al. (1985) Nucleic Acids Res. 13:3101-3109 and Van Heeke et al. (1989) J. Biol. Chem. 264:5503-5509); and the like. pGEX vectors may also be used to express foreign polypeptides as fusion proteins with glutathione S-transferase (GST). In general, such fusion proteins are soluble and can easily be purified from lysed cells by adsorption to glutathione-agarose beads followed by elution in the presence of free glutathione. The pGEX vectors are designed to include thrombin or factor Xa protease cleavage sites so that the cloned polypeptide of interest can be released from the GST moiety.

### Yeast

In one embodiment, the present polypeptides are produced in a yeast expression system. In yeast, a number of vectors containing constitutive or inducible promoters known in the art may be used. (See, e.g., Ausubel et al., *supra*, Vol. 2, Chapter 13; Grant et al. (1987) Expression and Secretion Vectors for Yeast, in Methods in Enzymology, Ed. Wu & Grossman, Acad. Press, N.Y. 153:516-544; Glover (1986) DNA Cloning, Vol. II, IRL Press, Wash., D.C., Ch. 3; Bitter (1987) Heterologous Gene Expression in Yeast, in Methods in Enzymology, Eds. Berger & Kimmel, Acad. Press, N.Y. 152:673-684; and The Molecular Biology of the Yeast Saccharomyces, Eds. Strathern et al., Cold Spring Harbor Press, Vols. I and II (1982).)

Polypeptides of the present invention can be expressed using host cells, for example, from the yeast *Saccharomyces cerevisiae*. This particular yeast can be used with any of a large number of expression vectors. Commonly employed expression vectors are shuttle vectors containing the 2µ origin of replication for propagation in yeast and the Col E1 origin for *E. coli*, for efficient transcription of the foreign gene. A typical example of such vectors based on 2µ plasmids is pWYG4, which has the 2µ ORI-STB elements, the GAL1-10 promoter, and the 2µ D gene terminator. In this vector, an Ncol cloning site is used to insert the gene for the polypeptide to be expressed, and to provide the ATG start codon. Another expression vector is pWYG7L, which has intact 2α ORI, STB, REP1 and REP2, and the GAL1-10 promoter, and uses the FLP terminator. In this vector, the encoding polynucleotide is inserted in the polylinker with its 5' ends at a *BamHI* or *Ncol* site. The vector containing the inserted polynucleotide is transformed into *S. cerevisiae* either after removal of the cell wall to produce spheroplasts that take up DNA on treatment with calcium and polyethylene glycol or by treatment of intact cells with lithium ions.

Alternatively, DNA can be introduced by electroporation. Transformants can be selected, for example, using host yeast cells that are auxotrophic for leucine, tryptophane, uracil, or histidine together with selectable marker genes such as LEU2, TRP1, URA3, HIS3, or LEU2-D.

In one embodiment of the invention, the present polynucleotides are introduced into host cells from the yeast *Pichia*. Species of non-*Saccharomyces* yeast such as *Pichia pastoris* appear to have special advantages in producing high yields of recombinant protein in scaled up procedures. Additionally, a *Pichia* expression kit is available from Invitrogen Corporation (San Diego, CA).

There are a number of methanol responsive genes in methylotrophic yeasts such as *Pichia pastoris*, the expression of each being controlled by methanol responsive regulatory regions, also referred to as promoters. Any of such methanol responsive promoters are suitable for use in the practice of the present invention. Examples of specific regulatory regions include the AOX1 promoter, the AOX2 promoter, the dihydroxyacetone synthase (DAS), the P40 promoter, and the promoter for the catalase gene from *P. pastoris*, etc.

In other embodiments, the present invention contemplates the use of the methylotrophic yeast *Hansenula polymorpha*. Growth on methanol results in the induction of key enzymes of the methanol metabolism, such as MOX (methanol oxidase), DAS (dihydroxyacetone synthase), and FMHD (formate dehydrogenase). These enzymes can constitute up to 30-40% of the total cell protein. The genes encoding MOX, DAS, and FMDH production are controlled by strong promoters induced by growth on methanol and repressed by growth on glucose. Any or all three of these promoters may be used to obtain high-level expression of heterologous genes in *H. polymorpha*. Therefore, in one aspect of the invention, a polynucleotide encoding animal collagen or fragments or variants thereof is cloned into an expression vector under the control of an inducible *H. polymorpha* promoter. If secretion of the product is desired, a polynucleotide encoding a signal sequence for secretion in yeast is fused in frame with the polynucleotide. In a further embodiment, the expression vector preferably contains an auxotrophic marker gene, such as URA3 or LEU2, which may be used to complement the deficiency of an auxotrophic host.

The expression vector is then used to transform *H. polymorpha* host cells using techniques known to those of skill in the art. A useful feature of *H. polymorpha* transformation is the spontaneous integration of up to 100 copies of the expression vector into the genome. In most cases, the integrated polynucleotide forms multimers exhibiting a head-to-tail arrangement. The integrated foreign polynucleotide has been shown to be mitotically stable in several recombinant strains, even under non-selective conditions. This phenomena of high copy integration further adds to the high productivity potential of the system.

### Fungi

Filamentous fungi may also be used to produce the present polypeptides. Vectors for expressing and/or secreting recombinant proteins in filamentous fungi are well known, and one of skill in the art could use these vectors to express the recombinant animal collagens of the present invention.

### Plant

In one aspect, the present invention contemplates the production of animal collagens and gelatins in plants and plant cells. In cases where plant expression vectors are used, the expression of sequences encoding the collagens of the invention may be driven by any of a number of promoters. For example, viral promoters such as the 35S RNA and 19S RNA promoters of CaMV (Brisson et al. (1984) Nature 310:511-514), or the coat protein promoter of TMV (Takamatsu et al. (1987) EMBO J. 6:307-311) may be used; alternatively, plant promoters such as the small subunit of RUBISCO (Coruzzi et al. (1984) EMBO J. 3:1671-1680; Broglie et al. (1984) Science 224:838-843) or heat shock promoters, e.g., soybean hsp17.5-E or hsp17.3-B (Gurley et al. (1986) Mol. Cell. Biol. 6:559-565) may be used. These constructs can be introduced into plant cells by a variety of methods known to those of skill in the art, such as by using Ti plasmids, Ri plasmids, plant virus vectors, direct DNA transformation, microinjection, electroporation, etc. For reviews of such techniques see, for example, Weissbach & Weissbach, Methods for Plant Molecular Biology, Academic Press, NY, Section VIII, pp. 421-463 (1988); Grierson & Corey, Plant Molecular Biology, 2d Ed., Blackie, London, Ch. 7-9 (1988); Transgenic Plants: A Production System for Industrial and Pharmaceutical Proteins, Owen and Pen eds., John Wiliey & Sons, 1996; Transgenic Plants, Galun and Breiman eds, Imperial College Press, 1997; and Applied Plant Biotechnology, Chopra, Malik, and Bhat eds., Science Publishers, Inc., 1999.

Plant cells do not naturally produce sufficient amounts of post-translational enzymes to efficiently produce stable collagen. Therefore, the present invention provides that, where hydroxylation is desired, plant cells used to express the present animal collagens are supplemented with the necessary post-translational enzymes to sufficiently produce stable collagen. In a preferred embodiment of the present invention, the post-translational enzyme is prolyl 4-hydroxylase.

Methods of producing the present animal collagens or gelatins in plant systems may be achieved by providing a biomass from plants or plant cells, wherein the plants or plant cells comprise at least one coding sequence is operably linked to a promoter to effect the expression of the polypeptide, and the polypeptide is then extracted from the biomass. Alternatively, the polypeptide can be non-extracted, i.e., expressed into the endosperm, etc.

Plant expression vectors and reporter genes are generally known in the art. (See, e.g., Gruber et al. (1993) in Methods of Plant Molecular Biology and Biotechnology, CRC Press.) Typically, the expression vector comprises a nucleic acid construct generated, for example, recombinantly or synthetically, and comprising a promoter that functions in a plant cell, wherein such promoter is operably linked to a nucleic acid sequence encoding an animal collagen or fragments or variants thereof, or a post-translational enzyme important to the biosynthesis of collagen.

Promoters drive the level of protein expression in plants. To produce a desired level of protein expression in plants, expression may be under the direction of a plant promoter. Promoters suitable for use in accordance with the present invention are generally available in the art. (See, e.g., PCT Publication No. WO 91/19806.) Examples of promoters that may be used in accordance with the present invention include non-constitutive promoters or constitutive promoters. These promoters include, but are not limited to, the promoter for the small subunit of ribulose-1,5-bis-phosphate carboxylase; promoters from tumor-inducing plasmids of *Agrobacterium tumefaciens*, such as the RUBISCO nopaline synthase (NOS) and octopine synthase promoters; bacterial T-DNA promoters such as *mas* and *ocs* promoters; and viral promoters such as the cauliflower mosaic virus (CaMV) 19S and 35S promoters or the figwort mosaic virus 35S promoter.

The polynucleotide sequences of the present invention may be under the transcriptional control of a constitutive promoter, directing expression of the collagen or post-translational enzyme in most tissues of a plant. In one embodiment, the polynucleotide sequence is under the control of the cauliflower mosaic virus (CaMV) 35S promoter. The double-stranded caulimorvirus family has provided the single most important promoter expression for transgene expression in plants, in particular, the 35S promoter. (See, e.g., Kay et al. (1987) Science 236:1299.) Additional promoters from this family such as the figwort mosaic virus promoter, etc., have been described in the art, and may also be used in accordance with the present invention. (See, e.g., Sanger et al. (1990) Plant Mol. Biol. 14:433-443; Medberry et al. (1992) Plant Cell 4:195-192; and Yin and Beachy (1995) Plant J. 7:969-980.)

The promoters used in the polynucleotide constructs of the present invention may be modified, if desired, to affect their control characteristics. For example, the CaMV promoter may be ligated to the portion of the RUBISCO gene that represses the expression of RUBISCO in the absence of light, to create a promoter which is active in leaves, but not in roots. The resulting chimeric promoter may be used as described herein.

Constitutive plant promoters having general expression properties known in the art may be used with the expression vectors of the present invention. These promoters are abundantly expressed in most plant tissues and include, for example, the actin promoter and the ubiquitin promoter. (See, e.g., McElroy et al. (1990) Plant Cell 2:163-171; and Christensen et al. (1992) Plant Mol. Biol. 18:675-689.)

Alternatively, the polypeptide of the present invention may be expressed in a specific tissue, cell type, or under more precise environmental conditions or developmental control. Promoters directing expression in these instances are known as inducible promoters. In the case where a tissue-specific promoter is used, protein expression is particularly high in the tissue from which extraction of the protein is desired. Depending on the desired tissue, expression may be targeted to the endosperm, aleurone layer, embryo (or its parts as scutellum and cotyledons), pericarp, stem, leaves tubers, roots, etc. Examples of known tissue-specific promoters include the tuber-directed class I patatin promoter, the promoters associated with potato tuber ADPGPP genes, the soybean promoter of β-conglycinin (7S protein) which drives seed-directed transcription, and seed-directed promoters from the zein genes of maize endosperm. (See, e.g., Bevan et al. (1986) Nucleic Acids Res. 14: 4625-38; Muller et al. (1990) Mol. Gen. Genet. 224:136-46; Bray (1987) Planta 172:364-370; and Pedersen et al. (1982) Cell 29:1015-26.)

In a preferred embodiment, the present polypeptides are produced in seed by way of seed-based production techniques using, for example, canola, corn, soybeans, rice and barley seed. In such a process, for example, the product is recovered during seed germination. (See, e.g., PCT Publication Numbers WO 9940210; WO 9916890; WO 9907206; U.S. Patent No. 5,866,121; U.S. Patent No. 5,792,933; and all references cited therein.) Promoters that may be used to direct the expression of the polypeptides may be heterologous or non-heterologous. These promoters can also be used to drive expression of antisense nucleic acids to reduce, increase, or alter concentration and composition of the present animal collagens in a desired tissue.

Other modifications that may be made to increase and/or maximize transcription of the present polypeptides in a plant or plant cell are standard and known to those in the art. For example a vector comprising a polynucleotide sequence encoding a recombinant animal collagen or gelatin, or a polypeptide from which the recombinant animal gelatin may be derived, or a fragment or variant thereof, operably linked to a promoter may further comprise at least one factor that modifies the transcription rate of collagen or related post-translational enzymes, including, but not limited to, peptide export signal sequence, codon usage, introns, polyadneylation, and transcription termination sites. Methods of modifying constructs to increase expression levels in plants are generally known in the art. (See, e.g. Rogers et al. (1985) J. Biol. Chem. 260:3731; and Cornejo et al. (1993) Plant Mol Biol 23:567-58.) In engineering a plant system that affects the rate of transcription of the present collagens and related post-translational enzymes, various factors known in the art, including regulatory sequences such as positively or negatively acting sequences, enhancers and silencers, as well as chromatin structure can affect the rate of transcription in plants. The present invention provides that at least one of these factors may be utilized in expressing the recombinant animal collagens and gelatins described herein.

The vectors comprising the present polynucleotides will typically comprise a marker gene which confers a selectable phenotype on plant cells. Usually, the selectable marker gene will encode antibiotic resistance, with suitable genes including at least one set of genes coding for resistance to the antibiotic spectinomycin, the streptomycin phophotransferase (SPT) gene coding for streptomycin resistance, the neomycin phophotransferase (NPTH) gene encoding kanamycin or geneticin resistance, the hygromycin resistance, genes coding for resistance to herbicides which act to inhibit the action of acetolactate synthase (ALS), in particular, the sulfonylurea-type herbicides (e.g., the acetolactate synthase (ALS) gene containing mutations leading to such resistance in particular the S4 and/or Hra mutations), genes coding for resistance to herbicides which act to inhibit action of glutamine synthase, such as phophinothricin or basta (e.g. the bar gene), or other similar genes known in the art. The bar gene encodes resistance to the herbicide basta, the *nptII* gene encodes resistance to the antibiotics kanamycin and geneticin, and the ALS gene encodes resistance to the herbicide chlorsulfuron.

Typical vectors useful for expression of foreign genes in plants are well known in the art, including, but not limited to, vectors derived from the tumor-inducing (Ti) plasmid of *Agrobacterium tumefaciens*. These vectors are plant integrating vectors, that upon transformation, integrate a portion of the DNA into the genome of the host plant. (See, e.g., Rogers et al. (1987) Meth. In Enzymol. 153:253-277; Schardl et al. (1987) Gene 61:1-11; and Berger et al., Proc. Natl. Acad. Sci. U.S.A. 86:8402-8406.)

Vectors comprising sequences encoding the present polypeptides and vectors comprising post-translational enzymes or subunits thereof may be co-introduced into the desired plant. Procedures for transforming plant cells are available in the art, for example, direct gene transfer, *in vitro* protoplast transformation, plant virus-mediated transformation, liposome-mediated transformation, microinjection, electroporation, *Agrobacterium* mediated transformation, and particle bombardment. (See, e.g., Paszkowski et al. (1984) EMBO J. 3:2717-2722; U.S. Patent No. 4,684,611; European Application No. 0 67 553; U.S. Patent No. 4,407,956; U.S. Patent No. 4,536,475; Crossway et al. (1986) Biotechniques 4:320-334; Riggs et al. (1986) Proc. Natl. Acad. Sci USA 83:5602-5606; Hinchee et al. (1988) Biotechnology 6:915-921; and U.S. Patent No. 4,945,050.) Standard methods for the transformation of, e.g., rice, wheat, corn, sorghum, and barley are described in the art. (See, e.g., Christou et al. (1992) Trends in Biotechnology 10: 239 and Lee et al. (1991) Proc. Nat'l Acad. Sci. USA 88:6389.) Wheat can be transformed by techniques similar to those employed for transforming corn or rice. Furthermore, Casas et al. (1993) Proc. Nat'l Acad. Sci. USA 90:11212, describe a method for transforming sorghum, while Wan et al. (1994) Plant Physiol. 104: 37, teach a method for transforming barley. Suitable methods for corn transformation are provided by Fromm et al. (1990) Bio/Technology 8:833 and by Gordon-Kamm et al., *supra.*

Additional methods that may be used to generate plants that produce animal collagens of the present invention are well established in the art. (See, e.g., U.S. Patent No. 5,959,091; U.S. Patent No. 5,859,347; U.S. Patent No. 5,763,241; U.S. Patent No. 5,659,122; U.S. Patent No. 5,593,874; U.S. Patent No. 5,495,071; U.S. Patent No. 5,424,412; U.S. Patent No. 5,362,865; U.S. Patent No. 5,229,112; U.S. Patent No. 5,981,841; U.S. Patent No. 5,959,179; U.S. Patent No. 5,932,439; U.S. Patent No. 5,869,720; U.S. Patent No. 5,804,425; U.S. Patent No. 5,763,245; U.S. Patent No. 5,716,837; U.S. Patent No. 5,689,052; U.S. Patent No. 5,633,435; U.S. Patent No. 5,631,152; U.S. Patent No. 5,627,061; U.S. Patent No. 5,602,321; U.S. Patent No. 5,589,612; U.S. Patent No. 5,510,253; U.S. Patent No. 5,503,999; U.S. Patent No. 5,378,619; U.S. Patent No. 5,349,124; U.S. Patent No. 5,304,730; U.S. Patent No. 5,185,253; U.S. Patent No. 4,970,168; European Publication No. EPA 00709462; European Publication No. EPA 00578627; European Publication No. EPA 00531273; European Publication No. EPA 00426641; PCT Publication No. WO 99/31248; PCT Publication No. WO 98/58069; PCT Publication No. WO 98/45457; PCT Publication No. WO 98/31812; PCT Publication No. WO 98/08962; PCT Publication No. WO 97/48814; PCT Publication No. WO 97/30582; and PCT Publication No. WO 9717459.)

### Insect

Another alternative expression system used in accordance with the present methods is an insect system. Baculoviruses are very efficient expression vectors for the large scale production of various recombinant proteins in insect cells. The methods as described in, for example, Luckow et al. (1989) Virology 170:31-39 and Gruenwald, S. and Heitz, J. (1993) Baculovirus Expression Vector System: Procedures & Methods Manual, Pharmingen, San Diego, CA, can be employed to construct expression vectors containing a collagen coding sequence for the collagens of the invention and the appropriate transcriptional/translational control signals. For example, recombinant production of proteins can be achieved in insect cells, by infection of baculovirus vectors encoding the polypeptide. In one aspcect of the present invention, production of recombinant polypeptides with stable triple helices can involve the co-infection of insect cells with three baculoviruses, one encoding the animal collagen to be expressed and one each encoding the a subunit and β subunit of prolyl 4-hydroxylase. This insect cell system allows for production of recombinant proteins in large quantities. In one such system, *Autographa californica* nuclear polyhidrosis virus (AcNPV) is used as a vector to express foreign genes. The virus grows in *Spodoptera frugiperda* cells. Coding sequence for the polypeptides of the invention may be cloned into non-essential regions (for example the polyhedron gene) of the virus and placed under control of an AcNPV promoter (for example, the polyhedron promoter). Successful insertion of a coding sequence will result in inactivation of the polyhedron gene and production of non-occluded recombinant virus (i.e., virus lacking the proteinaceous coat coded for by the polyhedron gene). These recombinant viruses are then used to infect *Spodoptera frugiperda* cells in which the inserted gene is expressed. (See, e.g., Smith et al. (1983) J. Virol. 46:584; and U.S. Patent No. 4,215,051). Further examples of this expression system may be found in, for example, Ausubel et al., *supra*.

### Animal

In animal host cells, a number of expression systems may be utilized. In cases where an adenovirus is used as an expression vector, polynucleotide sequences of the present invention may be ligated to an adenovirus transcription/translation control complex, e.g., the late promoter and tripartite leader sequence. This chimeric gene may then be inserted in the adenovirus genome by *in vitro* or *in vivo* recombination. Insertion in a non-essential region of the viral genome (e.g., region E1 or E3) will result in a recombinant virus that is viable and capable of expressing the encoded polypeptides in infected hosts. (See, e.g., Logan & Shenk, Proc. Natl. Acad. Sci. USA 81:3655-3659 (1984)). Alternatively, the vaccinia 7.5 K promoter may be used. (See, e.g., Mackett et al. (1982) Proc. Natl. Acad. Sci. USA 79:7415-7419; Mackett et al. (1982) J. Virol. 49:857-864; and Panicali et al. (1982) Proc. Natl. Acad. Sci. USA 79:4927-4931.

A preferred expression system in mammalian host cells is the Semliki Forest virus. Infection of mammalian host cells, for example, baby hamster kidney (BHK) cells and Chinese hamster ovary (CHO) cells can yield very high recombinant expression levels. Semliki Forest virus is a preferred expression system as the virus has a broad host range such that infection of mammalian cell lines will be possible. More specifically, it is expected that the use of the Semliki Forest virus can be used in a wide range of hosts, as the system is not based on chromosomal intergration, and therefore will be a quick way of obtaining modifications of the recombinant animal collagens in studies aiming at identifying structure-function relationships and testing the effects of various hybrid molecules. Methods for constructing Semliki Forest virus vectors for expression of exogenous proteins in mammalian host cells are described in, for example, Olkkonen et al. (1994) Methods Cell Biol 43:43-53.

Non-human Transgenic animals may also be used to express the polypeptides of the present invention. Such systems can be constructed by operably linking the polynucleotide of the invention to a promoter, along with other required or optional regulatory sequences capable of effecting expression in mammary glands. Likewise, required or optional post-translational enzymes may be produced simultaneously in the target cells employing suitable expression systems. Methods of using non-human transgenic animals to recombinantly produce proteins are known in the art. (See, e.g., U.S. Patent No. 4,736,866; U.S. Patent No. 5,824,838; U.S. Patent No. 5,487,992; and U.S. Patent No. 5,614,396.)

### Uses of Collagens and Gelatins

The recombinant collagens and gelatins of the present invention are useful in a variety of applications. Collagen is widely used in numerous applications in the medical, pharmaceutical, food, and cosmetic industries. For example, collagen is an important component of arterial sealants, bone grafts, drug delivery systems, dermal implants, hemostats, and incontinence implants. In treatments for autoimmune disorders such as rheumatoid arthritis, collagen has been evaluated in trials for its potential to induce oral-tolerance. Collagen is also applied in food products such as sausage casings, and other collagen-based casings derived from, for example, porcine, bovine, and ovine sources. In health and beauty applications, collagen can be found, for example, in cosmetics or facial and skin products such as moisturizers. To date, various collagens used in various applications are derived from animal sources using enzymatic and chemical processes. For example, commercially available bovine collagen is isolated from bovine tissues and bones, and is comprised of a mixture of primarily types I and III collagen. This form of collagen is also used as an injectable device in humans.

Gelatin appears in the manufacture or as a component of various pharmaceutical and medical products and devices, including pharmaceutical stabilizers, e.g., drug and vaccine, plasma extenders, sponges, hard and soft gelatin capsules, suppositories, etc. Gelatin's film-forming capabilities are employed in various film coating systems designed specifically for pharmaceutical oral solid dosage forms, including controlled release capsules and tablets.

Gelatin in various edible forms has long been used in the food and beverage industries. Gelatin serves as an emulsifier and thickener in various whipped toppings, as well as in soups and sauces. Gelatin is used as a flocculating agent in clarifying and fining various beverages, including wines and fruit juices. Gelatin is used in various low and reduced fat products as a thickener and stabilizer, and appears elsewhere as a fat substitute. Gelatin is also widely used in micro-encapsulation of flavorings, colors, and vitamins. Gelatin can also be used as a protein supplement in various high energy and nutritional beverages and foods, such as those prevalent in the weight-loss and athletic industries. As a film-former, gelatin is used in coating fruits, meats, deli items, and in various confectionery products, including candies and gum, etc.

In the cosmetics industry, gelatin appears in a variety of hair care and skin care products. Gelatin is used as a thickener and bodying agent in a number of shampoos, mousses, creams, lotions, face masks, lipsticks, manicuring solutions and products, and other cosmetic devices and applications. Gelatin is also used in the cosmetics industry in micro-encapsulation and packaging of various products.

Gelatin is used in a wide range of industrial applications. For example, gelatin is widely used as a glue and adhesive in various manufacturing processes. Gelatin can be used in various adhesive and gluing formulations, such as in the manufacture of remoistenable gummed paper packaging tapes, wood gluing, paper bonding of various grades of box boards and papers, and in various applications which provide adhesive surfaces which can be reactivated by remoistening.

Gelatin serves as a light-sensitive coating in various electronic devices and is used as a photoresist base in various photolithographic processes, for example, in color television and video camera manufacturing. In semiconductor manufacturing, gelatin is used in constructing lead frames and in the coating of various semiconductor elements. Gelatin is used in various printing processes and in the manufacturing of special quality papers, such as that used in bond and stock certificates, etc.

Gelatin is used in a variety of photographic applications, e.g., as a carrier for various active components in photographic solutions, including solutions used in X-ray and photographic film development. Gelatin, long used in various photoengraving techniques, is also included as a component of various types of film, and is heavily used in silver halide chemistry in various layers of film and paper products. Silver gelatin film appears in the form of microfiche film and in other forms of information storage. Gelatin is used as a self-sealing elements of various films, etc.

Gelatin has also been a valuable substance for use in various laboratory applications. For example, gelatin can be used in various cell culture applications, providing a suitable surface for cell attachment and growth, e.g., plate or flask coating, or providing a surface for cell attachment and growth. Hydrolyzed or low gel strength gelatin is used as a biological buffer in various processes, for example, in coating and blocking solutions used in assays such as enzyme-linked immunosorbent assays (ELISAs) and other immunoassays. Gelatin is also a component in various gels used for biochemical and electrophoretic analysis, including enzymography gels.

### EXAMPLES

The following examples are provided solely to illustrate the claimed invention. The present invention, however, is not limited in scope by the exemplified embodiments, which are intended as illustrations of single aspects of the invention only. Indeed, various modifications of the invention in addition to those described herein will become apparent to those skilled in the art from the foregoing description and accompanying drawings.

### Example 1: Sequencing of Bovine Procollagen Type Iα1

Experiments were performed to generate α1(I) collagen gene fragments by PCR from a commercial bovine aorta smooth muscle cDNA library (Stratagene #936705) that had been a successful source of bovine collagen (I) alpha 2 gene fragments in initial PCR experiments. In this initial screening process, PCR primers were designed from the bovine mRNA sequence (Shirai et al. (1998) Matrix Biology 17:85-88) of collagen (I) α2, and PCR amplifications performed, and DNA fragments were obtained. Although the commercial library was shown to contain the complete coding region of the bovine collagen (I) alpha 2 gene, attempts to generate fragments of the bovine α1(I) collagen gene using a variety of human α1(I) collagen sequence PCR primers proved unsuccessful. An alternative source of a cDNA pool likely to contain a bovine α1(I) collagen transcript was sought.

An ATCC bovine skin cell line (CRL-6054; skin, normal, bovine) was grown to approximately 60% confluency and total RNA was isolated (Qiagen RNeasy). A cDNA pool was prepared from the resulting RNA by RT-PCR (Clontech RT-for-PCR reagents). This cDNA pool was used as the template source for subsequent PCR experiments of overlapping gene fragments.

Primers were designed from known human α1(I) collagen mRNA sequence, and used to amplify overlapping segments of the open reading frame (ORF) of the gene. (Mackay et al. (1993) Human Molecular Genetics 2(8):1155-1160). The PCR primers were engineered to amplify fragments located in the triple helical coding region of the human α1(I) collagen gene and are set forth in Table 1.

**Table 1**

| **SEQ ID NO:** | **PRIMER** | **SEQUENCE** |
|---|---|---|
| 13 | SSCP 1F | CCGGCTCCTGCTCCTCTTAG |
| 14 | SSCP 1REV | GCCAGGAGCACCAGCAATAC |
| 15 | SSCP2F | GCTGATGGACAGCCTGGTGC |
| 16 | SSCP2REV | GCCCTGGAAGACCAGCTGCA |
| 17 | SSCP 3F | CCTGGCCTTAAGGGAATGCC |
| 18 | SSCP3REV | GCGCCAGGAGAACCGTCTCG |
| 19 | SSCP4F | CCGAAGGTTCCCCTGGACGA |
| 20 | SSCP4REV | CGGTCATGCTCTCGCCGAAC |

The primers were used to obtain four overlapping bovine PCR fragments covering the triple helical portion of the bovine α1(I) collagen gene. PCR (Clontech, Advantage GC-Rich cDNA PCR kit; all PCR primers used @ 100 pmol each per reaction) was performed using a thermal cycler (Hybaid, non-refrigerated) under the following conditions:

| | |
|---|---|
| Step 1: | 94°C for 4 minutes |
| Step 2: | 28 cycles of : |
| | 68°C for 3 minutes |
| | 94°C for 30 seconds |
| | 60°C for 30 seconds |
| Step 3: | 68°C for 10 minutes |
| | 30°C for 1 second |
| | Hold @ room temperature |

All PCR products were initially screened by gel electrophoresis, and those of the predicted size were purified by agarose gel electrophoresis and/or column purification (Qiagen Qiaquick). To facilitate sequencing, the selected PCR fragments were cloned into a vector (pCRII-TOPO kit, Invitrogen). Multiple clones of each PCR fragment were sequenced with an external vector sequencing primers (M13 forward and reverse) using an ABI 373 automated sequencer (ABI PRISM® BigDye™ Terminator Cycle Sequencing Kit, Perkin-Elmer). Sequence data obtained was analyzed with the use of SEQMAN software (DNASTAR) and a consensus sequence determined for the cloned fragments.

The resulting bovine α1(I) collagen sequence obtained was used to design internal bovine collagen sequencing primers, which were then used to complete the sequencing of these bovine clones. These primers were designed with the aid of primer design software (RightPrimer, BioDisk), and are set forth in Table 2.

**Table 2**

| **SEQ ID NO:** | **PRIMER** | **SEQUENCE** |
|---|---|---|
| 21 | B C1A1 SP 502F | CCCCAGTTGTCTTACGGCTATG |
| 22 | B C1A1 SP 502REV | CATAGCCGTAAGACAACTGGGG |
| 23 | B C1A1 SP 886F | GGTAGCCCCGGTGAAAATG |
| 24 | B C1A1 SP 886REV | CATTTTCACCGGGGCTACC |
| 25 | B C1A1 SP 1302F | GCCCCAAGGGTAACAGCGGT |
| 26 | B C1A1 SP 1302REV | ACCGCTGTTACCCTTGGGGC |
| 27 | B C1A1 SP 1560F | TCCTGGCCCTGCTGGCCCCAAA |
| 28 | B C1A1 SP 1560REV | TTTGGGGCCAGCAGGGCCAGGA |
| 29 | B C1A1 SP 1770F | TGGACCTAAAGGTGCTGCTGGA |
| 30 | B C1A1 SP 1770REV | TCCAGCAGCACCTTTAGGTCCA |
| 31 | B C1A1 SP 1997F | GAACAGGGTGTTCCTGGAGA |
| 32 | B C1A1 SP 1997REV | TCTCCAGGAACACCCTGTTC |
| 33 | B C1A1 SP 2289F | GGCAAAGATGGCGTCCGT |
| 34 | B C1A1 SP 2289REV | ACGGACGCCATCTTTGCC |
| 35 | B C1A1 SP 2592F | GCTAAAGGCGAACCTGGCGA |
| 36 | B C1A1 SP 2592REV | TCGCCAGGTTCGCCTTTAGC |
| 37 | B C1A1 SP 3198F | GCCGGCAAGAGCGGTGATCGT |
| 38 | B C1A1 SP 3198REV | ACGATCACCGCTCTTGCCGGC |
| 39 | B C1A1 SP 3648F | CGATGGTGGCCGCTACTAC |
| 40 | B C1A1 SP 3648REV | GTAGTAGCGGCCACCATCG |
| 41 | B C1A1 SP 4007F | AGAGCATGACCGAAGGGCGAATT |
| 42 | B C1A1 SP 4007REV | AATTCGCCCTTCGGTCATGCTCT |

After producing bovine PCR products with the eight SSCP human primers shown in Table 1 (SEQ ID NOs:13 through 20), three additional PCR fragments were amplified, overlapping the initial bovine clones, and extending to the putative ends (by analogy with the human α1(I) collagen sequence) of the ORF. The PCR primers used for this amplification are set forth in Table 3.

**Table 3**

| **SEQ ID NO:** | **PRIMER** | **SEQUENCE** |
|---|---|---|
| 43 | H AVR II F | TTAATTCCTAGGATGTTCAGCTTTGTGGACCTCCGGCTC |
| 44 | H EAR 1 F | TGCCACTCTGACTGGAAGAGTGGAGAGTACTG |
| 45 | H NOT1 REV | TTTTCCTTTTGCGGCCGCTTACAGGAAGCAGACAGGGCCAACGTC |

The resulting DNA fragments were cloned and sequenced, and a consensus sequence was established for most of the ORF of the gene by pairing of the following primers: H AVR II (SEQ ID NO:43) with SSCP 1REV (SEQ ID NO:14); H EAR 1 F (SEQ ID NO:44) with H NOT1 REV (SEQ ID NO:45); and SSCP 4F (SEQ ID NO:19) with H NOT1 REV (SEQ ID NO:45).

To obtain the 5' and 3' ends of the cDNA clone, nested PCR primers were designed from the bovine sequence by RACE (rapid amplification of cDNA ends) methodology (SMART RACE cDNA Amplification Kit, Clontech), and with the aid of primer design software. For increased specificity, the primers were designed to have particularly high melting temperatures. The designed primers are set forth in Table 4.

**Table 4**

| **SEQ ID NO:** | **PRIMER** | **SEQUENCE** |
|---|---|---|
| 46 | GS BC1A1 118REV | GTCATGGTACCTGAGGCCGTTCTGTACGCA |
| 47 | GS BC1A1 190REV | ACGTCATCGCACAGCACGTTGCCGTTGTC |
| 48 | GS BC1A1 213REV | AGGACAGTCCTTAAGTTCGTCGCAGATCACGTCA |
| 49 | GS BC1A1 761REV | AGGGAGGCCAGCTGTTCCAGGCAATC |
| 50 | GS BC1A1 3085F | CCGAAGGTTCCCCTGGACGAGATGGTT |
| 51 | GS BC1A1 3305F | CGTGGTGACAAGGGTGAGACAGGCGAACA |
| 52 | GS BC1A1 3675F | CGGGCTGATGATGCCAATGTGGTCCGT |
| 53 | GS BC1A1 3905F | AACATGGAAACCGGTGAGACCTGTGTATACCC |

The total bovine mRNA described above was further utilized to prepare new cDNA pools with the necessary external priming sites for use as PCR templates. PCR products were obtained at both the 5' and 3' ends of the gene using: (1) touchdown PCR techniques; (2) the newly designed bovine RACE PCR primers; and (3) materials supplied in the kit. Two touchdown PCR programs were used in a Peltier-cooled thermal cycler using the following protocol and conditions:

| | |
|---|---|
| 72 °C - 68 °C touchdown program I: | |
| Step 1: 8 cycles with the following conditions : | |
| | 94 °C for 10 seconds |
| | 72 °C for 10 seconds, each cycle thereafter drop 0.5°C |
| | 72 °C for 3 minutes |
| Step 2: 28 cycles of the following conditions: | |
| | 94 °C for 10 seconds |
| | 68 °C for 10 seconds |
| | 72 °C for 3 minutes |
| | 72 °C for 10 minutes |
| | 4 °C HOLD |

| | |
|---|---|
| 68 °C - 64 °C touchdown program II: | |
| Step 1: 8 cycles of the following conditions: | |
| | 94 °C for 10 seconds |
| | 68 °C for 10 seconds, each cycle thereafter drop 0.5°C |
| | 72 °C for 3 minutes |
| Step 2: 28 cycles of the following conditions: | |
| | 94 °C for 10 seconds |
| | 64 °C for 10 seconds |
| | 72 °C for 3 minutes |
| | 72 °C for 10 minutes |
| | 4 °C HOLD |

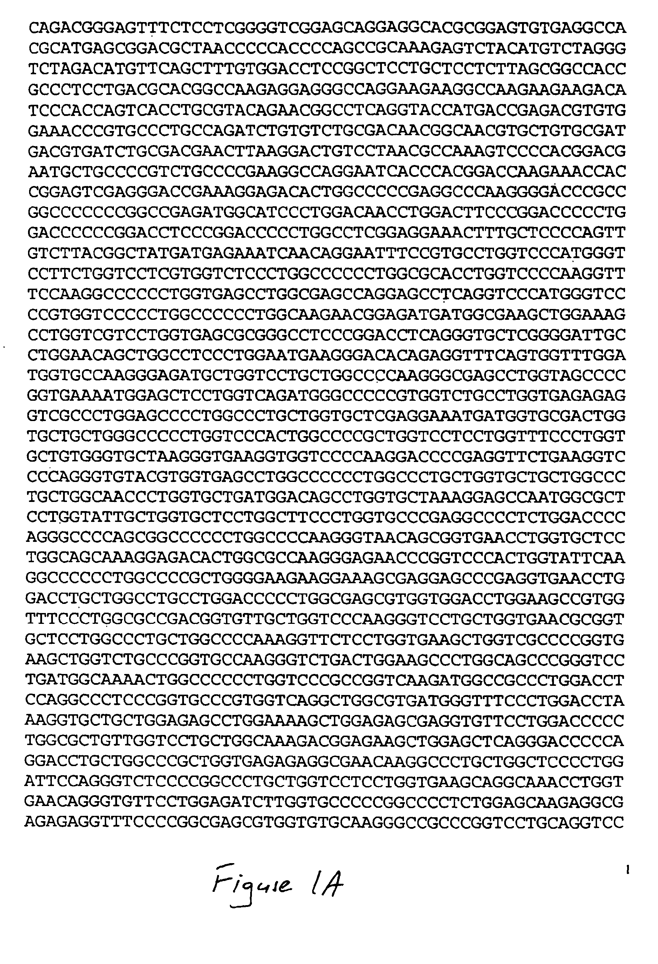

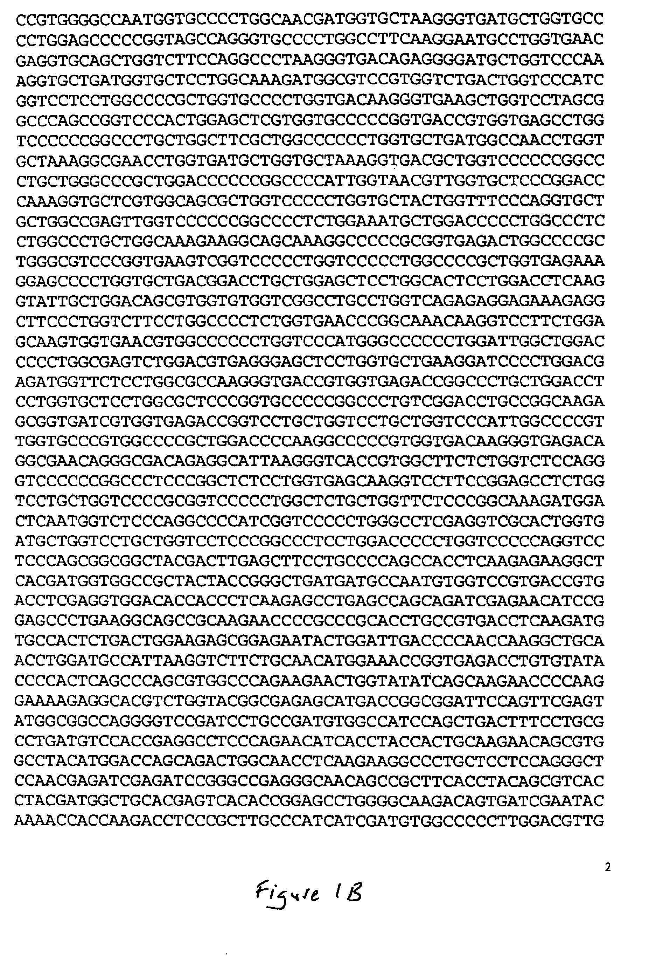

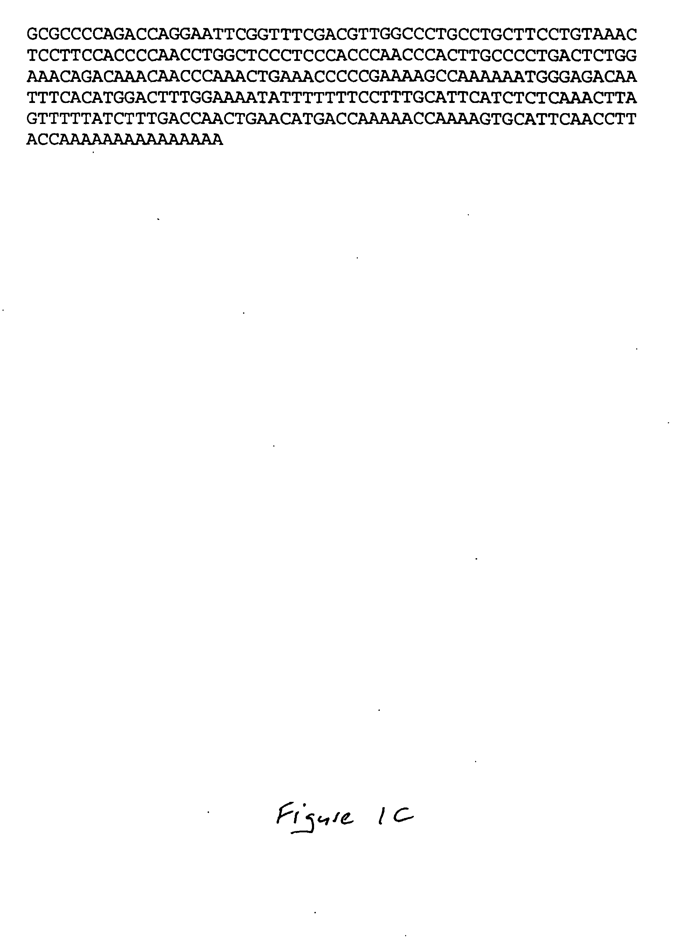

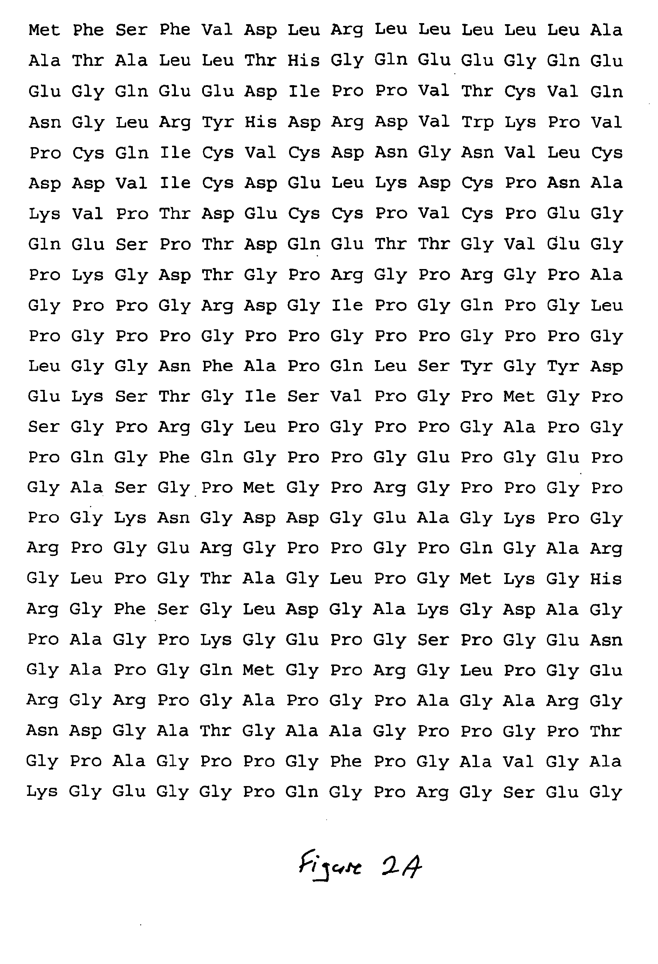

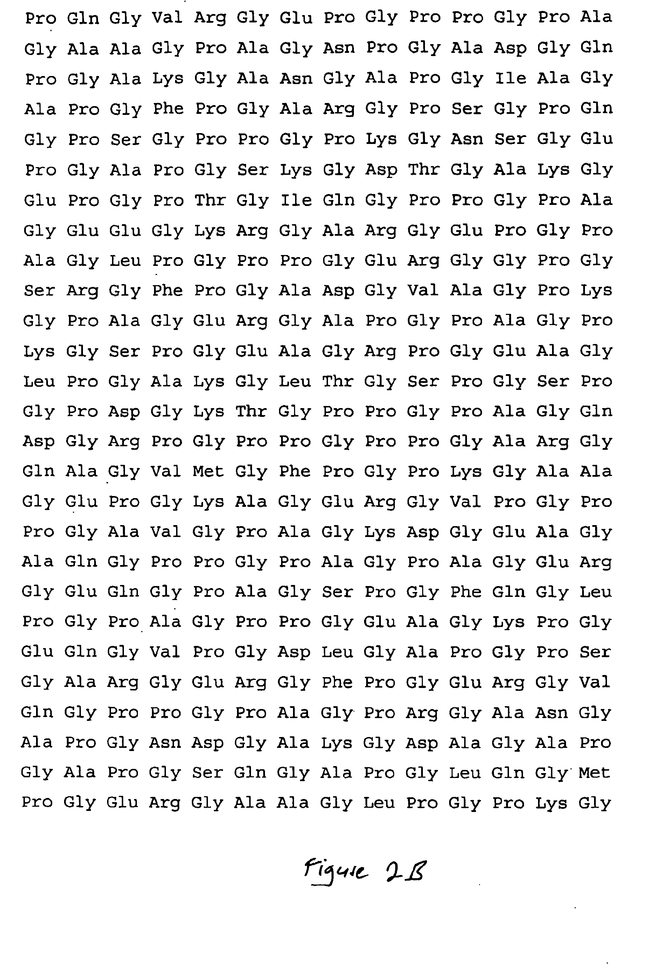

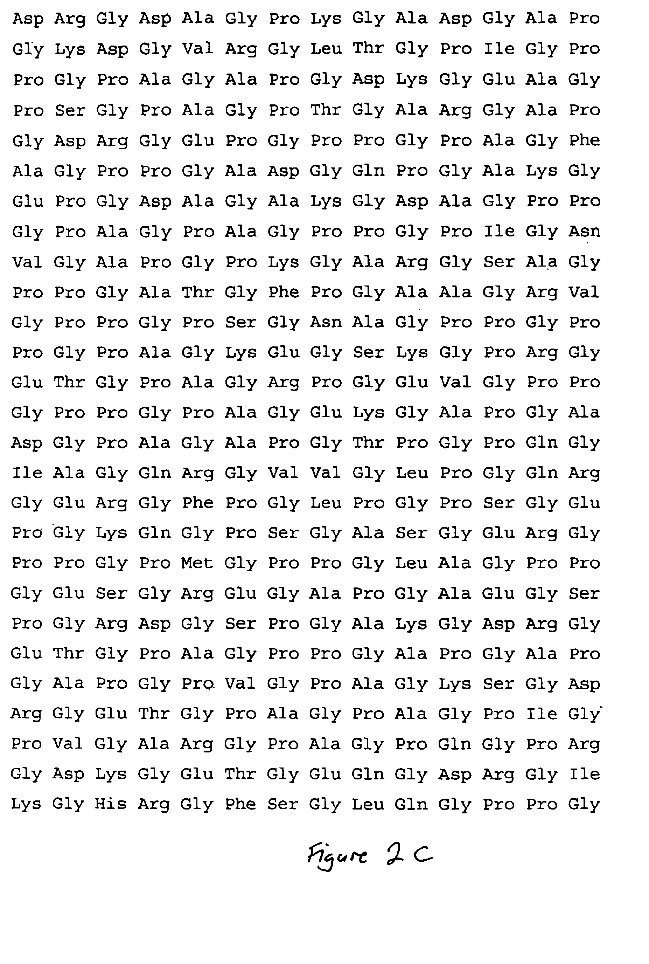

The resulting fragments were examined by 1.2% agarose gel electrophoresis, and subsequent cloning and sequencing analysis was performed. PCR products resulting from both programs were used. The resulting sequences overlapped the previously cloned bovine α1(I) collagen sequences, and encoded the 5' and 3' ends of the ORF as well as the contiguous untranslated cDNA regions. The nucleotide sequence for bovine procollagen type Iα1 is shown in Figures 1A through 1C (SEQ ID NO:1). The corresponding amino acid sequence is described in Figures 2A through 2D (SEQ ID NO:2).

As shown in Figures 13A through 13I, translated bovine collagen ORF sequences were aligned with known human (HU), mouse (MUS), dog (CANIS), bullfrog (RANA), and Japanese newt (CYNPS) sequences. The translated bovine sequence also aligns with published amino acid sequence fragments of the triple helical repeat domains of bovine α1(I) collagen. (See, e.g., Miller (1984) Extracellular Matrix Biochemistry, ed. Piez, et al., Elsevier Science Publishing, New York, pp. 41-81; and SWISSPROT database accession number p02453.) Numerous differences between the predicted bovine α1(I) collagen protein sequence provided by the present invention and previously known bovine protein sequences were noted. Some of these differences include substitutions of amino acids that are typically difficult to distinguish by protein sequencing (i.e., glutamine/glutamic acid and aspartic acid/asparagine). The polynucleotide sequence disclosed herein as SEQ ID NO:1 suggests these know bovine α1(I) collagen protein sequences may include errors, and therefore may, for example, be precluded for use in construction of a synthetic gene encoding authentic bovine α1(I) collagen gene by amino acid back-translation.

### Example 2: Production of Animal Collagens and Gelatins in Transgenic Plants

The cDNAs encoding an animal collagen of the present invention, an α subunit of prolyl 4-hydroxylase, and a β subunit of prolyl 4-hydroxylase are cloned into an appropriate plant expression vector that contains the necessary elements to properly express a foreign protein. Such elements may include, for example a signal peptide, promoter and a terminator, (See, e.g., Rogers et al., *supra;* Schardl et al., *supra;* Berger et al., *supra*.) For example, pVL vectors have been described in the art. (See, e.g., A. Lamberg et al. (1996) J. Biol. Chem 271:11988-11995.) These recombinant pVL vectors are used as a gene source for the construction of plant expression vectors using conventional methods known in the art. In order to express the collagen in plant or plant cells, the nucleic acid sequences are operably linked, for example, to a CaMV 35S promoter. The nucleic acid sequences encoding an a subunit or βsubunit of prolyl 4-hydroxylase are operably linked to a CaMV 35S promoter, and may be present on the same plasmid or on different plasmids to produce a biologically active prolyl 4-hydroxylase.

The expression vectors are transformed into plants or plant cells using transformation techniques well known in the art. The expression clones are selected by, for example, northern and western blotting, and can be cultivated in a fermentor to generate a cell mass for purification of recombinant collagen.

The expression of the α subunit and the β subunit of prolyl 4-hydroxylase and animal collagen is screened, for example, by immunoblotting using three hundred (300) mg cell pellets extraction in 10mM Tris, pH 7,8, 100mM NaCl, 100mM Glycine, 10uM DTT, 0.1% Triton X100, 2uM Leupeptin, and 0.25mM PMSF. The proteins in the extract are separated with 4-20% SDS-PAGE, and transferred to a nitrocellulose membrane to be probed with antibodies against the α subunit and β subunit of prolyl 4-hydroxylase and the animal collagen.

To characterize recombinant animal collagen produced in plants or plant cells, the following protocol is carried out:
1. Suspend and homogenize cell pellets in 1M NaCl, 0.05M Tris, pH7.4 and stir for 1 hour at 4°C. Collect the supernatant by centrifugation at 4°C;
2. Add 7.5ml acetic acid to the supernatant and incubate at 4°C for 2 hours. Collect the pellet by centrifugation at 4°C;
3. Wash the pellet twice with 2M NaCl, 0.05M Tris, pH 7.4;
4. Re-dissolve in 2M Urea, 0.2M NaCl, 0.05M Tris, pH 7.4;
5. Dialyze against 2M Urea, 0.2M NaCl, 0.05M Tris, pH 7.4;
6. Run through a DEAE-cellulose column. Collect the flow-through;
7. Add acetic acid to 0.5M and add NaCl to 0.9M and incubate for 2 hours at 4°C;
8. Collect pellets by centrifugation;
9. Resuspend the pellet in 0.5M acetic acid and stir overnight at 4°C;
10. Digest the pellet with 0.1mg/ml pepsin for 2 hours;
11. Add saturated Tris buffer and adjust pH to 7.4,
12. Incubate overnight to inactivate pepsin;
13. Add NaCl to 0.9M and acetic acid to 0.5M, Incubate for 2 hours at 4°C;
14. Collect the pellet by centrifugation at 4°C;
15. Wash the pellet with 2M NaCl, 0.05M Tris, pH 7.4;
16. Dissolve in 2M Urea, 150M NaCl and 0.05M Tris, pH 7.4; and
17. Heat the sample at 56°C for 5 min and then load to Bio-Gel TSK 40 column operated by HPLC system.

The resulting purified collagen is characterized by amino acid composition analysis.

Various modifications and variations of the described methods and systems of the invention will be apparent to those skilled in the art without departing from the scope of the appended claims. Although the invention has been described in connection with specific preferred embodiments, it should be understood that the invention as claimed should not be unduly limited to such specific embodiments. Indeed, various modifications of the described modes for carrying out the invention will be obvious to those skilled in molecular biology or related fields.

## Claims

1. A recombinant bovine collagen polypeptide comprising the amino acid sequence of SEQ ID NO:2.

2. The polypeptide of claim 1, wherein the polypeptide is single-chain.

3. The polypeptide of claim 1, wherein the polypeptide is homotrimeric.

4. The polypeptide of claim 1, wherein the polypeptide is heterotrimeric.

5. The polypeptide of claim 1, wherein the polypeptide consists of the amino acid sequence of SEQ ID NO:2.

6. A composition comprising a polypeptide according to any one of the preceding claims.

7. An isolated and purified polynucleotide encoding SEQ ID NO:2.

8. An isolated and purified polynucleotide complementary to the polynucleotide of claim 7.

9. A composition comprising the polynucleotide of claim 7.

10. An expression vector comprising the polynucleotide of claim 7.

11. A host cell comprising the polynucleotide of claim 7.

12. The host cell of claim 11, wherein the host cell is a prokaryotic host cell.

13. The host cell of claim 11, wherein the host cell is a eukaryotic host cell.

14. The host cell of claim 11, wherein the host cell is selected from the group consisting of an animal cell, a yeast cell, a plant cell, an insect cell, and a fungal cell.

15. A transgenic non-human animal comprising the polynucleotide of claim 7.

16. A transgenic plant comprising the polynucleotide of claim 7.

17. A method for producing a bovine α1(I) collagen, the method comprising:
(a) culturing the host cell of claim 11 under conditions suitable for expression of the polypeptide; and
(b) recovering the polypeptide from the host cell culture.

18. A method for producing recombinant gelatin, the method comprising:
(a) providing recombinant animal bovine α1(I) collagen polypeptides according to any of claims 1-5; and
(b) deriving recombinant animal gelatin therefrom.

19. A method for producing recombinant animal gelatin, the method comprising producing recombinant bovine gelatin directly from a polynucleotide encoding SEQ ID NO:2.

20. The polynucleotide of claim 7 wherein the polynucleotide comprises or consists of SEQ ID NO:1.

## Patentansprüche

1. Rekombinantes bovines Kollagenpolypeptid, umfassend die Aminosäuresequenz SEQ ID NR.: 2.

2. Polypeptid nach Anspruch 1, wobei das Polypeptid einzelkettig ist.

3. Polypeptid nach Anspruch 1, wobei das Polypeptid homotrimer ist.

4. Polypeptid nach Anspruch 1, wobei das Polypeptid heterotrimer ist.

5. Polypeptid nach Anspruch 1, wobei das Polypeptid aus der Aminosäuresequenz SEQ ID NR.: 2 besteht.

6. Zusammensetzung, umfassend ein Polypeptid nach einem der vorhergehenden Ansprüche.

7. Isoliertes und gereinigtes Polynukleotid, welches SEQ ID NR.: 2 kodiert.

8. Isoliertes und gereinigtes Polynukleotid, welches komplementär zum Polynukleotid nach Anspruch 7 ist.

9. Zusammensetzung, umfassend das Polynukleotid nach Anspruch 7.

10. Expressionsvektor, umfassend das Polynukleotid nach Anspruch 7.

11. Wirtszelle, umfassend das Polynukleotid nach Anspruch 7.

12. Wirtszelle nach Anspruch 11, wobei die Wirtszelle eine prokaryontische Wirtszelle ist.

13. Wirtszelle nach Anspruch 11, wobei die Wirtszelle eine eukaryontische Wirtszelle ist.

14. Wirtszelle nach Anspruch 11, wobei die Wirtszelle ausgewählt ist aus der Gruppe, bestehend aus einer Tierzelle, einer Hefezelle, einer Pflanzenzelle, einer Insektenzelle und einer Pilzzelle.

15. Transgenes, nicht-menschliches Tier, umfassend das Polynukleotid nach Anspruch 7.

16. Transgene Pflanze, umfassend das Polynukleotid nach Anspruch 7.

17. Verfahren zum Herstellen eines bovinen α1(I)-Kollagens, wobei das Verfahren umfaßt:
(a) Kultivieren der Wirtszelle nach Anspruch 11 unter für die Expression des Polypeptids geeigneten Bedingungen und
(b) Gewinnen des Polypeptids aus der Wirtszellenkultur.

18. Verfahren zum Herstellen von rekombinanter Gelatine, wobei das Verfahren umfaßt:
(a) zur Verfügung stellen von rekombinanten tierischen bovinen α1(I)-Kollagen-Polypeptiden nach einem der Ansprüche 1 bis 5, und
(b) Erhalten von rekombinanter Tiergelatine davon.

19. Verfahren zum Herstellen von rekombinanter Tierigelatine, wobei das Verfahren das Herstellen von rekombinanter boviner Gelatine direkt von einem Polynukleotid, welches SEQ ID NR.: 2 kodiert, umfaßt.

20. Polynukleotid nach Anspruch 7, wobei das Polynukleotid SEQ ID NR.: 1 umfaßt oder daraus besteht.

## Revendications

1. Polypeptide de collagène bovin recombinant comprenant la séquence d'acides aminés de SEQ ID N°2.

2. Polypeptide selon la revendication 1, ledit polypeptide étant un polypeptide à simple chaîne.

3. Polypeptide selon la revendication 1, ledit polypeptide étant un polypeptide homotrimère.

4. Polypeptide selon la revendication 1, ledit polypeptide étant un polypeptide hétérotrimète.

5. Polypeptide selon la revendication 1, ledit polypeptide se composant de la séquence d'acides aminés de SEQ ID N°2.

6. Composition comprenant un polypeptide selon l'une quelconque des revendications précédentes.

7. Polynucléotide isolé et purifié codant pour la SEQ ID N° 2.

8. Polynucléotide isolé et purifié complémentaire du polynucléotide selon la revendication 7.

9. Composition comprenant le polynucléotide selon la revendication 7.

10. Vecteur d'expression comprenant le polynucléotide selon la revendication 7.

11. Cellule hôte comprenant le polynucléotide selon la revendication 7.

12. Cellule hôte selon la revendication 11, ladite cellule hôte étant une cellule hôte procaryote.

13. Cellule hôte selon la revendication 11, ladite cellule hôte étant une cellule hôte eucaryote.

14. Cellule hôte selon la revendication 11, ladite cellule hôte étant choisie dans le groupe constitué d'une cellule animale, d'une cellule de levure, d'une cellule végétale, d'une cellule d'insecte et d'une cellule de champignon.

15. Animal non humain transgénique comprenant le polynucléotide selon la revendication 7.

16. Plante transgénique comprenant le polynucléotide selon la revendication 7.

17. Procédé de préparation d'un collagène α1 (type I) bovin, ledit procédé comprenant les étapes consistant à :
(a) procéder à la culture de la cellule hôte selon la revendication 11 dans des conditions adaptées à l'expression du polypeptide ; et
(b) récupérer le polypeptide à partir de la culture de cellule hôte.

18. Procédé de préparation d'une gélatine recombinante, ledit procédé comprenant les étapes consistant à :
(a) fournir les polypeptides de collagène α1 (type I) bovin, animal recombinants selon l'une quelconque des revendications 1 à 5 ; et
(b) en faire dériver une gélatine animale recombinante.

19. Procédé de préparation d'une gélatine animale recombinante, ledit procédé comprenant la production de gélatine bovine recombinante directement à partir d'un polynucléotide codant pour la SEQ ID N° 2.

20. Polynucléotide selon la revendication 7, ledit polynucléotide comprenant ou se composant de la SEQ ID N° 1.
